# EUROPEAN PATENT SPECIFICATION

(11) **EP 4 384 268 B1**
(45) Date of publication and mention of the grant of the patent: **18.03.2026**
(21) Application number: 22783559.2
(22) Date of filing: 11.08.2022
(51) Int. Cl.: A61P 1/16, A61P 3/10, A61P 29/00, C07D 413/12, C07D 417/12, A61K 31/53, A61K 31/5415

(54) **SMALL MOLECULE UREA DERIVATIVES AS STING ANTAGONISTS**
KLEINMOLEKÜLIGE HARNSTOFFDERIVATE ALS STING-ANTAGONISTEN
DÉRIVÉS D'URÉE À PETITES MOLÉCULES EN TANT QU'ANTAGONISTES DE STING

(30) Priority: 11.08.2021 IN 202111036319
(43) Date of publication of application: 19.06.2024
(73) Proprietor: Curadev Pharma Pvt. Ltd., Noida 201305 (IN)
(72) Inventor: BANERJEE, Monali, Noida Uttar Pradesh 201305 (IN); BASU, Sourav, Noida Uttar Pradesh 201305 (IN); SHRIVASTAVA, Ritesh Kumar, Noida Uttar Pradesh 201305 (IN); PRYDE, David Cameron, Sandwich Kent CT13 9ND (GB); MIDDYA, Sandip Kumar, Noida Uttar Pradesh 201305 (IN); GHOSH, Rajib, Noida Uttar Pradesh 201305 (IN); YADAV, Dharmendra B., Noida Uttar Pradesh 201305 (IN); SURYA, Arjun, Noida Uttar Pradesh 201305 (IN)
(74) Representative: Petty, Catrin Helen
(86) International application number: PCT/IB2022/057491
(87) International publication number: WO 2023/017452

(56) References cited:
- WO-A1-03/092670
- WO-A1-2018/234805
- WO-A1-2018/234808
- WO-A1-2019/243823
- WO-A1-2021/161230
- YONG-JIN WU ET AL: "(S)-N-[1-(4-Cyclopropylmethyl-3,4-dihydro-2H-benzooxazin-6-yl)-ethyl]-3-(2-fluoro-phenyl)-acrylamide is a potent and efficacious KCNQ2 opener which inhibits induced hyperexcitability of rat hippocampal neurons", BIOORGANIC & MEDICINAL CHEMISTRY LETTERS, vol. 14, no. 8, 1 April 2004 (2004-04-01), Amsterdam NL, pages 1991 - 1995, XP055497119, ISSN: 0960-894X, DOI: 10.1016/j.bmcl.2004.01.069

## Description

The present invention relates to small molecule antagonists of the Stimulator of Interferon Genes (STING) protein. Accordingly, the small molecule antagonists may be of use in the treatment of various inflammatory diseases such as fatty liver disease, pulmonary fibrosis, pancreatitis, lupus, and so on. The invention extends to the pharmaceutical compositions of the compounds *per se.* Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

This disclosure also provides methods of making the compounds and methods of modulating the STING protein using these compounds.

STING (STimulator of INterferon Genes) is an innate signalling molecule that plays a crucial role in mediating an immune response to cytosolic DNA.

The human immune system has evolved to recognize and respond to different types of threats and pathogens to maintain a healthy host. The innate arm of the immune system is mainly responsible for a rapid initial inflammatory response to danger signals associated with cellular or tissue damage from bacteria, viruses and other infectious threats. The innate immune system responds to these damage-associated molecular patterns (DAMPs) or microbial product pathogen-associated molecular patterns (PAMPs) through an array of sentinel proteins called pattern recognition receptors (PRRs) to provide broad and lasting protection to the host against a wide range of threats (P. Broz et. al., Nat. Revs Immunol., 2013, 13, 551).

The PAMPs and DAMPs are often constituents or replication intermediates of intracellular pathogens. PRRs include Toll-like receptors (TLRs; activated by endosomal nucleic acids), C-type lectin receptors, retinoic acid inducible gene I (RIGI-like receptors; activated by cytosolic RNA), NOD-like receptors (NLRs) and also double stranded DNA sensors (Diebold et. al., Science, 2004, 303, 1529-1531; O. Takeuchi et. al., Cell, 2010, 140, 805; Pichlmair et. al., **2006,** 314, 997). PRRs respond to DAMPs and PAMPs by up-regulating type-1 interferons and cytokines. Free cytosolic nucleic acids (DNA and RNA) are known PAMPs/DAMPs. The main sensor for cytosolic DNA is cGAS (cyclic GMP-AMP synthase). Upon recognition of cytosolic dsDNA, cGAS triggers formation of one specific isomer of the cyclic dinucleotide (CDN) cGAMP, c[G(2',5')pA(3',5')p] (Gao et. al., Cell, 2013, 153, 1094).

CDNs are second messenger signalling molecules produced by diverse bacteria and consist of two ribonucleotides that are connected via phosphodiester bonds to make a cyclic structure. CDNs cyclo-di(GMP) (c-diGMP), cyclo-di(AMP) (c-diAMP) and hybrid cyclo-(AMP/GMP) (cGAMP) derivatives (A. Ablasser et. al., Nature, 2013, 498, 380) all bind strongly to the ER-transmembrane adaptor protein STING (D.L. Burdette et. al., Nature, 2011, 478, 515; H. Ishikawa, Nature, 2008, 455, 674).

STING recognises CDNs through its cytosolic carboxy-terminal domain, which forms a homodimer and adopts a V-shaped binding pocket to bind CDNs (Zhang et. al., Mol. Cell, 2013, 51, 226; G. N. Barber et. al., Nat. Immunol., 2011, 12, 929). Ligand-induced activation of STING triggers its relocation to the Golgi and a conformational change to facilitate binding to TBK1. TBK1 in turn signals through the transcription factors IRF-3, STAT6 and NFκB to induce type-I interferons and other cytokines and interferon-stimulated genes (C. Greenhill, Nat. Revs., Endocrinol., 2018, 14, 192; Y. Li, H.L. Wilson, and E. Kiss-Toth, J. Inflamm., 2017, 14, 11). Following its activation, STING is rapidly degraded in the normal response.

Excessive activation of STING is associated with a range of monogenic autoinflammatory disorders referred to as interferonopathies (Y.J. Crow and N. Manel, Nat. Revs. Immunol., 2015, 15, 429-440). Loss of function mutations in the human DNAse Trex1 are associated with elevated levels of cGAMP and autoimmune diseases such as the rare but severe inflammatory disease Aicardi-Goutieres syndrome (AGS), familial chilblain lupus (FCL), systemic lupus erythematosus (SLE) and retinal vasculopathy (Y. Crow et. al., Hum. Mol. Gen., 2009, 18, R130).

Inhalation of silica particles can result in lung inflammation and pulmonary fibrosis, triggered by lung cell death and release of dsDNA products. Benmerzoug *et. al.* have reported that this increase in circulating dsDNA activates STING and via increased levels of CXCL10 and IFN signalling produces lung inflammation (S. Benmerzoug et. al., Nat. Comm., 2018, 9, 5226).

Increased cytosolic dsDNA was detected in fibroblast-like synoviocytes (FLS) taken from rheumatoid arthritis (RA) patients with the levels of dsDNA correlating with the severity of rheumatoid synovitis (J. Wang et. al., Int. Immunopharm., 2019, 76, 105791). These findings indicated that increased dsDNA promoted an inflammatory response via the STING pathway in RA FLS and led to increased expression of STING, suggesting that cytosolic DNA accumulation is an important factor in RA-related inflammation.

Patients with autosomal dominant gain of function mutations in STING have a pediatric autoinflammatory condition called SAVI (STING-associated vasculopathy with onset in infancy), manifest clinically as skin rash, vasculopathy, lupus-like syndromes and pulmonary fibrosis characterised by aberrant IFN production and systemic inflammation that are associated with high morbidity and mortality (N. Konig, et. al., Ann. Rheum., Dis., 2017, 76, 468). Characterised mutations in humans include V147L, N154S, V155M and G166E which are all located at the interfacial region between the trans-membrane domain and the ligand binding domain and result in ligand-independent constitutively activated protein. More recently, three other gain of function STING mutations C206Y, R281Q and R284S have been identified at a cluster region that is proposed to promote STING aggregation and disfavour complexation to the C-terminal tail region (H. Konno, et. al., Cell Rep. 2018, 23, 1112 and I. Melki, et. al., J Allergy Clin. Immunol. 2017, 140(2), 543.

A recent report by Habtezion et al. has shown that in mice with acute pancreatitis, STING responds to acinar cell death by detecting DNA from necrotic cells and promotes acute pancreatic inflammation (A. Habtezion et. al., Gastroenterology, 2018, 154, 1822). STING-knockout mice had less severe acute pancreatitis (less edema, less inflammation) while administering a STING agonist resulted in more severe pancreatitis.

Luo et al. have also shown recently that levels of STING were increased in liver tissues from patients with non-alcoholic fatty liver disease and in mice with a high-fat diet induced hepatic steatosis. Once again, STING-knockout mice developed less severe liver fibrosis and a less acute inflammatory response (X. Luo et.al., Gastroenterology, 2018, 155, 1971).

Elevated cGAMP levels in the peripheral blood mononuclear cells of SLE patients was associated with higher disease scores (J. An et. al., Arthritis Rheum., 2017, 69, 800) suggesting a link between disease severity in lupus and activation of the STING pathway.

The kidney tubule cells of subjects with fibrosis have been shown to lack mitochondrial transcription factor A (TFAM). Mice lacking tubule TFAM developed severe mitochondrial loss and energy deficit caused by aberrant packaging of mitochondrial DNA and its translocation to the cytosol, where the STING pathway was activated (K.W. Chung, Cell Metab., 2019, 30, 1). The ensuing cytokine expression and inflammation led to renal fibrosis.

Bennion et. al. have demonstrated that the gain of function mutation N153S knock-in mice showed enhanced susceptibility to viral infection and responded to infection by a murine gamma herpesvirus yHV68 with severe autoinflammation and pulmonary fibrosis (B. Bennion et. al., J. Virol., 2019, 93, e01806).

Other conditions where excessive immune system activation may be linked to STING pathway activation include systemic inflammatory response syndrome (R.K. Boyapati et. al., F1000 Res., 2017, 6, 169), cardiovascular disease (K.R. King et. al., Nat. Med., 2017, 23, 1481), stroke (A.M. Jeffries et. al., Neurosci. Lett., 2017, 658, 53) and age-related macular degeneration (N. Kerur et. al., Nat. Med., 2018, 24, 50).

There is therefore a compelling body of evidence that blocking, inhibiting or antagonising the STING pathway could have therapeutic benefit in a number of conditions and disease states.

There have been few small molecule antagonists of the STING protein reported, for example by T. Siu et al. (ACS Med Chem Letts, 2019, 10(1), 92) but the compounds described therein reportedly have low cell-based potency. Other reports of STING antagonists include S. Haag et al. (Nature, 2018, 559(7713), 269) and Z. Hong et al. (PNAS, 2021, 118(24), e2105465118).

There is therefore a pressing need for improved small molecule blockers of the STING pathway, and in particular for small molecule direct antagonists of the STING protein.

WO 2021/161230 relates to small molecule STING antagonists which may be used in the treatment of inflammatory diseases.

WO 2018/234805 and WO 2019/243823 relate to small molecule STING modulators which may be used in the treatment of diseases.

Yong-Jin Wu et al. (Yong-Jin Wu et al., Bioorg. Med. Chem. Lett., 2004, 14(8), 1991-1995) describes a KCNQ2 channel opener, (*S*)-N-[1-(4-Cyclopropylmethyl-3,4-dihydro-2Hbenzooxazin-6-yl)-ethyl]-3-(2-fluoro-phenyl)-acrylamide.

WO 03/092670 relates to compounds which are bombesin antagonists, uses thereof, processes for the production thereof, intermediates used in the preparation thereof and compositions thereof.

The present invention has arisen from the inventors work in attempting to identify STING protein modulators.

The invention is as defined in the claims.

In accordance with a first aspect of the invention, there is provided a compound of formula (I): 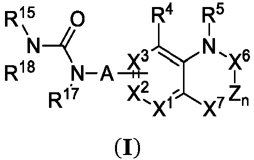 ,
wherein X¹ is CR¹;
X² is CR²;
X³ is CR³;
X⁶ is C=O or CR⁷R⁸;
Z is CR⁹R¹⁰;
X⁷ is S, SO or O;
A is an optionally substituted C₁-C₆ alkylene, an optionally substituted C₂-C₆ alkenylene or an optionally substituted C₂-C₆ alkynylene;
n is 1;
R¹, R⁴ and R⁸ are each independently selected from the group consisting of H, halogen, OR¹³, CN, CONR¹³R¹⁴, NR¹³R¹⁴, NR¹³COR¹⁴, optionally substituted C₁-C₆ alkyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl and optionally substituted mono or bicyclic 3 to 8 membered heterocycle;
R⁹ and R¹⁰ are each independently selected from the group consisting of H, halogen, OR¹³, CN, CONR¹³R¹⁴, NR¹³R¹⁴, NR¹³COR¹⁴, optionally substituted C₁-C₆ alkyl, optionally substituted C₂-C₆ alkenyl or optionally substituted C₂-C₆ alkynyl;
one of R² and R³ is -A-NR¹⁷-C(O)-NR¹⁸-R¹⁵ and the other of R² and R³ is selected from the group consisting of H, halogen, OR¹³, CN, COOR¹³, CONR¹³R¹⁴, NR¹³R¹⁴, NR¹³COR¹⁴, optionally substituted C₁-C₆ alkyl, optionally substituted mono or bicyclic C₃-C₆ cycloalkyl mono or bicyclic optionally substituted C₆-C₁₂ aryl, mono or bicyclic optionally substituted 5 to 10 membered heteroaryl and optionally substituted mono or bicyclic 3 to 8 membered heterocycle;
R⁵ is selected from the group consisting of H, CONR¹³R¹⁴, optionally substituted C₁-C₆ alkyl, optionally substituted C₁-C₆ alkylsulfonyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl, optionally substituted mono or bicyclic 3 to 8 membered heterocycle and L¹-L²-R¹⁶;
R⁷ is selected from the group consisting of H, halogen, CONR¹³R¹⁴, optionally substituted C₁-C₆ alkyl, optionally substituted mono or bicyclic C₃-C₆ cycloalkyl, optionally substituted C₂-C₆ alkenyl, optionally substituted C₂-C₆ alkynyl, mono or bicyclic optionally substituted C₆-C₁₂ aryl, mono or bicyclic optionally substituted 5 to 10 membered heteroaryl, optionally substituted mono or bicyclic 3 to 8 membered heterocycle and L¹-L²-R¹⁶;
wherein a maximum of one of R⁵ and R⁷ is -L¹-L²-R¹⁶;
R¹³ and R¹⁴ are each independently selected from the group consisting of H, halogen, OH, CN, COOH, CONH₂, NH₂, NHCOH, optionally substituted C₁-C₆ alkyl, optionally substituted C₁-C₆ alkylsulfonyl, optionally substituted mono or bicyclic C₃-C₆ cycloalkyl, optionally substituted C₂-C₆ alkenyl, optionally substituted C₂-C₆ alkynyl, optionally substituted C₁-C₆ alkoxy, optionally substituted C₁-C₆ alkoxycarbonyl group, mono or bicyclic optionally substituted C₆-C₁₂ aryl, mono or bicyclic optionally substituted 5 to 10 membered heteroaryl, optionally substituted mono or bicyclic 3 to 8 membered heterocycle, optionally substituted aryloxy, optionally substituted heteroaryloxy and optionally substituted heterocyclyloxy;
L¹ is absent or an optionally substituted C₁-C₆ alkylene, an optionally substituted C₂-C₆ alkenylene, an optionally substituted C₂-C₆ alkynylene, O, S, S=O, SO₂ or NR¹⁹;
L² is absent or an optionally substituted C₁-C₆ alkylene, an optionally substituted C₂-C₆ alkenylene, an optionally substituted C₂-C₆ alkynylene, O, S, S=O, SO₂ or NR¹⁹;
R¹⁵ is optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle;
R¹⁶ is optionally substituted C₁-C₆ alkyl, optionally substituted C₂-C₆ alkenyl, optionally substituted C₂-C₆ alkynyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle; and
R¹⁷ to R¹⁹ are independently H, an optionally substituted C₁-C₆ alkyl or an optionally substituted C₂-C₆ alkenyl;
or a pharmaceutically acceptable complex, salt, solvate, tautomeric form or polymorphic form thereof; and
wherein the compound is not 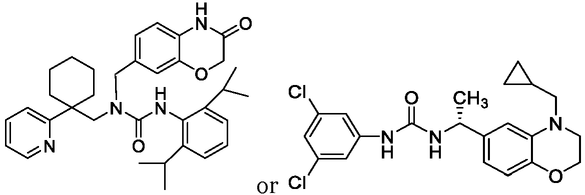 .

The compounds of formula (I) may be used as medicaments.

Hence, in a second aspect, there is provided a compound of formula (I), or a pharmaceutically acceptable complex, salt, solvate, tautomeric form or polymorphic form thereof, for use as a medicament.

The inventors have found that compounds of formula (I) are useful in modulating the STimulator of INterferon Genes (STING) protein.

Hence, this disclosure also provides a compound of formula (I), or a pharmaceutically acceptable complex, salt, solvate, tautomeric form or polymorphic form thereof, for use in modulating the STimulator of INterferon Genes (STING) protein.

Preferably, the compound of formula (I) is for use in inhibiting, or inactivating, the STING protein. The compound of formula (I) may be for use in inhibiting, or inactivating, STING functional activity as evidenced by a reduction of one or more biological effects selected from the group consisting of cellular interferon β production, cellular levels of interferon-stimulated genes, production of cytokines and phosphorylation of the transcription factors IRF-3 and NF-κB.

By inhibiting the STING protein, it is possible to treat, ameliorate or prevent liver fibrosis, fatty liver disease, pulmonary fibrosis, lupus, rheumatoid arthritis (RA), STING-associated vasculopathy with onset in infancy (SAVI), pancreatitis, cardiovascular disease, non-alcoholic fatty liver disease and renal fibrosis.

By inhibiting the STING protein, it is possible to treat, ameliorate or prevent liver fibrosis, fatty liver disease, non-alcoholic steatohepatitis (NASH), pulmonary fibrosis, lupus, rheumatoid arthritis (RA), STING-associated vasculopathy with onset in infancy (SAVI), Aicardi-Goutieres syndrome (AGS), familial chilblain lupus (FCL), systemic lupus erythematosus (SLE), retinal vasculopathy, neuroinflammation, systemic inflammatory response syndrome, pancreatitis, cardiovascular disease, renal fibrosis, stroke and age-related macular degeneration (AMD).

Accordingly, in a third aspect there is provided a compound of formula (I), 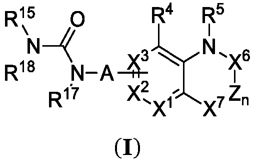
, wherein X¹ is CR¹;
X² is CR² and X³ is CR³;
X⁶ is C=O or CR⁷R⁸;
Z is CR⁹R¹⁰;
X⁷ is S, SO or O;
A is an optionally substituted C₁-C₆ alkylene, an optionally substituted C₂-C₆ alkenylene or an optionally substituted C₂-C₆ alkynylene;
n is 1;
R¹, R⁴ and R⁸ are each independently selected from the group consisting of H, halogen, OR¹³, CN, CONR¹³R¹⁴, NR¹³R¹⁴, NR¹³COR¹⁴, optionally substituted C₁-C₆ alkyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl and optionally substituted mono or bicyclic 3 to 8 membered heterocycle;
R⁹ and R¹⁰ are each independently selected from the group consisting of H, halogen, OR¹³, CN, CONR¹³R¹⁴, NR¹³R¹⁴, NR¹³COR¹⁴, optionally substituted C₁-C₆ alkyl, optionally substituted C₂-C₆ alkenyl or optionally substituted C₂-C₆ alkynyl;
one of R² and R³ is -A-NR¹⁷-C(O)-NR¹⁸-R¹⁵ and the other of R² and R³ is selected from the group consisting of H, halogen, OR¹³, CN, COOR¹³, CONR¹³R¹⁴, NR¹³R¹⁴, NR¹³COR¹⁴, optionally substituted C₁-C₆ alkyl, optionally substituted mono or bicyclic C₃-C₆ cycloalkyl mono or bicyclic optionally substituted C₆-C₁₂ aryl, mono or bicyclic optionally substituted 5 to 10 membered heteroaryl and optionally substituted mono or bicyclic 3 to 8 membered heterocycle;
R⁵ is selected from the group consisting of H, CONR¹³R¹⁴, optionally substituted C₁-C₆ alkyl, optionally substituted C₁-C₆ alkylsulfonyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl, optionally substituted mono or bicyclic 3 to 8 membered heterocycle and L¹-L²-R¹⁶;
R⁷ is selected from the group consisting of H, halogen, CONR¹³R¹⁴, optionally substituted C₁-C₆ alkyl, optionally substituted mono or bicyclic C₃-C₆ cycloalkyl, optionally substituted C₂-C₆ alkenyl, optionally substituted C₂-C₆ alkynyl, mono or bicyclic optionally substituted C₆-C₁₂ aryl, mono or bicyclic optionally substituted 5 to 10 membered heteroaryl, optionally substituted mono or bicyclic 3 to 8 membered heterocycle and L¹-L²-R¹⁶;
wherein a maximum of one of R⁵ and R⁷ is -L¹-L²-R¹⁶;
R¹³ and R¹⁴ are each independently selected from the group consisting of H, halogen, OH, CN, COOH, CONH₂, NH₂, NHCOH, optionally substituted C₁-C₆ alkyl, optionally substituted C₁-C₆ alkylsulfonyl, optionally substituted mono or bicyclic C₃-C₆ cycloalkyl, optionally substituted C₂-C₆ alkenyl, optionally substituted C₂-C₆ alkynyl, optionally substituted C₁-C₆ alkoxy, optionally substituted C₁-C₆ alkoxycarbonyl group, mono or bicyclic optionally substituted C₆-C₁₂ aryl, mono or bicyclic optionally substituted 5 to 10 membered heteroaryl, optionally substituted mono or bicyclic 3 to 8 membered heterocycle, optionally substituted aryloxy, optionally substituted heteroaryloxy and optionally substituted heterocyclyloxy;
L¹ is absent or an optionally substituted C₁-C₆ alkylene, an optionally substituted C₂-C₆ alkenylene, an optionally substituted C₂-C₆ alkynylene, O, S, S=O, SO₂ or NR¹⁹;
L² is absent or an optionally substituted C₁-C₆ alkylene, an optionally substituted C₂-C₆ alkenylene, an optionally substituted C₂-C₆ alkynylene, O, S, S=O, SO₂ or NR¹⁹;
R¹⁵ is optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle;
R¹⁶ is optionally substituted C₁-C₆ alkyl, optionally substituted C₂-C₆ alkenyl, optionally substituted C₂-C₆ alkynyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle; and
R¹⁷ to R¹⁹ are independently H, an optionally substituted C₁-C₆ alkyl or an optionally substituted C₂-C₆ alkenyl;
or a pharmaceutically acceptable complex, salt, solvate, tautomeric form or polymorphic form thereof, for use in treating, ameliorating or preventing a disease selected from liver fibrosis, fatty liver disease, non-alcoholic steatohepatitis (NASH), pulmonary fibrosis, lupus, sepsis, rheumatoid arthritis (RA), type I diabetes, STING-associated vasculopathy with onset in infancy (SAVI), Aicardi-Goutieres syndrome (AGS), familial chilblain lupus (FCL), systemic lupus erythematosus (SLE), retinal vasculopathy, neuroinflammation, systemic inflammatory response syndrome, pancreatitis, cardiovascular disease, renal fibrosis, stroke and age-related macular degeneration (AMD).

This disclosure also provides a method of modulating the STING protein in a subject, the method comprising administering, to a subject in need of such treatment, a therapeutically effective amount of a compound of formula (I), or a pharmaceutically acceptable complex, salt, solvate, tautomeric form or polymorphic form thereof.

Preferably, the method comprises inhibiting the STING protein.

Preferably, the method is a method of inhibiting, or inactivating, the STING protein.

This disclosure also provides a method of treating, ameliorating or preventing a disease selected from liver fibrosis, fatty liver disease, non-alcoholic steatohepatitis (NASH), pulmonary fibrosis, lupus, sepsis, rheumatoid arthritis (RA), type I diabetes, STING-associated vasculopathy with onset in infancy (SAVI), Aicardi-Goutieres syndrome (AGS), familial chilblain lupus (FCL), systemic lupus erythematosus (SLE), retinal vasculopathy, neuroinflammation, systemic inflammatory response syndrome, pancreatitis, cardiovascular disease, renal fibrosis, stroke and age-related macular degeneration (AMD); the method comprising administering, to a subject in need of such treatment, a therapeutically effective amount of a compound of formula (I), or a pharmaceutically acceptable complex, salt, solvate, tautomeric form or polymorphic form thereof.

It may be appreciated that the term "preventing" can mean "reducing the likelihood of". In one preferred embodiment, the disease is fibrosis. The fibrosis may be selected from the group consisting of liver fibrosis, pulmonary fibrosis or renal fibrosis. In some embodiments, the fibrosis patient may have upregulated STING expression and /or STING activity in a tissue compared to that of a healthy subject.

In an alternative preferred embodiment, the disease is fatty liver disease. The fatty liver disease may be non-alcoholic (or simple) fatty liver or non-alcoholic steatohepatitis (NASH).

The following definitions are used in connection with the compounds of the present invention unless the context indicates otherwise.

Throughout the description and the claims of this specification the word "comprise" and other forms of the word, such as "comprising" and "comprises," means including but not limited to, and is not intended to exclude for example, other additives, components, integers, or steps.

"Optional" or "optionally" means that the subsequently described event, operation or circumstances can or cannot occur, and that the description includes instances where the event, operation or circumstance occurs and instances where it does not.

The term "alkyl" as used herein, unless otherwise specified, refers to a saturated straight or branched hydrocarbon. In certain embodiments, the alkyl group is a primary, secondary, or tertiary hydrocarbon. In certain embodiments, the alkyl group includes one to six carbon atoms, *i.e.* C₁-C₆ alkyl. C₁-C₆ alkyl includes for example methyl, ethyl, n-propyl (1-propyl) and isopropyl (2-propyl, 1-methylethyl), butyl, pentyl, hexyl, isobutyl, *sec*-butyl, *tert*-butyl, *iso*pentyl, neopentyl and *iso*hexyl. An alkyl group can be unsubstituted or substituted with one or more of halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. Accordingly, it will be appreciated that an optionally substituted C₁-C₆ alkyl may be an optionally substituted C₁-C₆ haloalkyl, *i.e.* a C₁-C₆ alkyl substituted with at least one halogen, and optionally further substituted with one or more of OR²⁰, optionally substituted C₁-C₆ alkoxy, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. The optionally substituted C₁-C₆ alkyl may be a polyfluoroalkyl, preferably a C₁-C₃ polyfluoroalkyl.

R²⁰ and R²¹ may each independently be selected from the group consisting of H, halogen, OH, CN, COOH, CONH₂, NH₂, NHCOH, optionally substituted C₁-C₆ alkyl, optionally substituted C₁-C₆ alkylsulfonyl, optionally substituted mono or bicyclic C₃-C₆ cycloalkyl, optionally substituted C₂-C₆ alkenyl, optionally substituted C₂-C₆ alkynyl, optionally substituted C₁-C₆ alkoxy, optionally substituted C₁-C₆ alkoxycarbonyl group, mono or bicyclic optionally substituted C₆-C₁₂ aryl, mono or bicyclic optionally substituted 5 to 10 membered heteroaryl, optionally substituted mono or bicyclic 3 to 8 membered heterocycle, optionally substituted aryloxy, optionally substituted heteroaryloxy and optionally substituted heterocyclyloxy. R²⁰ and R²¹ may each independently be selected from the group consisting of H and halogen.

The term "alkylene", as used herein, unless otherwise specified, refers to a bivalent saturated straight or branched hydrocarbon. In certain embodiments, the alkylene group is a primary, secondary, or tertiary hydrocarbon. In certain embodiments, the alkylene group includes one to six carbon atoms, *i.e.* C₁-C₆ alkylene. C₁-C₆ alkylene includes for example methylene, ethylene, n-propylene and isopropylene, butylene, pentylene, hexylene, *iso*butylene, *sec*-butylene, *tert*-butylene, *iso*pentylene, neopentylene, and isohexylene. An alkylene group may be as defined above in relation the alkyl group, but with a hydrogen atom removed therefrom to cause the group to be bivalent. It may be appreciated that if an alkylene is described, for example, as eth-1,1-ylene then both attachment points to the rest of the structure are in the 1 position.

The term "halo" or "halogen" includes fluoro (-F), chloro (-Cl), bromo (-Br) and iodo (-I).

The term "polyfluoroalkyl" may denote a C₁-C₃ alkyl group in which two or more hydrogen atoms are replaced by fluorine atoms. The term may include perfluoroalkyl groups, *i.e.* a C₁-C₃ alkyl group in which all the hydrogen atoms are replaced by fluorine atoms. Accordingly, the term C₁-C₃ polyfluoroalkyl includes, but is not limited to, difluoromethyl, trifluoromethyl, 2,2,2-trifluoroethyl, pentafluoroethyl, 3,3,3-trifluoropropyl, 2,2,3,3,3-pentafluoropropyl, and 2,2,2-trifluoro-1-(trifluoromethyl)ethyl.

"Alkoxy" refers to the group R²²-O-, where R²² is an optionally substituted C₁-C₆ alkyl group, an optionally substituted C₃-C₆ cycloalkyl group, an optionally substituted C₂-C₆ alkenyl or an optionally substituted C₂-C₆ alkynyl. Exemplary C₁-C₆ alkoxy groups include but are not limited to methoxy, ethoxy, n-propoxy (1-propoxy), n-butoxy and *tert*-butoxy*.* An alkoxy group can be unsubstituted or substituted with one or more of halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. R²⁰ and R²¹ may be as defined above. R²⁰ and R²¹ may each independently be selected from the group consisting of H, halogen and optionally substituted C₁-C₆ alkyl.

"Aryl" refers to an aromatic 6 to 12 membered hydrocarbon group. The term includes bicyclic groups where one of the rings is aromatic and the other is not. Examples of a C₆-C₁₂ aryl group include, but are not limited to, phenyl, α-naphthyl, β-naphthyl, biphenyl, tetrahydronaphthyl and indanyl. An aryl group can be unsubstituted or substituted with one or more of optionally substituted C₁-C₆ alkyl, optionally substituted C₂-C₆ alkenyl, optionally substituted C₂-C₆ alkynyl,optionally substituted C₁-C₆ alkoxy, halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. R²⁰ and R²¹ may be as defined above. R²⁰ and R²¹ may each independently be selected from the group consisting of H, halogen and optionally substituted C₁-C₆ alkyl.

"Arylene" refers to a bivalent aromatic 6 to 10 membered hydrocarbon group. An arylene group may be as defined above in relation the aryl group, but with a hydrogen atom removed therefrom to cause the group to be bivalent.

The term "bicycle" or "bicyclic" as used herein refers to a molecule that features two fused rings, which rings are a cycloalkyl, heterocyclyl, or heteroaryl. In one embodiment, the rings are fused across a bond between two atoms. The bicyclic moiety formed therefrom shares a bond between the rings. In another embodiment, the bicyclic moiety is formed by the fusion of two rings across a sequence of atoms of the rings to form a bridgehead. Similarly, a "bridge" is an unbranched chain of one or more atoms connecting two bridgeheads in a polycyclic compound. In another embodiment, the bicyclic molecule is a "spiro" or "spirocyclic" moiety. The spirocyclic group may be a C₃-C₆ cycloalkyl or a mono or bicyclic 3 to 8 membered heterocycle which is bound through a single carbon atom of the spirocyclic moiety to a single carbon atom of a carbocyclic or heterocyclic moiety. In one embodiment, the spirocyclic group is a cycloalkyl and is bound to another cycloalkyl. In another embodiment, the spirocyclic group is a cycloalkyl and is bound to a heterocyclyl. In a further embodiment, the spirocyclic group is a heterocyclyl and is bound to another heterocyclyl. In still another embodiment, the spirocyclic group is a heterocyclyl and is bound to a cycloalkyl. A spirocyclic group can be unsubstituted or substituted with one or more of optionally substituted C₁-C₆ alkyl, optionally substituted C₂-C₆ alkenyl, optionally substituted C₂-C₆ alkynyl, optionally substituted C₁-C₆ alkoxy, halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. R²⁰ and R²¹ may be as defined above. R²⁰ and R²¹ may each independently be selected from the group consisting of H, halogen and optionally substituted C₁-C₆ alkyl.

"Cycloalkyl" refers to a non-aromatic, saturated, partially saturated, monocyclic, bicyclic or polycyclic hydrocarbon 3 to 6 membered ring system. Representative examples of a C₃-C₆ cycloalkyl include, but are not limited to, cyclopropyl, cyclobutyl, cyclopentyl, cyclohexyl. A cycloalkyl group can be unsubstituted or substituted with one or more of optionally substituted C₁-C₆ alkyl, optionally substituted C₂-C₆ alkenyl, optionally substituted C₂-C₆ alkynyl, optionally substituted C₁-C₆ alkoxy, halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. R²⁰ and R²¹ may be as defined above. R²⁰ and R²¹ may each independently be selected from the group consisting of H, halogen and optionally substituted C₁-C₆ alkyl.

"Cycloalkylene" refers to a bivalent non-aromatic, saturated, partially saturated, monocyclic, bicyclic or polycyclic hydrocarbon 3 to 6 membered ring system. A cycloalkylene group may be as defined above in relation to the cycloalkyl group, but with a hydrogen atom removed therefrom to cause the group to be bivalent.

"Heteroaryl" refers to a monocyclic or bicyclic aromatic 5 to 10 membered ring system in which at least one ring atom is a heteroatom. The term includes bicyclic groups where one of the rings is aromatic and the other is not. The or each heteroatom may be independently selected from the group consisting of oxygen, sulfur and nitrogen. Examples of 5 to 10 membered heteroaryl groups include furan, thiophene, indole, azaindole, oxazole, thiazole, isoxazole, isothiazole, imidazole, N-methylimidazole, pyridine, pyrimidine, pyrazine, pyrrole, N-methylpyrrole, pyrazole, N-methylpyrazole, 1,3,4-oxadiazole, 1,2,4-triazole, 1- methyl-1,2,4-triazole, 1H-tetrazole, 1-methyltetrazole, benzoxazole, benzothiazole, benzofuran, benzisoxazole, benzimidazole, N-methylbenzimidazole, azabenzimidazole, indazole, quinazoline, quinoline, and isoquinoline. Bicyclic 5 to 10 membered heteroaryl groups include those where a phenyl, pyridine, pyrimidine, pyrazine or pyridazine ring is fused to a 5 or 6-membered monocyclic heteroaryl ring. A heteroaryl group can be unsubstituted or substituted with one or more of optionally substituted C₁-C₆ alkyl, optionally substituted C₂-C₆ alkenyl, optionally substituted C₂-C₆ alkynyl, optionally substituted C₁-C₆ alkoxy, halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. R²⁰ and R²¹ may be as defined above. R²⁰ and R²¹ may each independently be selected from the group consisting of H, halogen and optionally substituted C₁-C₆ alkyl.

"Heterocycle" or "heterocyclyl" refers to 3 to 8 membered monocyclic, bicyclic or bridged molecules in which at least one ring atom is a heteroatom. The or each heteroatom may be independently selected from the group consisting of oxygen, sulfur and nitrogen. A heterocycle may be saturated or partially saturated. Exemplary 3 to 8 membered heterocycle groups include but are not limited to aziridine, oxirane, oxirene, thiirane, pyrroline, pyrrolidine, dihydrofuran, tetrahydrofuran, dihydrothiophene, tetrahydrothiophene, dithiolane, piperidine, 1,2,3,6-tetrahydropyridine-1-yl, tetrahydropyran, pyran, morpholine, piperazine, thiane, thiine, piperazine, azepane, diazepane and oxazine. A heterocycle group can be unsubstituted or substituted with one or more of optionally substituted C₁-C₆ alkyl, optionally substituted C₂-C₆ alkenyl, optionally substituted C₂-C₆ alkynyl, optionally substituted C₁-C₆ alkoxy, halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. R²⁰ and R²¹ may be as defined above. R²⁰ and R²¹ may each independently be selected from the group consisting of H, halogen and optionally substituted C₁-C₆ alkyl.

"Heterocyclylene" refers to a bivalent 3 to 8 membered monocyclic, bicyclic or bridged molecules in which at least one ring atom is a heteroatom. A heterocyclylene group may be as defined above in relation to the heterocyclyl group, but with a hydrogen atom removed therefrom to cause the group to be bivalent.

"Alkenyl" refers to an olefinically unsaturated hydrocarbon groups which can be unbranched or branched. In certain embodiments, the alkenyl group has 2 to 6 carbons, i.e. it is a C₂-C₆ alkenyl. C₂-C₆ alkenyl includes for example vinyl, allyl, propenyl, butenyl, pentenyl and hexenyl. An alkenyl group can be unsubstituted or substituted with one or more of optionally substituted C₂-C₆ alkynyl, optionally substituted C₁-C₆ alkoxy, halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. R²⁰ and R²¹ may be as defined above. R₂₀ and R²¹ may each independently be selected from the group consisting of H, halogen and optionally substituted C₁-C₆ alkyl.

"Alkynyl" refers to an acetylenically unsaturated hydrocarbon groups which can be unbranched or branched. In certain embodiments, the alkynyl group has 2 to 6 carbons, *i.e.* it is a C₂-C₆ alkynyl. C₂-C₆ alkynyl includes for example propargyl, propynyl, butynyl, pentynyl and hexynyl. An alkynyl group can be unsubstituted or substituted with one or more of optionally substituted C₂-C₆ alkenyl, optionally substituted C₁-C₆ alkoxy, halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. R²⁰ and R²¹ may be as defined above. R²⁰ and R²¹ may each independently be selected from the group consisting of H, halogen and optionally substituted C₁-C₆ alkyl.

The term "alkenylene", as used herein, unless otherwise specified, refers to a bivalent olefinically unsaturated straight or branched hydrocarbon. An alkenylene group may be as defined above in relation the alkenyl group, but with a hydrogen atom removed therefrom to cause the group to be bivalent. It may be appreciated that if an alkenylene is described, for example, as ethen-1,1-ylene then both attachment points to the rest of the structure are in the 1 position.

The term "alkynylene", as used herein, unless otherwise specified, refers to a bivalent acetylenically unsaturated straight or branched hydrocarbon. An alkynylene group may be as defined above in relation the alkynyl group, but with a hydrogen atom removed therefrom to cause the group to be bivalent. It may be appreciated that if an alkynylene is described, for example, as ethyn-1,1-ylene then both attachment points to the rest of the structure are in the 1 position.

"Alkylsulfonyl" refers to the group alkyl-SO₂- where alkyl is an optionally substituted C₁-C₆ alkyl, and is as defined as above.

"Alkoxycarbonyl" refers to the group alkyl-O-C(O)-, where alkyl is an optionally substituted C₁-C₆ alkyl. An alkoxycarbonyl group can be unsubstituted or substituted with one or more of optionally substituted C₂-C₆ alkenyl, optionally substituted C₂-C₆ alkynyl, optionally substituted C₁-C₆ alkoxy, halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle.

"Aryloxy" refers to the group Ar-O- where Ar is a mono or bicyclic optionally substituted C₆-C₁₂ aryl group, as defined above.

"Heteroaryloxy" refers to the group heteroaryl-O- where the heteroaryl is a mono or bicyclic optionally substituted 5 to 10 membered heteroaryl, and is as defined above.

"Heterocyclyloxy" refers to the group heterocycle-O- where heterocycle is an optionally substituted mono or bicyclic 3 to 8 membered heterocycle, and is as defined as above.

A complex of the compound of formula **(I)** may be understood to be a multi-component complex, wherein the drug and at least one other component are present in stoichiometric or non-stoichiometric amounts. The complex may be other than a salt or solvate. Complexes of this type include clathrates (drug-host inclusion complexes) and co-crystals. The latter are typically defined as crystalline complexes of neutral molecular constituents which are bound together through non-covalent interactions, but could also be a complex of a neutral molecule with a salt. Co-crystals may be prepared by melt crystallisation, by recrystallisation from solvents, or by physically grinding the components together - see Chem Commun, 17, 1889-1896, by O. Almarsson and M. J. Zaworotko (2004**),** incorporated herein by reference. For a general review of multi-component complexes, see J Pharm Sci, 64 (8), 1269-1288, by Haleblian (August 1975**),** incorporated herein by reference.

The term "pharmaceutically acceptable salt" may be understood to refer to any salt of a compound provided herein which retains its biological properties and which is not toxic or otherwise undesirable for pharmaceutical use. Such salts may be derived from a variety of organic and inorganic counter-ions well known in the art. Such salts include, but are not limited to: (1) acid addition salts formed with organic or inorganic acids such as hydrochloric, hydrobromic, sulfuric, nitric, phosphoric, sulfamic, acetic, adepic, aspartic, trifluoroacetic, trichloroacetic, propionic, hexanoic, cyclopentylpropionic, glycolic, glutaric, pyruvic, lactic, malonic, succinic, sorbic, ascorbic, malic, maleic, fumaric, tartaric, citric, benzoic, 3-(4-hydroxybenzoyl)benzoic, picric, cinnamic, mandelic, phthalic, lauric, methanesulfonic, ethanesulfonic, 1,2-ethane-disulfonic, 2-hydroxyethanesulfonic, benzenesulfonic, 4-chlorobenzenesulfonic, 2-naphthalenesulfonic, 4-toluenesulfonic, camphoric, camphorsulfonic, 4-methylbicyclo[2.2.2]-oct-2-ene-1-carboxylic, glucoheptonic, 3-phenylpropionic, trimethylacetic, tert-butylacetic, lauryl sulfuric, gluconic, benzoic, glutamic, hydroxynaphthoic, salicylic, stearic, cyclohexylsulfamic, quinic, muconic acid and the like acids; or (2) base addition salts formed when an acidic proton present in the parent compound either (a) is replaced by a metal ion, e.g., an alkali metal ion, an alkaline earth ion or an aluminium ion, or alkali metal or alkaline earth metal hydroxides, such as sodium, potassium, calcium, magnesium, aluminium, lithium, zinc, and barium hydroxide, ammonia or (b) coordinates with an organic base, such as aliphatic, alicyclic, or aromatic organic amines, such as ammonia, methylamine, dimethylamine, diethylamine, picoline, ethanolamine, diethanolamine, triethanolamine, ethylenediamine, lysine, arginine, ornithine, choline, N,N'-dibenzylethylene-diamine, chloroprocaine, diethanolamine, procaine, N-benzylphenethylamine, N-methylglucamine piperazine, tris(hydroxymethyl)-aminomethane, tetramethylammonium hydroxide, and the like.

Pharmaceutically acceptable salts may include, sodium, potassium, calcium, magnesium, ammonium, tetraalkylammonium and the like, and when the compound contains a basic functionality, salts of non-toxic organic or inorganic acids, such as hydrohalides, e.g. hydrochloride, hydrobromide and hydroiodide, carbonate or bicarbonate, sulfate or bisulfate, borate, phosphate, hydrogen phosphate, dihydrogen phosphate, pyroglutamate, saccharate, stearate, sulfamate, nitrate, orotate, oxalate, palmitate, pamoate, acetate, trifluoroacetate, trichloroacetate, propionate, hexanoate, cyclopentylpropionate, glycolate, glutarate, pyruvate, lactate, malonate, succinate, tannate, tartrate, tosylate, sorbate, ascorbate, malate, maleate, fumarate, tartarate, camsylate, citrate, cyclamate, benzoate, isethionate, esylate, formate, 3-(4-hydroxybenzoyl)benzoate, picrate, cinnamate, mandelate, phthalate, laurate, methanesulfonate (mesylate), methylsulphate, naphthylate, 2-napsylate, nicotinate, ethanesulfonate, 1,2-ethane-disulfonate, 2-hydroxyethanesulfonate, benzenesulfonate (besylate), 4-chlorobenzenesulfonate, 2-naphthalenesulfonate, 4-toluenesulfonate, camphorate, camphorsulfonate, 4-methylbicyclo[2.2.2]-oct-2-ene-1-carboxylate, glucoheptonate, 3-phenylpropionate, trimethylacetate, tert-butylacetate, lauryl sulfate, gluceptate, gluconate, glucoronate, hexafluorophosphate, hibenzate, benzoate, glutamate, hydroxynaphthoate, salicylate, stearate, cyclohexylsulfamate, quinate, muconate, xinofoate and the like.

Hemisalts of acids and bases may also be formed, for example, hemisulphate salts.

The skilled person will appreciate that the aforementioned salts include ones wherein the counterion is optically active, for example D-lactate, or racemic, for example DL-tartrate.

For a review on suitable salts, see "Handbook of Pharmaceutical Salts: Properties, Selection, and Use" by Stahl and Wermuth (Wiley-VCH, Weinheim, Germany, 2002).

Pharmaceutically acceptable salts of compounds of formula **(I)** may be prepared by one or more of three methods:
(i) by reacting the compound of formula **(I)** with the desired acid or base;
(ii) by removing an acid- or base-labile protecting group from a suitable precursor of the compound of formula **(I)** using the desired acid or base; or
(iii) by converting one salt of the compound of formula **(I)** to another by reaction with an appropriate acid or base or by means of a suitable ion exchange column.

All three reactions are typically carried out in solution. The resulting salt may precipitate out and be collected by filtration or may be recovered by evaporation of the solvent. The degree of ionisation in the resulting salt may vary from completely ionised to almost non-ionised.

The term "solvate" may be understood to refer to a compound provided herein or a salt thereof, that further includes a stoichiometric or non-stoichiometric amount of solvent bound by non-covalent intermolecular forces. Where the solvent is water, the solvate is a hydrate. Pharmaceutically acceptable solvates in accordance with the invention include those wherein the solvent of crystallization may be isotopically substituted, e.g. D₂O, d₆-acetone and d₆-DMSO.

A currently accepted classification system for organic hydrates is one that defines isolated site, channel, or metal-ion coordinated hydrates - see Polymorphism in Pharmaceutical Solids by K. R. Morris (Ed. H. G. Brittain, Marcel Dekker, 1995), incorporated herein by reference. Isolated site hydrates are ones in which the water molecules are isolated from direct contact with each other by intervening organic molecules. In channel hydrates, the water molecules lie in lattice channels where they are next to other water molecules. In metal-ion coordinated hydrates, the water molecules are bonded to the metal ion.

When the solvent or water is tightly bound, the complex will have a well-defined stoichiometry independent of humidity. When, however, the solvent or water is weakly bound, as in channel solvates and hygroscopic compounds, the water/solvent content will be dependent on humidity and drying conditions. In such cases, non-stoichiometry will be the norm.

The compounds of the invention may exist in a continuum of solid states ranging from fully amorphous to fully crystalline, including polymorphs of said crystalline material. The term 'amorphous' refers to a state in which the material lacks long range order at the molecular level and, depending upon temperature, may exhibit the physical properties of a solid or a liquid. Typically such materials do not give distinctive X-ray diffraction patterns and, while exhibiting the properties of a solid, are more formally described as a liquid. Upon heating, a change from solid to liquid properties occurs which is characterised by a change of state, typically second order ('glass transition'). The term 'crystalline' refers to a solid phase in which the material has a regular ordered internal structure at the molecular level and gives a distinctive X-ray diffraction pattern with defined peaks. Such materials when heated sufficiently will also exhibit the properties of a liquid, but the change from solid to liquid is characterised by a phase change, typically first order ('melting point').

The compounds of the invention may also exist in a mesomorphic state (mesophase or liquid crystal) when subjected to suitable conditions. The mesomorphic state is intermediate between the true crystalline state and the true liquid state (either melt or solution). Mesomorphism arising as the result of a change in temperature is described as 'thermotropic' and that resulting from the addition of a second component, such as water or another solvent, is described as 'lyotropic'. Compounds that have the potential to form lyotropic mesophases are described as 'amphiphilic' and consist of molecules which possess an ionic (such as -COO⁻Na⁺, -COO⁻K⁺, or -SO₃⁻Na⁺) or non-ionic (such as -N⁻N⁺(CH₃)₃) polar head group. For more information, see Crystals and the Polarizing Microscope by N. H. Hartshorne and A. Stuart, 4th Edition (Edward Arnold, 1970), incorporated herein by reference.

Compounds of formula **(I)** may include one or more stereogenic centers and so may exist as optical isomers, such as enantiomers and diastereomers. All such isomers and mixtures thereof are included within the scope of the present invention.

It will be understood that the above compounds may exist as enantiomers and as diastereoisomeric pairs. These isomers also represent further embodiments of the invention.

Conventional techniques for the preparation/isolation of individual enantiomers include chiral synthesis from a suitable optically pure precursor or resolution of the racemate (or the racemate of a salt or derivative) using, for example, chiral high pressure liquid chromatography (HPLC).

Alternatively, the racemate (or a racemic precursor) may be reacted with a suitable optically active compound, for example, an alcohol, or, in the case where the compound of formula (I) contains an acidic or basic moiety, a base or acid such as 1-phenylethylamine or tartaric acid. The resulting diastereomeric mixture may be separated by chromatography and/or fractional crystallization and one or both of the diastereoisomers converted to the corresponding pure enantiomer(s) by means well known to a skilled person.

Chiral compounds of the invention (and chiral precursors thereof) may be obtained in enantiomerically-enriched form using chromatography, typically HPLC, on an asymmetric resin with a mobile phase consisting of a hydrocarbon, typically heptane or hexane, containing from 0 to 50% by volume of isopropanol, typically from 2% to 20%, and from 0 to 5% by volume of an alkylamine, typically 0.1% diethylamine. Concentration of the eluate affords the enriched mixture.

Mixtures of stereoisomers may be separated by conventional techniques known to those skilled in the art; see, for example, "Stereochemistry of Organic Compounds" by E. L. Eliel and S. H. Wilen (Wiley, New York, 1994).

The term 'STING' refers to STimulator of INterferon Genes, an adaptor protein that is functionally activated by cyclic dinucleotides which leads to the production of interferons and inflammatory cytokines.

It will be appreciated that an 'antagonist', or 'inhibitor' as it relates to a ligand and STING, comprises a molecule, combination of molecules, or a complex, that inhibits, counteracts, downregulates, and/or desensitizes STING activity. 'Antagonist' encompasses any reagent that inhibits a constitutive activity of STING. A constitutive activity is one that is manifest in the absence of a ligand/STING interaction. 'Antagonist' also encompasses any reagent that inhibits or prevents a stimulated (or regulated) activity of STING.

Preferably, the compound of formula **(I)** is an inhibitor of the STING protein.

R¹ may be H, halogen, OH, CN or optionally substituted C₁-C₆ alkyl. R¹ may be H, halogen, OH, CN or C₁-C₃ alkyl. Preferably, R¹ is H.

In some embodiments, R² is -A-NR¹⁷-C(O)-NR¹⁸-R¹⁵. In alternative embodiments, R³ is -A-NR¹⁷-C(O)-NR¹⁸-R¹⁵. Accordingly, the compound may be a compound of Formula **(Ia)** or Formula **(Ib):**

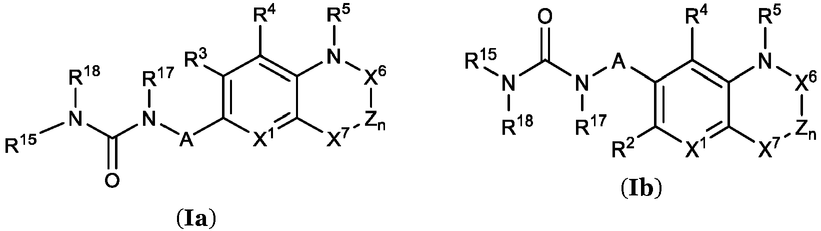

Preferably, one of R² and R³ is -A-NR¹⁷-C(O)-NR¹⁸-R¹⁵ and the other of R² and R³ is H, halogen, OH, CN, COOR¹³, CONR¹³R¹⁴, NR¹³R¹⁴, NR¹³COR¹⁴, optionally substituted C₁-C₆ alkyl, optionally substituted C₂-C₆ alkenyl or optionally substituted C₂-C₆ alkynyl, and R¹³ and R¹⁴ are each independently selected from the group consisting of H, optionally substituted C₁-C₃ alkyl, optionally substituted C₂-C₃ alkenyl and optionally substituted C₂-C alkynyl. More preferably, one of R² and R³ is -A-NR¹⁷-C(O)-NR¹⁸-R¹⁵ and the other of R² and R³ is H, halogen, OH, CN, CONR¹³R¹⁴, NR¹³R¹⁴, C₁-C₃ alkyl, C₂-C₃ alkenyl or C₂-C₃ alkynyl, and R¹³ and R¹⁴ are each independently selected from the group consisting of H, C₁-C₃ alkyl, C₂-C₃ alkenyl and C₂-C alkynyl. Preferably, one of R² and R³ is -A-NR¹⁷-C(O)-NR¹⁸-R¹⁵ and the other of R² and R³ is H, bromine or CONH₂. In a preferred embodiment, one of R² and R³ is -A-NR¹⁷-C(O)-NR¹⁸-R¹⁵ and the other of R² and R³ is H.

A may be an optionally substituted methylene, an optionally substituted ethylene, an optionally substituted propylene, an optionally substituted butylene, an optionally substituted pentylene, an optionally substituted hexylene, an optionally substituted ethenylene, an optionally substituted propenylene, an optionally substituted butenylene, an optionally substituted pentenylene, an optionally substituted hexenylene, an optionally substituted ethynylene, an optionally substituted propynylene, an optionally substituted butynylene, an optionally substituted pentynylene or an optionally substituted hexynylene. A may be an optionally substituted methylene, an optionally substituted eth-1,1-ylene, an optionally substituted prop-1,1-ylene, an optionally substituted but-1,1-ylene, an optionally substituted pent-1,1-ylene, an optionally substituted hex-1,1-ylene, an optionally substituted ethen-1,1-ylene, an optionally substituted propen-1,1-ylene, an optionally substituted buten-1,1-ylene, an optionally substituted penten-1,1-ylene, an optionally substituted hexen-1,1-ylene, an optionally substituted propyn-1,1-ylene, an optionally substituted butyn-1,1-ylene, an optionally substituted pentyn-1,1-ylene or an optionally substituted hexyn-1,1-ylene. In some embodiments, A is an optionally substituted methylene, an optionally substituted eth-1,1-ylene, an optionally substituted prop-1,1-ylene, an optionally substituted but-1,1-ylene or an optionally substituted prop-2-en-1,1-ylene.

The alkylene, alkenylene or alkynylene may be unsubstituted or substituted with one or more of halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl or optionally substituted 3 to 8 membered heterocycle. Preferably, the alkylene, alkenylene or alkynylene is unsubstituted or substituted with one or more of OR²⁰, oxo, COOR²⁰, CONR²⁰R²¹ optionally substituted 5 or 6 membered heteroaryl or optionally substituted 5 or 6 membered heterocycle. R²⁰ and R²¹ may each independently be H, optionally substituted C₁-C₆ alkyl, optionally substituted C₂-C₆ alkenyl, optionally substituted C₂-C₆ alkynyl, optionally substituted C₁-C₆ alkoxy, optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle. The alkyl, alkenyl, alkynyl or alkoxy may be unsubstituted or substituted with one or more of halogen, OH, CN or C₁₋₆ alkoxy. Preferably R²⁰ and R²¹ are each independently H, methyl, OCH₃, CH₂CH₂OH, CH₂CH₂OCH₃, cyclopropyl or pyridinyl.

When A is substituted, directly or indirectly, with an optionally substituted aryl, the optionally substituted aryl may be optionally substituted phenyl. When A is substituted, directly or indirectly, with an optionally substituted heteroaryl, the optionally substituted heteroaryl may be optionally substituted 1H-pyrrolyl, optionally substituted pyrazolyl, optionally substituted imidazolyl, optionally substituted 1,2,3-triazolyl, optionally substituted 1,2,4-triazolyl, optionally substituted tetrazolyl, optionally substituted furanyl, optionally substituted thiophenyl, optionally substituted oxazolyl, optionally substituted isoxazolyl, optionally substituted iosthiazolyl, optionally substituted thiazolyl optionally substituted pyridinyl, optionally substituted pyridazinyl, optionally substituted pyridazinyl, optionally substituted pyrimidineyl, optionally substituted pyrazinyl, optionally substituted 1,2,4-triazinyl or optionally substituted 1,2,5-triazinyl. When A is substituted, directly or indirectly, with an optionally substituted cycloalkyl, the optionally substituted cycloalkyl may be optionally substituted cyclopropyl, optionally substituted cyclobutyl, optionally substituted cyclopentyl or optionally substituted cyclohexyl. When A is substituted, directly or indirectly, with an optionally substituted heterocycle, the optionally substituted heterocycle may be optionally substituted pyrrolidinyl, optionally substituted pyrazolidinyl, optionally substituted imidazolinyl, optionally substituted tetrahydrofuranyl, optionally substituted tetrahydrothiophenyl, optionally substituted piperidinyl, optionally substituted piperazinyl, optionally substituted tetrahydropyranyl, optionally substituted 1,3-dioxanyl, optionally substituted 1,4-dioxanyl, optionally substituted thianyl, optionally substituted 1,3-dithianyl, optionally substituted 1,4-dithianyl or optionally substituted morpholinyl. The aryl, heteroaryl, cycloalkyl or the heterocycle may be unsubstituted or substituted with one or more of halogen, OH, CN or C₁₋₆ alkyl. Preferably, the aryl, heteroaryl, cycloalkyl or the heterocycle is unsubstituted or substituted with OH.

In some embodiments, A may be -CH₂-, 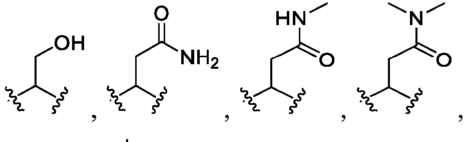 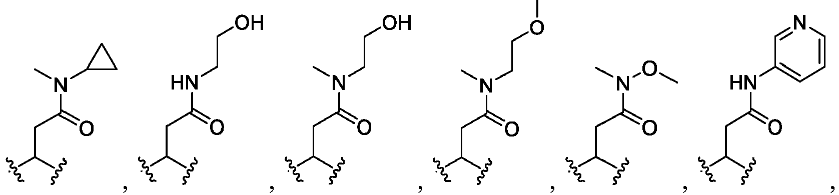 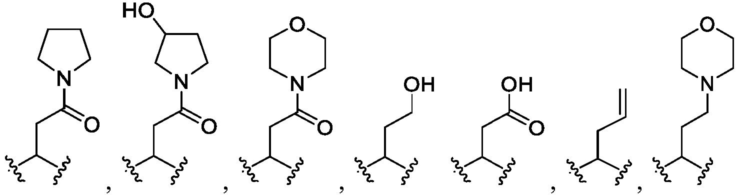 or

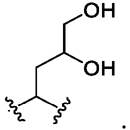

R¹⁷ and R¹⁸ may independently be H, C₁-C₃ alkyl or C₂-C₃ alkenyl. Preferably, R¹⁷ and R¹⁸ are H or methyl. Most preferably, R¹⁷ and R¹⁸ are H.

In embodiments where R¹⁵ is an aryl it may be an optionally substituted phenyl, an optionally substituted 5,6,7,8-tetrahydronaphthalenyl or an optionally substituted 2,3-dihydro-1H-indenyl. In embodiments where R¹⁵ is an optionally substituted 5 to 10 membered heteroaryl, it may be optionally substituted pyrrolyl, optionally substituted furanyl, optionally substituted thiophenyl, optionally substituted oxazolyl, optionally substituted thiazolyl, optionally substituted isoxazolyl, optionally substituted isothiazolyl, optionally substituted imidazolyl, optionally substituted pyrazolyl, optionally substituted pyridinyl, optionally substituted pyridazinyl, optionally substituted pyrimidinyl, optionally substituted pyrazinyl, optionally substituted indolinyl, optionally substituted 1H-indolyl, optionally substituted 7-azaindolyl, optionally substituted 1H-pyrrolo[3,2-b]pyridinyl, optionally substituted benzofuranyl, optionally substituted azaindolyl, optionally substituted benzisoxazolyl, optionally substituted azabenzimidazolyl, optionally substituted indazolyl, optionally substituted benzo[b]thiophenyl, optionally substituted benzimidazolyl, optionally substituted, benzo[d]oxazolyl, optionally substituted benzo[d]thiazolyl, optionally substituted 1,4-benzodioxanyl, optionally substituted 1,2,3,4-tetrahydroquinolinyl, optionally substituted quinazolinyl, optionally substituted quinolinyl, optionally substituted isoquinolinyl, optionally substituted 1,2,3,4-tetrahydroisoquinolinyl, optionally substituted 3,4-dihydro-2H-1,4-benzoxazyl or optionally substituted 7,8-dihydropyrido[4,3-d]pyrimidinyl. In embodiments where R¹⁵ is an 3 to 8 membered heterocycle, it may be optionally substituted tetrahydrofuranyl, optionally substituted tetrahydrothiophenyl, optionally substituted pyrrolidinyl, optionally substituted piperidinyl, optionally substituted piperazinyl, optionally substituted tetrahydropyranyl, optionally substituted thianyl, optionally substituted morpholinyl, optionally substituted thiomorpholinyl, optionally substituted 1,2-oxazinyl, optionally substituted 1,3-oxazinyl, optionally substituted 1,4-oxazinyl, optionally substituted azepanyl, optionally substituted 1,2-diazepinyl, optionally substituted 1,3-diazepinyl, optionally substituted 1,4-diazepinyl or optionally substituted 3,4-dihydro-2H-benzo[b][1,4]oxazine. In a preferred embodiment, R¹⁵ is an optionally substituted 1H-indolyl. R¹⁵ may be an optionally substituted 1H-indol-6-yl or an optionally substituted 1H-indol-3-yl.

When R¹⁵ is an optionally substituted aryl, optionally substituted heteroaryl, optionally substituted cycloalkyl or an optionally substituted heterocycle, the heteroaryl, cycloalkyl or heterocycle may be unsubstituted or substituted with one or more substituents selected from the group consisting of optionally substituted C₁-C₆ alkyl, halogen, OH, oxo, OP(O)(OR²⁰)(OR²¹), optionally substituted C₁-C₆ alkoxy, NR²⁰R²¹, CONR²⁰R²¹, CN, C(O)R²⁰, COOR²⁰, NO₂, azido, SO₂R²⁰, C(O)R²⁰ and NR²⁰COR²¹. When the heteroaryl, cycloalkyl or heterocycle is substituted with an optionally substituted alkyl, the alkyl may be unsubstituted or substituted with one or more substituents selected from the group consisting of halogen, OH, C₁-C₆ alkoxy, NR²⁰R²¹, C(O)R²⁰, CN, oxo, OP(O)(OR²⁰)(OR²¹), OC(O)R²⁰, COOR²⁰, CONR²⁰R²¹, C₁-C₆ alkenyl, C₁-C₆ alkynyl, =NOR²⁰, NR²⁰C(O)R²¹, SO₂R²⁰ and SO₂NR²⁰R²¹. Halogen may be F or Cl. Preferably, halogen is F. R²⁰ and R²¹ may independently be H or methyl. Accordingly, the heteroaryl, cycloalkyl or heterocycle may be substituted with one or more substituents selected from the group consisting of F, oxo, CN, NH₂, C(O)CH₃, CONH₂, CH₃ and CH₂COOH.

R⁴ may be H, halogen, OH, CN or optionally substituted C₁-C₆ alkyl. R⁴ may be H, halogen, OH, CN or C₁-C₃ alkyl. Preferably, R⁴ is H.

R⁵ may be -L¹-L²-R¹⁶.

Preferably, L¹ is absent, an optionally substituted C₁-C₃ alkylene, an optionally substituted C₂-C₃ alkenylene or an optionally substituted C₂-C₃ alkynylene. The alkylene, alkenylene or alkynylene may be unsubstituted or substituted with one or more of halogen, OH, CN, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹ and oxo. R²⁰ and R²¹ may be independently be H, optionally substituted C₁-C₃ alkyl, optionally substituted C₂-C₃ alkenyl, optionally substituted C₂-C₃ alkynyl, optionally substituted mono or bicyclic C₃-C₆ cycloalkyl or optionally substituted mono or bicyclic 3 to 8 membered heterocycle. Preferably, R²⁰ and R²¹ are independently H, methyl or cyclopropyl. Preferably, L¹ is absent, CH₂, CH₂CH₂, CO, 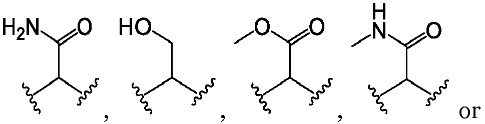 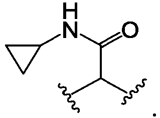 Most preferably, L¹ is absent or CH₂.

In some embodiments, L² is absent.

Alternatively, L² may be O, S, S=O, SO₂ or NR¹⁹. R¹⁹ may be H, an optionally substituted C₁-C₃ alkyl, an optionally substituted C₂-C₃ alkenyl or an optionally substituted C₂-C₃ alkynyl. Preferably, L² is O or S, and most preferably is O.

R¹⁶ may be optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle. Preferably, R¹⁶ is a mono or bicyclic optionally substituted C₆-C₁₂ aryl, a mono or bicyclic optionally substituted 5 to 10 membered heteroaryl or optionally substituted mono or bicyclic 3 to 8 membered heterocycle. Mono or bicyclic optionally substituted C₆-C₁₂ aryl may be optionally substituted phenyl. Optionally substituted mono or bicyclic C₃-C₆ cycloalkyl may be cyclopropyl, cyclobutyl, cyclopentyl or cyclohexyl. Mono or bicyclic optionally substituted 5 to 10 membered heteroaryl may be optionally substituted oxazolyl, optionally substituted thiazolyl, optionally substituted isoxazolyl, optionally substituted isothiazolyl, optionally substituted imidazolyl, optionally substituted pyrazolyl, optionally substituted 1,2,3-oxadiazolyl, optionally substituted 1,2,4-oxadiazolyl, optionally substituted 1,2,5-oxadiazolyl, optionally substituted 1,3,4-oxadiazolyl, optionally substituted pyridinyl, optionally substituted pyridazinyl, optionally substituted pyrimidinyl, optionally substituted pyrazinyl, optionally substituted 1H-indolyl, optionally substituted azaindolyl, optionally substituted benzisoxazolyl, optionally substituted 4-azabenzimidazolyl, optionally substituted 5-benzimidazolyl, optionally substituted indazolyl, optionally substituted benzimidazolyl, optionally substituted benzofuranyl, optionally substituted benzo[b]thiophenyl, optionally substituted benzo[d]isoxazolyl, optionally substituted benzo[d]isothiazolyl, optionally substituted imidazo[1,2-a]pyridinyl, optionally substituted quinazolinyl, optionally substituted quinolinyl, optionally substituted isoquinolinyl, optionally substituted benzothiazole, optionally substituted 1,3-benzodioxolyl, optionally substituted benzofuranyl, optionally substituted 2,1,3-benzothiadiazolyl, optionally substituted 3,4-dihydro-2H,1,4-benzoxazinyl, or optionally substituted benzo-1,4-dioxanyl. Mono or bicyclic 3 to 8 membered heterocycle may be an optionally substituted pyrrolidinyl, optionally substituted tetrahydrofuranyl, optionally substituted tetrahydrothiophenyl, optionally substituted piperidinyl, an optionally substituted piperazinyl, an optionally substituted tetrahydropyranyl, an optionally substituted dioxanyl, an optionally substituted thianyl, an optionally substituted dithianyl or an optionally substituted morpholinyl.

When R¹⁶ is an aryl, heteroaryl, cycloalkyl or heterocycle, the aryl, heteroaryl, cycloalkyl or heterocycle may be unsubstituted or substituted with one or more substituents selected from the group consisting of optionally substituted C₁-C₆ alkyl, optionally substituted C₂-C₆ alkenyl, optionally substituted C₂-C₆ alkynyl, halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl and optionally substituted 3 to 8 membered heterocycle. Halogen may be F or Cl. When the aryl, heteroaryl, cycloalkyl or heterocycle is substituted, directly or indirectly, with an optionally substituted alkyl, alkenyl or alkynyl or the alkyl, alkenyl, or alkynyl may be unsubstituted or substituted with one or more substituents selected from the group consisting of halogen, OH, C₁-C₆ alkoxy, NR²⁰R²¹, CONR²⁰R²¹, C(O)R²⁰, CN, oxo, OP(O)(OR²⁰)(OR²¹), OC(O)R²⁰, COOR²⁰, C₁-C₆ alkenyl, C₁-C₆ alkynyl, =NOR²⁰, NR²⁰C(O)R²¹, SO₂R²⁰ and SO₂NR²⁰R²¹. Preferably, when the aryl, heteroaryl, cycloalkyl or heterocycle is substituted, directly or indirectly, with an optionally substituted alkyl, alkenyl or alkynyl, the alkyl, alkenyl or alkynyl is unsubstituted or substituted with one or more of halogen and OH. When the aryl, heteroaryl, cycloalkyl or heterocycle is substituted with an optionally substituted aryl or optionally substituted heteroaryl it may be substituted with an optionally substituted phenyl or an optionally substituted 5 or 6 membered heteroaryl. R²⁰ and R²¹ may independently be H, optionally substituted C₁-C₃ alkyl, optionally substituted C₂-C₃ alkenyl or optionally substituted C₂-C₃ alkynyl. Preferably, R²⁰ and R²¹ are independently H and optionally substituted methyl, and more preferably are H, CH₃ or CF₃. Accordingly, the cycloalkyl, aryl, heteroaryl or heterocycle may be unsubstituted or substituted with one or more of F, Cl, oxo, OH, CN, NH₂, methyl, t-butyl, CF₃, CH₂OH, OCH₃, OCHF₂, OCF₃, SCF₃, COCH₃, COOH, COOCH₃, CONH₂, SO₂CH₃, 1,2,4-triazolyl and phenyl. The aryl, heteroaryl, cycloalkyl or heterocycle is preferably unsubstituted or substituted with 1 or 2 substituents.

Accordingly, R¹⁶ may be cyclopropyl, cyclopentyl, phenyl, 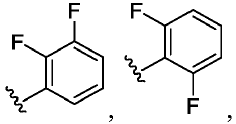 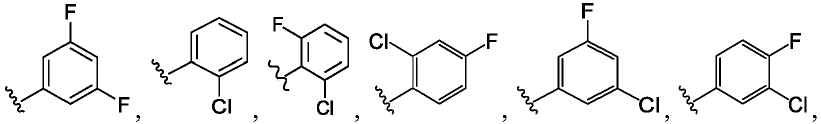 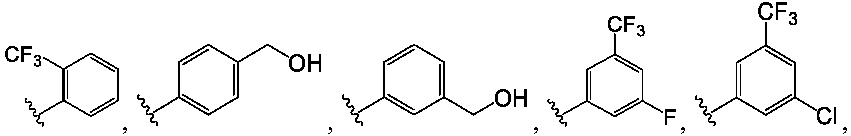 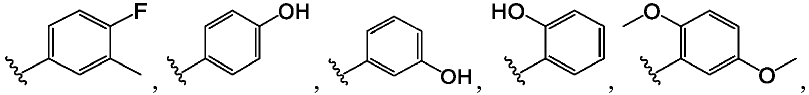 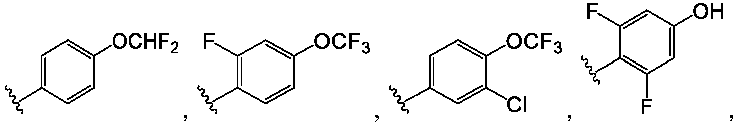 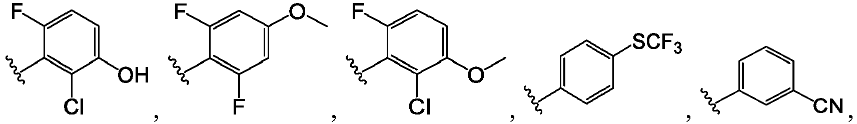 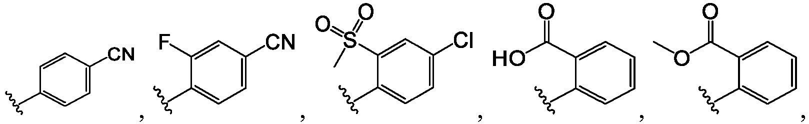 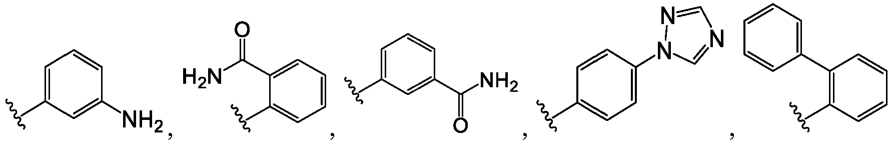 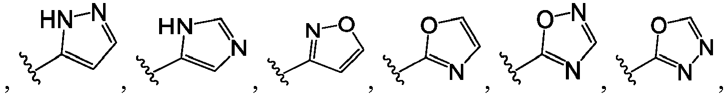 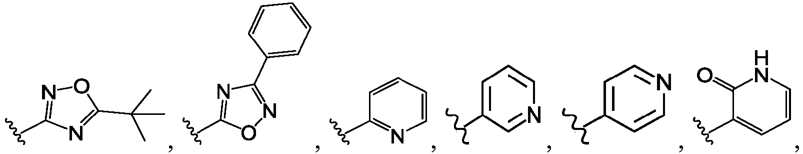 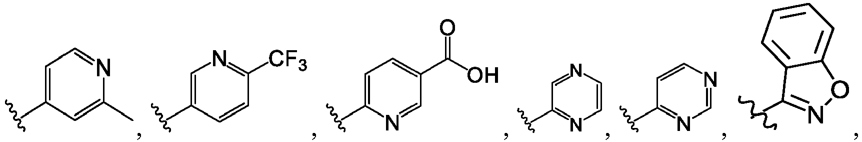 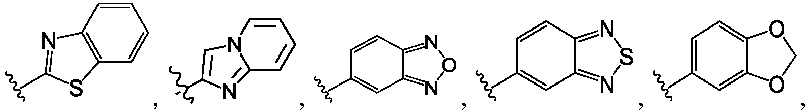 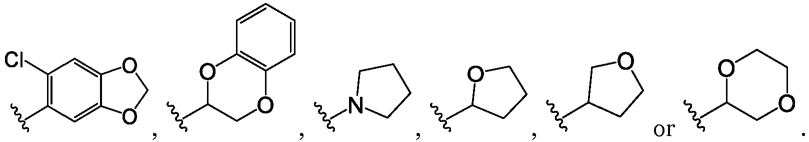 More preferably, R¹⁶ is phenyl or

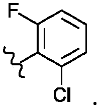

In an alternative embodiment, R⁵ is optionally substituted C₁-C₆ alkyl. R⁵ may be optionally substituted C₁-C₃ alkyl. The alkyl may be unsubstituted or substituted with one or more of halogen, OH, CN and oxo. R⁵ may be CH₃ or CH₂CN.

In one embodiment, X⁶ is CR⁷R⁸. R⁷ may be H, halogen, CONR¹³R¹⁴ or optionally substituted C₁-C₃ alkyl. R⁸ may be H, halogen, OH, CN, COOR¹³, CONR¹³R¹⁴, NR¹³R¹⁴, NR¹³COR¹⁴ or optionally substituted C₁-C₃ alkyl. R¹³ and R¹⁴ are preferably H, optionally substituted C₁-C₃ alkyl, optionally substituted C₂-C₃ alkenyl or optionally substituted C₂-C₃ alkynyl, and most preferably H. The alkyl, alkenyl or alkynyl may be unsubstituted or substituted with one or more of halogen, OH, oxo, CN, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹ and OP(O)(OR²⁰)(OR²¹). R²⁰ and R²¹ may independently be H or methyl. Preferably, R⁷ and R⁸ are independently H, CN, CONH₂, CH₂NH₂, CH₂CH₂OH, 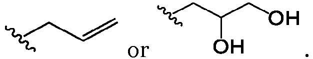 Most preferably, R⁷ and R⁸ are H. Accordingly, X⁶ may be X⁶ is CH₂, 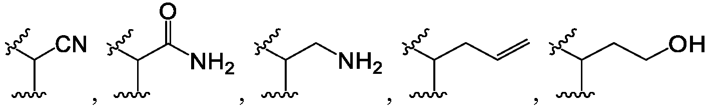 or 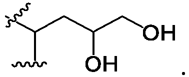 Preferably X⁶ is CH₂.

In an alternative embodiment, X⁶ is CO.

This disclosure also provides examples where n is o. X⁷ may be CR¹¹R¹². R¹¹ and R¹² may independently be H, halogen, OH, CN, optionally substituted C₁-C₃ alkyl, optionally substituted C₂-C₃ alkenyl or optionally substituted C₂-C₃ alkynyl. Preferably, R¹¹ and R¹² are independently H or methyl. Most preferably, R¹¹ and R¹² are H.

R⁹ and R¹⁰ may independently be H, halogen, OR¹³, CN, COOR¹³, CONR¹³R¹⁴, NR¹³R¹⁴, NR¹³COR¹⁴, optionally substituted C₁-C₃ alkyl, optionally substituted C₂-C₃ alkenyl or optionally substituted C₂-C₃ alkynyl. R¹³ and R¹⁴ may independently be H, optionally substituted C₁-C₃ alkyl, optionally substituted C₂-C₃ alkenyl or optionally substituted C₂-C₃ alkynyl. The alkyl, alkenyl or alkynyl may be unsubstituted or substituted with one or more of halogen, OH, oxo, CN, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹ and OP(O)(OR²⁰)(OR²¹). R²⁰ and R²¹ may independently be H or methyl. Preferably, R⁹ and R¹⁰ are independently H, methyl, CH₂CONH₂ or CH₂CN. More preferably, R⁹ and R¹⁰ are H. R¹¹ may be H, C₁-C₃ alkyl, C₂-C₃ alkenyl or C₂-C₃ alkynyl. Preferably, R¹¹ is H or methyl. Most preferably, X⁷ is S or O.

This disclosure also provides examples where Z is NR⁹ and X⁷ is CR¹¹R¹². R⁹ may be H, optionally substituted C₁-C₆ alkyl, optionally substituted C₂-C₆ alkenyl or optionally substituted C₂-C₆ alkynyl. R⁹ may be H, C₁-C₃ alkyl, C₂-C₃ alkenyl or C₂-C₃ alkynyl. Preferably, R⁹ is methyl. R¹¹ and R¹² may independently be H, halogen, OH, CN, optionally substituted C₁-C₆ alkyl, optionally substituted C₂-C₆ alkenyl or optionally substituted C₂-C₆ alkynyl. R¹¹ and R¹² may independently be H, halogen, OH, CN, C₁-C₃ alkyl, C₂-C₃ alkenyl or C₂-C₃ alkynyl. Preferably, R¹¹ and R¹² are H or methyl. In examples where X⁷ is CR¹¹R¹² and R¹¹ and R¹² are different, the carbon to which R¹¹ and R¹² are bonded defines a chiral centre. The chiral centre may be an *S* or *R* chiral centre. In some examples, the chiral centre is an S chiral centre.

. This disclosure provides examples where n is 1. Z may be CR⁹R¹⁰ and X⁷ may be S, SO, SO₂, O or NR¹¹. Alternatively, Z may be NR⁹ and X⁷ may be CR¹¹R¹². Accordingly, the compound may be a compound of formula **(II)** or **(III):**

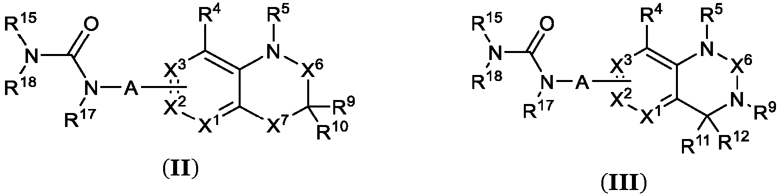

In alternative examples, n is 0. X⁷ may be CR¹¹R¹². Accordingly, the compound may be a compound of formula (IV):

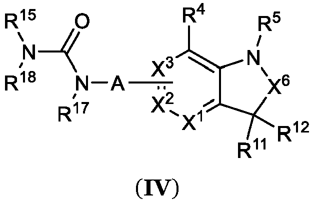

In some examples, X² is CR² and X³ is CR³. In some examples, R² is -A-NR¹⁷-C(O)-NR¹⁸-R¹⁵. In alternative examples, R³ is -A-NR¹⁷-C(O)-NR¹⁸-R¹⁵. Accordingly, the compound of formula **(II)** or **(III)** may be a compound of formula **(IIa), (IIb), (IIIa), (IIIb), (IVa)** or **(IVb):**

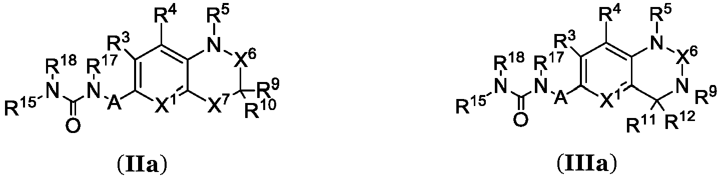

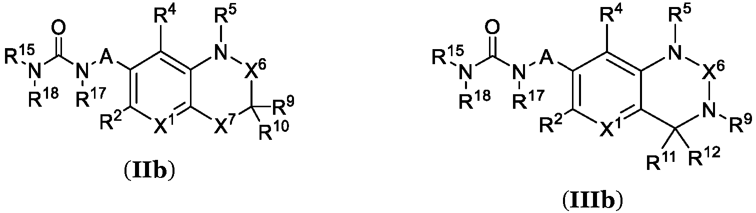

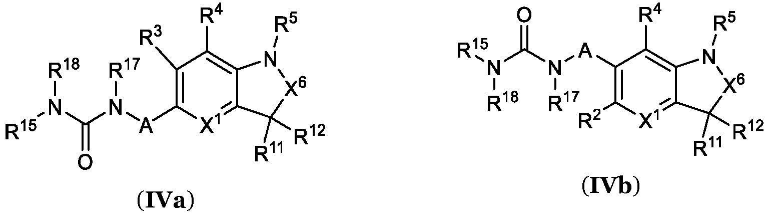

In some examples of a compound of formula **(II), (III), (IIa), (IIb), (IIIa), (IIIb), (IVa)** or **(IVb)** R⁵ is H, optionally substituted C₁-C₆ alkyl, optionally substituted C₂-C₆ alkenyl or optionally substituted C₂-C₆ alkynyl. R⁵ may be H, optionally substituted C₁-C₃ alkyl, optionally substituted C₂-C₃ alkenyl or optionally substituted C₂-C₃ alkynyl. The alkyl, alkenyl or alkynyl may be unsubstituted or substituted with one or more of halogen, OH, CN and oxo. Preferably, R⁵ is H or CH₃.

In alternative examples of a compound of formula **(II), (III), (IIa), (IIb), (IIIa), (IIIb), (IVa)** or **(IVb)** R⁵ is -L¹-L²-R¹⁶. Accordingly, the compound may be a compound of formula **(IIc), (IId), (IIIc), (IIId), (IVc)** or **(IVd):**

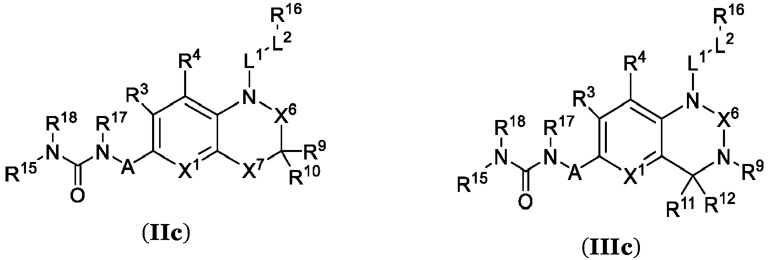

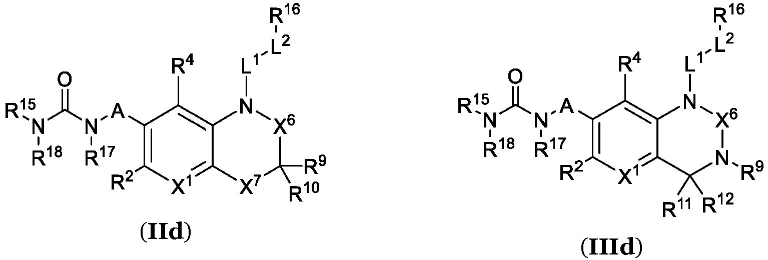

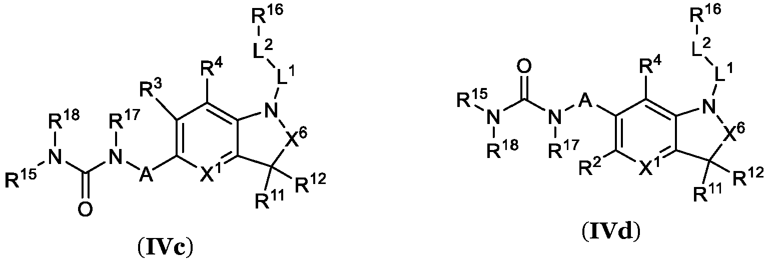

In some examples, L⁶ may be absent and R⁵ may be -L⁵-R¹⁶. Accordingly, the compound may be a compound of formula **(IIci), (IIdi), (IIIci), (IIIdi), (IVci)** or **(IVdi):**

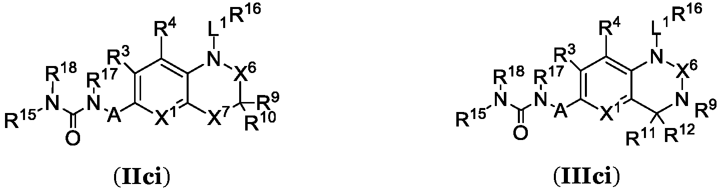

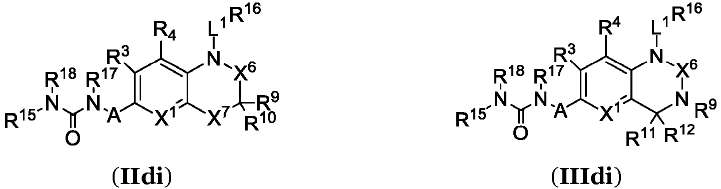

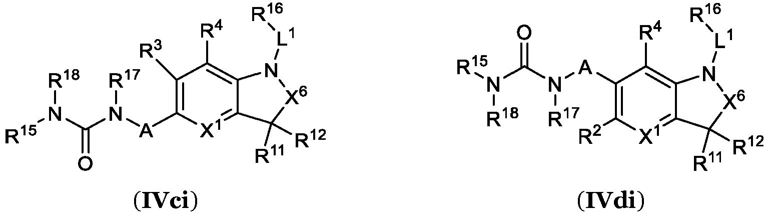

In a compound of formula **(II), (III), (IIa)** to **(IIdi), (IIIa)** to **(IIIdi)** or **(IVa)** to **(IVdi),** X⁶ may be C=O or CR⁷R⁸. In some examples, X⁶ is C=O.

In a compound of formula **(II)** or **(IIa)** to **(IId),** X⁷ may be S or O. Preferably, X⁷ is S.

It will be appreciated that the compounds described herein or a pharmaceutically acceptable salt, solvate, tautomeric form or polymorphic form thereof may be used in a medicament which may be used in a monotherapy (i.e. use of the compound alone), for modulating the STING protein and/or treating, ameliorating or preventing a disease.

Alternatively, the compounds or a pharmaceutically acceptable salt, solvate, tautomeric form or polymorphic form thereof may be used as an adjunct to, or in combination with, known therapies for modulating the STING protein and/or treating, ameliorating or preventing a disease.

The compound of Formula **(I)** may be combined in compositions having a number of different forms depending, in particular, on the manner in which the composition is to be used. Thus, for example, the composition may be in the form of a powder, tablet, capsule, liquid, ointment, cream, gel, hydrogel, aerosol, spray, micellar solution, transdermal patch, liposome suspension or any other suitable form that may be administered to a person or animal in need of treatment. It will be appreciated that the vehicle of medicaments according to the invention should be one which is well-tolerated by the subject to whom it is given.

Medicaments comprising the compounds described herein may be used in a number of ways. Suitable modes of administration include oral, intra-tumoral, parenteral, topical, inhaled/intranasal, rectal/intravaginal, and ocular/aural administration. Formulations suitable for the aforementioned modes of administration may be formulated to be immediate and/or modified release. Modified release formulations include delayed-, sustained-, pulsed-, controlled-, targeted and programmed release.

The compounds of the invention may be administered orally. Oral administration may involve swallowing, so that the compound enters the gastrointestinal tract, or buccal or sublingual administration may be employed by which the compound enters the blood stream directly from the mouth. Formulations suitable for oral administration include solid formulations such as tablets, capsules containing particulates, liquids, or powders, lozenges (including liquid-filled), chews, multi- and nano-particulates, gels, solid solution, liposome, films, ovules, sprays, liquid formulations and buccal/mucoadhesive patches.

Liquid formulations include suspensions, solutions, syrups and elixirs. Such formulations may be employed as fillers in soft or hard capsules and typically comprise a carrier, for example, water, ethanol, polyethylene glycol, propylene glycol, methylcellulose, or a suitable oil, and one or more emulsifying agents and/or suspending agents. Liquid formulations may also be prepared by the reconstitution of a solid, for example, from a sachet.

The compounds of the invention may also be used in fast-dissolving, fast-disintegrating dosage forms such as those described in Expert Opinion in Therapeutic Patents, 11 (6), 981-986, by Liang and Chen (2001).

For tablet dosage forms, depending on dose, the drug may make up from 1 weight % to 80 weight % of the dosage form, more typically from 5 weight % to 60 weight % of the dosage form. In addition to the drug, tablets generally contain a disintegrant. Examples of disintegrants include sodium starch glycolate, sodium carboxymethyl cellulose, calcium carboxymethyl cellulose, croscarmellose sodium, crospovidone, polyvinylpyrrolidone, methyl cellulose, microcrystalline cellulose, lower alkylsubstituted hydroxypropyl cellulose, starch, pregelatinised starch and sodium alginate. Generally, the disintegrant will comprise from 1 weight % to 25 weight %, preferably from 5 weight % to 20 weight % of the dosage form.

Binders are generally used to impart cohesive qualities to a tablet formulation. Suitable binders include microcrystalline cellulose, gelatin, sugars, polyethylene glycol, natural and synthetic gums, polyvinylpyrrolidone, pregelatinised starch, hydroxypropyl cellulose and hydroxypropyl methylcellulose. Tablets may also contain diluents, such as lactose (monohydrate, spray-dried monohydrate, anhydrous and the like), mannitol, xylitol, dextrose, sucrose, sorbitol, microcrystalline cellulose, starch and dibasic calcium phosphate dihydrate.

Tablets may also optionally comprise surface active agents, such as sodium lauryl sulfate and polysorbate 80, and glidants such as silicon dioxide and talc. When present, surface active agents may comprise from 0.2 weight % to 5 weight % of the tablet, and glidants may comprise from 0.2 weight % to 1 weight % of the tablet.

Tablets also generally contain lubricants such as magnesium stearate, calcium stearate, zinc stearate, sodium stearyl fumarate, and mixtures of magnesium stearate with sodium lauryl sulphate. Lubricants generally comprise from 0.25 weight % to 10 weight %, preferably from 0.5 weight % to 3 weight % of the tablet. Other possible ingredients include anti-oxidants, colourants, flavouring agents, preservatives and taste-masking agents.

Exemplary tablets contain up to about 80% drug, from about 10 weight % to about 90 weight % binder, from about 0 weight % to about 85 weight % diluent, from about 2 weight % to about 10 weight % disintegrant, and from about 0.25 weight % to about 10 weight % lubricant. Tablet blends may be compressed directly or by roller to form tablets. Tablet blends or portions of blends may alternatively be wet-, dry-, or melt-granulated, melt congealed, or extruded before tabletting. The final formulation may comprise one or more layers and may be coated or uncoated; it may even be encapsulated. The formulation of tablets is discussed in "Pharmaceutical Dosage Forms: Tablets", Vol. 1, by H. Lieberman and L. Lachman (Marcel Dekker, New York, 1980).

Suitable modified release formulations for the purposes of the invention are described in US Patent No. 6,106,864. Details of other suitable release technologies such as high energy dispersions and osmotic and coated particles are to be found in "Pharmaceutical Technology On-line", 25(2), 1-14, by Verma et al (2001). The use of chewing gum to achieve controlled release is described in WO 00/35298.

The compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

Parenteral formulations are typically aqueous solutions which may contain excipients such as salts, carbohydrates and buffering agents (preferably to a pH of from 3 to 9), but, for some applications, they may be more suitably formulated as a sterile nonaqueous solution or as a dried form to be used in conjunction with a suitable vehicle such as sterile, pyrogen-free water.

The preparation of parenteral formulations under sterile conditions, for example, by lyophilisation, may readily be accomplished using standard pharmaceutical techniques well known to those skilled in the art.

The solubility of compounds of formula **(I)** used in the preparation of parenteral solutions may be increased by the use of appropriate formulation techniques, such as the incorporation of solubility-enhancing agents. Formulations for parenteral administration may be formulated to be immediate and/or modified release. Modified release formulations include delayed-, sustained-, pulsed-, controlled-, targeted and programmed release. Thus compounds of the invention may be formulated as a solid, semi-solid, or thixotropic liquid for administration as an implanted depot providing modified release of the active compound. Examples of such formulations include drug-coated stents and poly(dl-lactic-coglycolic)acid (PGLA) microspheres.

The compounds of the invention may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Other means of topical administration include delivery by electroporation, iontophoresis, phonophoresis, sonophoresis and microneedle or needle-free (e.g. Powderject^{™}, Bioject^{™}, etc.) injection.

The compounds of the invention can also be administered intranasally or by inhalation, typically in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

The pressurised container, pump, spray, atomizer, or nebuliser contains a solution or suspension of the compound(s) of the invention comprising, for example, ethanol, aqueous ethanol, or a suitable alternative agent for dispersing, solubilising, or extending release of the active, a propellant(s) as solvent and an optional surfactant, such as sorbitan trioleate, oleic acid, or an oligolactic acid.

Prior to use in a dry powder or suspension formulation, the drug product is micronised to a size suitable for delivery by inhalation (typically less than 5 microns). This may be achieved by any appropriate comminuting method, such as spiral jet milling, fluid bed jet milling, and supercritical fluid processing to form nanoparticles, high pressure homogenisation, or spray drying.

Capsules (made, for example, from gelatin or hydroxypropylmethylcellulose), blisters and cartridges for use in an inhaler or insufflator may be formulated to contain a powder mix of the compound of the invention, a suitable powder base such as lactose or starch and a performance modifier such as L-leucine, mannitol, or magnesium stearate. The lactose may be anhydrous or in the form of the monohydrate, preferably the latter. Other suitable excipients include dextran, glucose, maltose, sorbitol, xylitol, fructose, sucrose and trehalose.

A suitable solution formulation for use in an atomiser using electrohydrodynamics to produce a fine mist may contain from 1µg to 20mg of the compound of the invention per actuation and the actuation volume may vary from 1µl to 100µl. A typical formulation may comprise a compound of formula **(I),** propylene glycol, sterile water, ethanol and sodium chloride. Alternative solvents which may be used instead of propylene glycol include glycerol and polyethylene glycol.

Suitable flavours, such as menthol and levomenthol, or sweeteners, such as saccharin or saccharin sodium, may be added to those formulations of the invention intended for inhaled/intranasal administration.

In the case of dry powder inhalers and aerosols, the dosage unit is determined by means of a valve which delivers a metered amount. Units in accordance with the invention are typically arranged to administer a metered dose or "puff" containing from 1µg to 100mg of the compound of formula **(I).** The overall daily dose will typically be in the range 1µg to 200mg which may be administered in a single dose or, more usually, as divided doses throughout the day.

The compounds of the invention may be administered rectally or vaginally, for example, in the form of a suppository, pessary, microbicide, vaginal ring or enema. Cocoa butter is a traditional suppository base, but various alternatives may be used as appropriate.

The compounds of the invention may also be administered directly to the eye or ear, typically in the form of drops of a micronised suspension or solution in isotonic, pH-adjusted, sterile saline. Other formulations suitable for ocular and aural administration include ointments, biodegradable (e.g. absorbable gel sponges, collagen) and non-biodegradable (e.g. silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

The compounds of the invention may also be administered directly to a site of interest by injection of a solution or suspension containing the active drug substance. The site of interest may be a tumour and the compound may by administer via intratumoral injection. Typical injection solutions are comprised of propylene glycol, sterile water, ethanol and sodium chloride. Alternative solvents which may be used instead of propylene glycol include glycerol and polyethylene glycol.

The compounds of the invention may be combined with soluble macromolecular entities, such as cyclodextrin and suitable derivatives thereof or polyethylene glycol-containing polymers, in order to improve their solubility, dissolution rate, taste-masking, bioavailability and/or stability for use in any of the aforementioned modes of administration.

Drug-cyclodextrin complexes, for example, are found to be generally useful for most dosage forms and administration routes. Both inclusion and non-inclusion complexes may be used. As an alternative to direct complexation with the drug, the cyclodextrin may be used as an auxiliary additive, i.e. as a carrier, diluent, or solubiliser. Most commonly used for these purposes are alpha-, beta- and gamma-cyclodextrins, examples of which may be found in International Patent Applications Nos. WO 91/11172, WO 94/02518 and WO 98/55148.

It will be appreciated that the amount of the compound that is required is determined by its biological activity and bioavailability, which in turn depends on the mode of administration, the physiochemical properties of the compound, and whether it is being used as a monotherapy, or in a combined therapy. The frequency of administration will also be influenced by the half-life of the compound within the subject being treated. Optimal dosages to be administered may be determined by those skilled in the art, and will vary with the particular compound in use, the strength of the pharmaceutical composition, the mode of administration, and the advancement of the disease. Additional factors depending on the particular subject being treated will result in a need to adjust dosages, including subject age, weight, sex, diet, and time of administration.

Generally, for administration to a human, the total daily dose of the compounds of the invention is typically in the range 100µg to 10g, such as 1mg to 1g, for example 10mg to 500mg. For example, oral administration may require a total daily dose of from 25mg to 250mg. The total daily dose may be administered in single or divided doses and may, at the physician's discretion, fall outside of the typical range given herein. These dosages are based on an average human subject having a weight of about 60kg to 70kg. The physician will readily be able to determine doses for subjects whose weight falls outside this range, such as infants and the elderly.

The compound may be administered before, during or after onset of the disease to be treated.

Known procedures, such as those conventionally employed by the pharmaceutical industry (e.g. in *vivo* experimentation, clinical trials, etc.), may be used to form specific formulations comprising the compounds according to the invention and precise therapeutic regimes (such as daily doses of the compounds and the frequency of administration). The inventors believe that they are the first to describe a pharmaceutical composition for treating a disease, based on the use of the compounds of the invention.

Hence, in an fourth aspect of the invention, there is provided a pharmaceutical composition comprising a compound according to the first aspect, or a pharmaceutically acceptable salt, solvate, tautomeric form or polymorphic form thereof, and a pharmaceutically acceptable vehicle.

This disclosure also provides, a process for making the composition according to the fourth aspect, the process comprising contacting a therapeutically effective amount of a compound of the first aspect, or a pharmaceutically acceptable salt, solvate, tautomeric form or polymorphic form thereof, and a pharmaceutically acceptable vehicle.

A "subject" may be a vertebrate, mammal, or domestic animal. Hence, compounds, compositions and medicaments according to the invention may be used to treat any mammal, for example livestock (e.g. a horse), pets, or may be used in other veterinary applications. Most preferably, however, the subject is a human being.

A "therapeutically effective amount" of compound is any amount which, when administered to a subject, is the amount of drug that is needed to treat the target disease, or produce the desired effect, i.e. inhibit the STING protein.

For example, the therapeutically effective amount of compound used may be from about 0.01 mg to about 800 mg, and preferably from about 0.01 mg to about 500 mg. It is preferred that the amount of compound is an amount from about 0.1 mg to about 250 mg, and most preferably from about 0.1 mg to about 20 mg.

A "pharmaceutically acceptable vehicle" as referred to herein, is any known compound or combination of known compounds that are known to those skilled in the art to be useful in formulating pharmaceutical compositions.

In one example, the pharmaceutically acceptable vehicle may be a solid, and the composition may be in the form of a powder or tablet. A solid pharmaceutically acceptable vehicle may include one or more substances which may also act as flavouring agents, lubricants, solubilisers, suspending agents, dyes, fillers, glidants, compression aids, inert binders, sweeteners, preservatives, dyes, coatings, or tablet-disintegrating agents. The vehicle may also be an encapsulating material. In powders, the vehicle is a finely divided solid that is in admixture with the finely divided active agents (i.e. the compound according to the first aspect) according to the invention. In tablets, the active compound may be mixed with a vehicle having the necessary compression properties in suitable proportions and compacted in the shape and size desired. The powders and tablets preferably contain up to 99% of the active compound. Suitable solid vehicles include, for example calcium phosphate, magnesium stearate, talc, sugars, lactose, dextrin, starch, gelatin, cellulose, polyvinylpyrrolidine, low melting waxes and ion exchange resins. In another example, the pharmaceutical vehicle may be a gel and the composition may be in the form of a cream or the like.

However, the pharmaceutical vehicle may be a liquid, and the pharmaceutical composition is in the form of a solution. Liquid vehicles are used in preparing solutions, suspensions, emulsions, syrups, elixirs and pressurized compositions. The compound according to the invention may be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilisers, emulsifiers, buffers, preservatives, sweeteners, flavouring agents, suspending agents, thickening agents, colours, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be a halogenated hydrocarbon or other pharmaceutically acceptable propellant.

Liquid pharmaceutical compositions, which are sterile solutions or suspensions, can be utilized by, for example, intramuscular, intrathecal, epidural, intraperitoneal, intravenous and particularly subcutaneous injection. The compound may be prepared as a sterile solid composition that may be dissolved or suspended at the time of administration using sterile water, saline, or other appropriate sterile injectable medium.

The compound and compositions of the invention may be administered in the form of a sterile solution or suspension containing other solutes or suspending agents (for example, enough saline or glucose to make the solution isotonic), bile salts, acacia, gelatin, sorbitan monoleate, polysorbate 80 (oleate esters of sorbitol and its anhydrides copolymerized with ethylene oxide) and the like. The compounds used according to the invention can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

It will be known to those skilled in the art that active drug ingredients may be converted into a prodrug, which is a metabolically labile derivative that is converted within the body into the active drug substance. This disclosure also provides prodrugs which are compounds of formula **(I)** which contain metabolically or hydrolytically labile moieties which in vivo are converted into the active drug of formula **(I).** The processes by which the prodrug is converted into the active drug substance include, but are not limited to, ester or carbonate or carbamate hydrolysis, phosphate ester hydrolysis, *S*-oxidation, *N-*oxidation, dealkylation and metabolic oxidation as described in Beaumont et. al., Curr. Drug Metab., 2003, 4, 461-485 and Huttenen et. al., Pharmacol. Revs., 2011, 63, 750-771. Such prodrug derivatives may offer improved solubility, stability or permeability compared to the parent drug substance, or may better allow the drug substance to be administered by an alternative route of administration, for example as an intravenous solution.

This disclosure also provides soft drugs or antedrugs which are compounds of formula **(I)** which contain metabolically or hydrolytically labile moieties which in *vivo* are converted into inactive derivatives. The processes by which the active drug substance is converted into an inactive derivative include, but are not limited to, ester hydrolysis, *S-*oxidation, N-oxidation, dealkylation and metabolic oxidation as described for example in Pearce et al., Drug Metab. Dispos., 2006, 34, 1035-1040 and B. Testa, Prodrug and Soft Drug Design, in Comprehensive Medicinal Chemistry II, Volume 5, Elsevier, Oxford, 2007, pp. 1009-1041 and Bodor, N. Chem. Tech. 1984, 14, 28-38.

The scope of the invention includes all pharmaceutically acceptable isotopically-labelled compounds of the invention wherein one or more atoms are replaced by atoms having the same atomic number, but an atomic mass or mass number different from the atomic mass or mass number which predominates in nature.

Examples of isotopes suitable for inclusion in the compounds of the invention include isotopes of hydrogen, such as ²H and ³H, carbon, such as ¹¹C, ¹³C and ¹⁴C, chlorine, such as ³⁶Cl, fluorine, such as ¹⁸F, iodine, such as ¹²³I and ¹²⁵I, nitrogen, such as ¹³N and ¹⁵N, oxygen, such as ¹⁵O, ¹⁷O and ¹⁸O, phosphorus, such as ³²P, and sulphur, such as ³⁵S.

Certain isotopically-labelled compounds of the invention, for example those incorporating a radioactive isotope, are useful in drug and/or substrate tissue distribution studies. The radioactive isotopes tritium, i.e. ³H, and carbon-14, i.e. ¹⁴C, are particularly useful for this purpose in view of their ease of incorporation and ready means of detection. Substitution with isotopes such as deuterium, i.e. ²H, may afford certain therapeutic advantages resulting from greater metabolic stability, for example, increased in vivo half-life or reduced dosage requirements, and hence may be preferred in some circumstances. Substitution with positron emitting isotopes, such as ¹¹C, ¹⁸F, ¹⁵O and ¹³N, can be useful in Positron Emission Topography (PET) studies for examining substrate receptor occupancy.

Isotopically-labelled compounds of formula **(I)** can generally be prepared by conventional techniques known to those skilled in the art or by processes analogous to those described in the accompanying Examples and Preparations using an appropriate isotopically-labelled reagent in place of the non-labelled reagent previously employed.

All features described herein (including any accompanying claims and abstract), and/or all of the steps of any method or process so disclosed, may be combined with any of the above aspects in any combination, except combinations where at least some of such features and/or steps are mutually exclusive.

### General Schemes

### General Scheme 1

Compounds of formula **(IVe)** and **(IVf)** may be prepared from compounds of formula **(VIa)** and **(VIb)** using a urea bond forming reaction, as shown below.

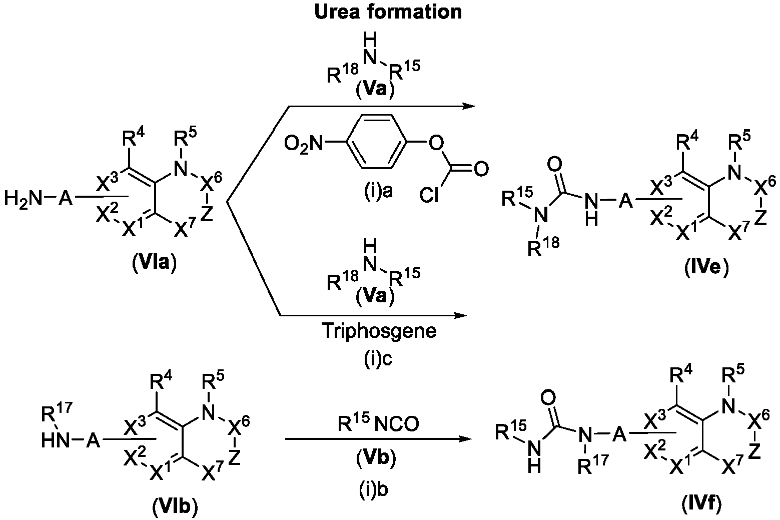

Typical reaction conditions for the activation of the aromatic amine of the compounds of formula **(VIa)** or **(VIb)** employ 4-nitrophenyl chloroformate or triphosgene to generate an activated intermediate which can be attacked by a suitable nucleophile such as amine **(Va)** to give a urea compound of formula **(IVe)** or **(IVf).** Preferred organic bases include DIPEA or TEA in a suitable organic solvent such as DCM, DMF, DMA or MeCN. The reaction may be shaken or stirred at room temperature. Alternatively, the compounds of formula **(IVe)** or **(IVf)** can also be prepared with an isocyanate R¹⁵NCO **(Vb)** in a suitable solvent such as THF, DMF or MeCN and a preferred organic base such as TEA or DIPEA. The reaction may be shaken or stirred at room temperature.

Compounds of formula **(V)** and **(VI)** are commercially available or may be synthesized by those skilled in the art. In particular, methods of synthesizing compounds of formula **(VI)** are described in General Schemes 2 to 4.

### General Scheme 2

Compounds of formula **(X)** may be synthesized from esters of formula **(VII),** where R is methyl, ethyl, benzyl or *tert-*butyl*,* by a hydrolysis reaction.

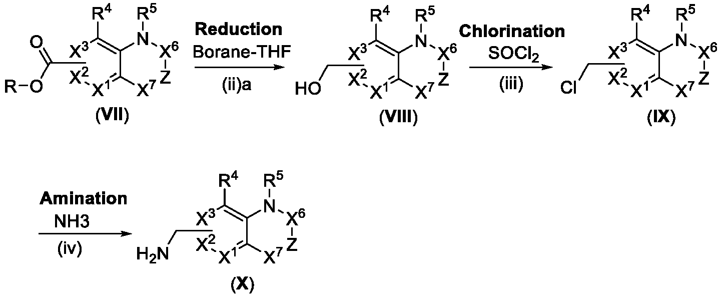

Esters of formula **(VII)** may be reduced with a suitable reducing agent such as borane-THF, NaBH4, DIBAL or LiAlH4 to provide the corresponding alcohols **(VIII).** The hydroxyl functional group may then be chlorinated with for example thionyl chloride or oxalyl chloride to give the corresponding chlorides **(IX),** which may then be aminated with any suitable primary or secondary amine, for example ammonia, to give the amines of formula **(X).**

### General Scheme 3

Compounds of formula **(X)** may be also synthesized from esters of formula **(VII),** where R is methyl, ethyl, benzyl or *tert-*butyl*,* by a hydrolysis reaction.

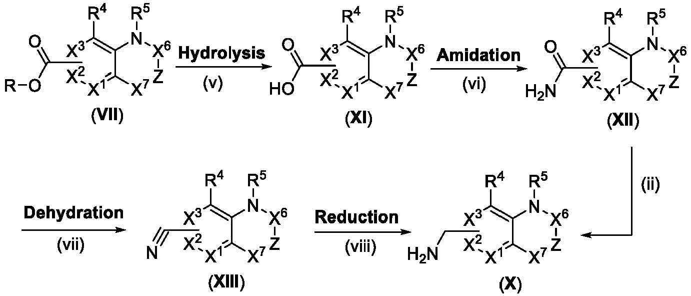

Esters of formula **(VII)** may be hydrolysed using suitable conditions, for example using alkalis NaOH, LiOH or KOH to give the acids **(XI)** which may then be converted into the amides **(XII)** using a standard amide coupling reaction. Typical conditions employ activation of the carboxylic acid **(XI)** using a suitable organic base and a suitable coupling agent. Preferred coupling agents are either EDCI with HOBt, T₃P, HATU, HBTU or BOP. Preferred organic bases comprise either DIPEA or TEA in a suitable organic solvent such as DCM, DMF, DMA or MeCN. The reaction may be shaken or stirred at room temperature. These amides **(XII)** may then either be subjected to a dehydration reaction with a suitable dehydrating reagent such as thionyl chloride or phosphorus pentoxide to give the corresponding nitriles **(XIII)** which may then be reduced with a suitable reducing agent such as LiAlH₄ to give the corresponding amines **(X).** Alternatively, the amides **(XII)** may be directly reduced to the amines of formula **(X)** with a suitable reducing agent such as LiAlH₄ typically in alcoholic solvents such as ether or THF to give the amines **(X).**

### General Scheme 4

Compounds of formula **(XVI)** may be synthesized by those skilled in the art via an alkylation/acylation/sulfonylation reaction with a compound of formula **(XIV),** where X is a leaving group such as an optionally substituted alkylaryl(het), alkyl, aryl(het), cycloalkyl, alkylcycloalkyl halide, triflate or tosylate.

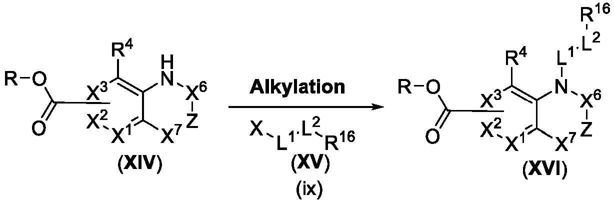

Compounds of formula **(XIV)** may be reacted with compounds of formula **(XV)** in the presence of a suitable base such as NaH, NaHCO₃ or TEA to furnish compounds of formula **(XVI).** Suitable reaction solvents include THF, DMA and DMF.

### General Scheme 5

Alternatively, a compound of formula **(XIV)** may be prepared in a two-step process, as shown below, from a compound of formula **(XVII),** where R is methyl, ethyl, benzyl or *tert-*butyl.

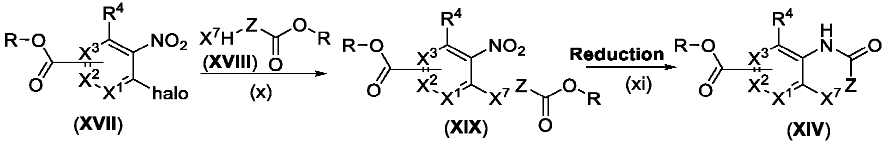

Firstly, compounds of formula **(XVII)** undergo a nucleophilic substitution reaction with a compound of formula **(XVIII),** where R is methyl, ethyl, benzyl or *tert-*butyl*,* to produce a compound of formula **(XIX).** The nucleophilic substitution reaction may be conducted in the presence of a mild base, such as DBU, NaH, TEA, DIPEA, K₂CO₃, Cs₂CO₃ or KHCO₃. The solvent used may be 1,4-dioxane, acetone, MeCN, THF or DMF.

The nitro group of compounds of formula **(XIX)** may then be reduced to an amino group using a suitable reducing agent, such as Fe/AcOH, Zn/HCl, Zn/NH₄Cl, Zn/HCOONH₄, SnCl₂/HCl or Pd/C/H₂, in a suitable solvent such as EtOH, MeOH or THF. The ensuing amino compounds typically undergo in-situ cyclization resulting in the formation of compounds of formula **(XIV).**

It will be appreciated that the compound of formula **(XIV)** is a compound of formula **(VII)** where R⁵ is H and X⁶ is C=O.

### General Scheme 6

A compound of formula **(XXI)** may be prepared from a compound of formula **(XX),** where R is methyl, ethyl, benzyl or *tert-*butyl*.*

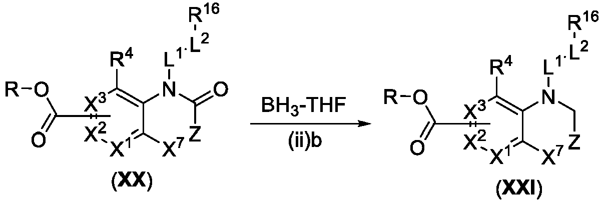

The lactam carbonyl group of a compound of formula **(XX)** can be reduced to the corresponding methylene group of a compound of formula **(XXI)** using borane-THF solution in a suitable solvent such as THF, typically at low temperatures.

It will be appreciated that the compound of formula **(XXI)** is a compound of formula **(XVI)** where X⁶ is CH₂.

### General Scheme 7

A compound of formula **(XXIII)** may be prepared from a compound of formula **(XXII)** where R is methyl, ethyl, benzyl or *tert-*butyl*.*

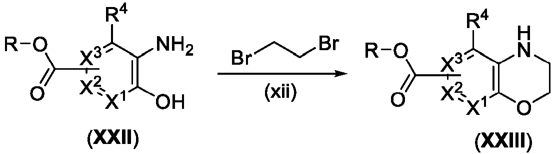

Compounds of formula **(XXII)** may undergo cyclization with 1,2-dibromoethane in a basic reaction medium to give a fused-morpholine derivative compound of formula **(XXIII).**

It will be appreciated that the compound of formula **(XXIII)** is a compound of formula **(VII)** where X⁶ and Z are CH₂, X⁷ is O and R⁵ is H.

### General Scheme 8

A compound of formula **(XXIV)** may be prepared from a compound of formula **(XIV)** in the one step reaction described in the below scheme where R is methyl, ethyl, benzyl or *tert-butyl.*

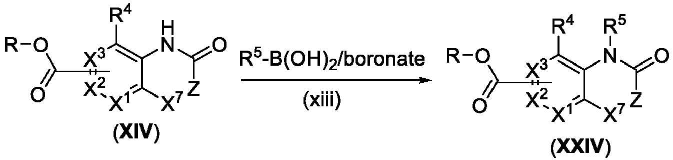

A compound of formula **(XIV)** may undergo a Chan-Lam coupling reaction with a suitable boronic acid/boronate ester in the presence of a suitable catalyst and base to give a compound of formula **(XXIV).**

It will be appreciated that the compound of formula **(XXIV)** is a compound of formula **(VII)** where X⁶ is C=O.

### General Scheme 9

A compound of formula **(XXVI)** may be prepared from a compound of formula **(XXV)** in a one-step reaction described in the below scheme where R is methyl, ethyl, benzyl or *tert*-butyl.

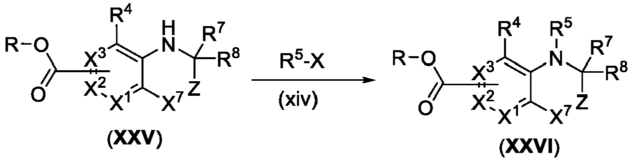

A compound of formula **(XXV)** may undergo a Buchwald coupling reaction with a suitable aromatic halide (R⁵-X) to give a compound of formula **(XXVI).**

It will be appreciated that the compound of formula **(XXVI)** is a compound of formula **(VII)** where X⁶ is CR⁷R⁸.

### General Scheme 10

A compound of formula **(XXXII)** may be prepared from a compound of formula **(VIII)** in a sequence of reaction described in the below scheme where R is H, alkyl, cyclo(het)alkyl, aryl(het).

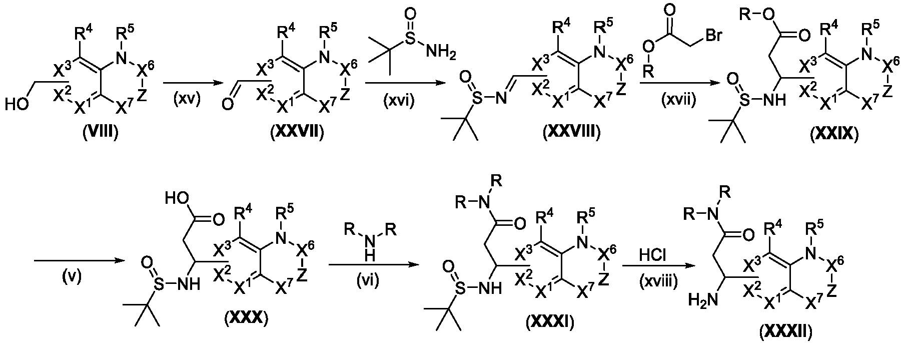

Alcohols **(VIII)** may be converted into the corresponding aldehydes **(XXVII)** using suitable oxidation conditions, for example Swern, Dess-Martin periodinane, TPAP/NMO or PCC. The aldehydes **(XXVII)** may then be converted into the sulfoximines **(XXVIII)** by treatment with tert-butyl sulfonamide, which may then be reacted with a suitable nucleophilic reagent, for example a Grignard or other organometallic reagent derived from the requisite halide to give the addition products **(XXIX).** It is possible that this reaction may be adapted to prepare non-racemic material using a suitable chiral, non-racemic sulfonamide reagent to prepare the sulfoximines. In the example shown, the addition products **(XXIX)** may undergo a hydrolysis reaction using, for example, alkali conditions to give the corresponding acids **(XXX).** The acids **(XXX)** may then be used to form amides **(XXXI)** using any standard amide coupling condition. Typical conditions employ activation of the carboxylic acid **(XI)** using a suitable organic base and a suitable coupling agent. Preferred coupling agents are either EDCI with HOBt, T₃P, HATU, HBTU or BOP. Preferred organic bases comprise either DIPEA or TEA in a suitable organic solvent such as DCM, DMF, DMA or MeCN. The reaction may be shaken or stirred at room temperature. Finally, the target amines **(XXXII)** may be accessed by removal of the sulfoxyl group using acidic conditions, for example aqueous HCl.

### General Scheme 11

A compound of formula **(XXXIV)** may be prepared from a compound of formula **(XXVIII)** in a two steps reaction described in the below scheme where R is alky, cyclo(het)alkyl, aryl(het).

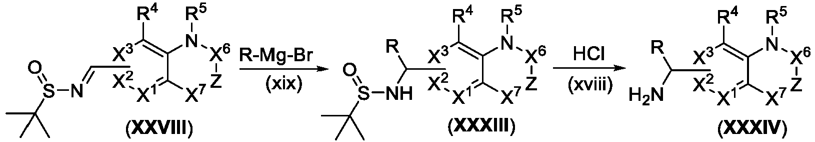

Similarly to General Scheme 10, alkyl amines of formula **(XXXIV)** may be obtained using a similar sequence in which the sulfoximine **(XXVIII)** may be reacted with simple Grignard reagents to give the addition products **(XXXIII),** which following acidic removal of the sulfoxyl group, gives the alkyl amines **(XXXIV).**

### General Scheme 12

A compound of formula **(XXXV)** may be prepared from a compound of formula **(XXVII)** in a one-step reaction described in the below scheme where R is methyl, ethyl, benzyl or *tert-*butyl.

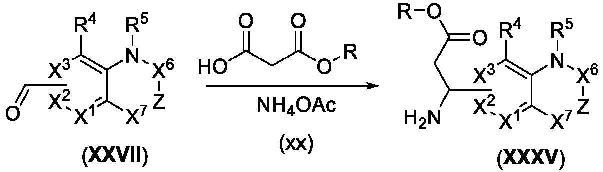

Alternatively, aldehydes of formula **(XXVII)** as prepared in General Scheme 10 may undergo direct conversion to amines of formula **(XXXV)** using ammonium acetate to form an intial imine, which is then treated with a suitable malonate ester to add to the imine, and form the amines **(XXXV)** after decarboxylation.

### General Synthetic Procedures

### General Purification and Analytical Methods

All final compounds were purified by either Combi-flash or prep-HPLC purification, and analysed for purity and product identity by UPLC or LCMS according to one of the below conditions.

### Prep-HPLC

Preparative HPLC was carried out on a Waters auto purification instrument using a Gemini C18 column (250 x 21.2 mm, 10 µm) operating at ambient temperature with a flow rate of 16.0 - 25.0 mL/min.

Mobile phase 1: A = 0.1% formic acid in water, B = Acetonitrile; Gradient Profile: Mobile phase initial composition of 80% A and 20% B, then to 60% A and 40% B after 3 min., then to 30% A and 70% B after 20 min., then to 5% A and 95% B after 21 min., held at this composition for 1 min. for column washing, then returned to the initial composition for 3 min.

Mobile phase 2: A = 10mM Ammonium Acetate in water, B = Acetonitrile; Gradient Profile: Mobile phase initial composition of 90% A and 10% B, then to 70% A and 30% B after 2 min., then to 20% A and 80% B after 20 min., then to 5% A and 95% B after 21 min., held at this composition for 1 min. for column washing, then returned to the initial composition for 3 min.

### LCMS method

General 5 min method: Gemini C18 column (50 x 4.6 mm, 5µm) operating at ambient temperature and a flow rate of 1.2 mL/min. Mobile phase: A = 10 mM Ammonium Acetate in water, B = Acetonitrile; Gradient profile: from 90 % A and 10 % B to 70 % A and 30 B in 1.5 min, and then to 10 % A and 90 % B in 3.0 min, held at this composition for 1.0 min, and finally back to the initial composition for 2.0 min.

### UPLC method

UPLC was carried out on a Waters UPLC using Kinetex EVo C18 column (100 x 2.1 mm, 1.7µm) at ambient temperature and a flow rate of 1.5ml/min.

Mobile phase 1: A = 5 mM Ammonium Acetate in water, B = 5 mM Ammonium Acetate in 90:10 Acetonitrile/water; Gradient profile from 95% A and 5% B to 65% A and 35% B in 2 min., then to 10% A and 90% B in 3.0 min., held at this composition for 2.0 min. and finally back to the initial composition for 6.0 min.

Mobile phase 2: A = 0.05 % formic acid in water, B = Acetonitrile; Gradient profile from 95 % A and 5 % B over 1 min., then 90 % A and 10 % B for 1 min., then 2 % A and 98 % B for 4 min. and then back to the initial composition for 6 min.

### General Procedure 1 (Method a)

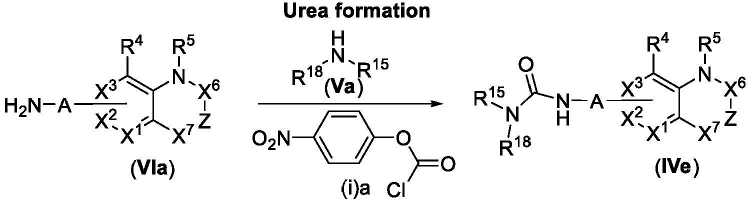

To a stirred solution of an aromatic amine of formula **(VIa)** (1.0 eq.) in a suitable solvent, such as THF, DMF, MeCN or DCM (8 mL/mmol) was added p-nitrophenyl chloroformate (1.2 eq.) at 0-5 °C and the whole stirred for 1-3h at RT. Then amine R¹⁵-NH-R¹⁸ **(Va)** (1.1 eq.) and TEA or DIPEA (6 eq.) were added dropwise successively at 0-5 °C and the whole further stirred for 1-5h at RT. Progress of the reaction was monitored by TLC/LCMS and after completion the reaction mass was diluted with water and extracted with EtOAc. The combined organic layers were washed with a dilute solution of a suitable inorganic base such as NaHCO₃ or 1N NaOH followed by 1N HCl and finally with brine. The organic layer was dried over anhydrous Na₂SO₄ and evaporated *in vacuo* to give a residue which was purified by column chromatography or Combi-flash or prep-HPLC to afford a compound of formula **(IVe)** (yield 6-70%) as solids. A similar procedure can be followed to synthesize all ureas of formula **(IVe).**

### General Procedure 1 (Method b)

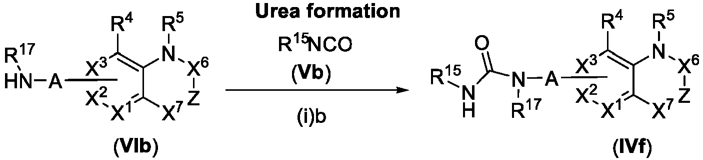

To a stirred solution of an aromatic amine of formula **(VIb)** (1.0 eq.) in a suitable solvent such as THF, DMF, MeCN or DCM (5.5 mL/mmol) was added R¹⁵NCO **(Vb)** (1.08 eq.) followed by TEA (1.08 eq.) at 0-5 °C and the whole stirred for 5-10 min. at the same temperature. The reaction mixture was brought slowly to RT and stirred for 1-2 h. Progress of the reaction was monitored by TLC and LC-MS. After completion, the reaction mixture was diluted with water and extracted with EtOAc. The combined organic layers were washed with brine, dried over anhydrous Na₂SO₄, filtered and evaporated under reduced pressure to afford a crude solid which was purified by column chromatography or Combi-flash or prep-HPLC to afford a compound of formula **(IVf)** (yield 10-70%) as solids. A similar procedure can be followed to synthesize all ureas of formula **(IVe).**

### General Procedure 1 (Method c)

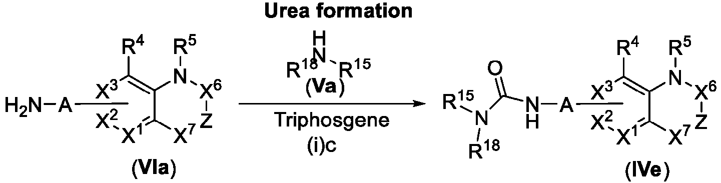

To a stirred solution of a compound of formula **(Va)** (1.0 eq.) in THF (10 mL/mmol) was added triphosgene (0.5 eq.) at 0-5 °C. The combined mixture was stirred at RT for 1 h. Completion of the first stage of the reaction was confirmed by TLC or UPLC-MS then, a compound of formula **(VIa)** (0.9 mmol) and TEA (2.5 eq.) were added into the reaction mixture and stirring continued at RT for 1-2 h. Progress of the reaction was monitored by TLC and or UPLC-MS. After completion of the reaction, the solvent was evaporated *in vacuo* to afford the crude material which was purified by column chromatography or prep-HPLC to give a compound of formula **(IVe)** (12-50% yield) as a solid.

### General Procedure 2a

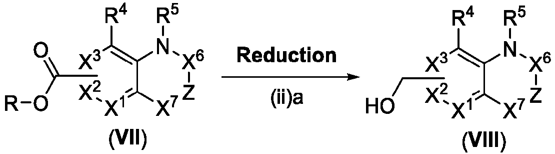

To a stirred solution of a compound of formula **(VII)** (1.0 eq.) in dry THF (5 mL/mmol) was added borane-THF (9 eq., 1M in THF) at 0-5 °C and the resulting solution was allowed to stir at room temperature for 3 h. After completion of the reaction the reaction mixture was quenched with methanol and concentrated *in vacuo* to obtain the crude compound which was purified by column chromatography to afford the compound of formula **(VIII)** (70-75% yield) as an off-white solid.

### General Procedure 2b

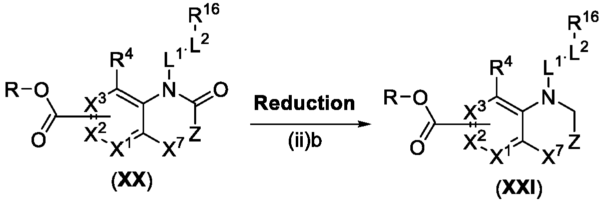

A stirred solution of a compound of formula **(XX)** (1.0 eq.) in THF (5 mL/mmol) was cooled to 0-5 °C and borane-THF complex (1M solution in THF) (10 mL/mmol, 10 eq.) added portion wise. The resulting reaction mixture was allowed to warm to RT, and then heated to reflux for 1-2 h. Progress of the reaction was monitored by UPLC-MS which showed formation of a compound of formula **(XXI).** After completion the reaction mixture was diluted with methanol and refluxed for 5-10 min., the solvent was evaporated to give a crude material which was purified by Combi-flash or column chromatography to afford a compound of formula **(XXI)** as a colorless oil.

### General Procedure 3

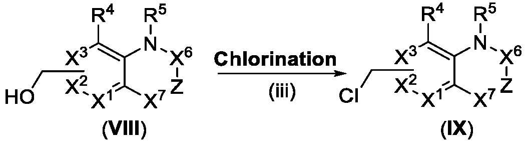

To a stirred solution of a compound of formula **(VIII)** (1.0 eq.) in DCM (7 mL/mmol) was added few drops of DMF followed by addition of SOCl₂ (2.0 eq) at RT. The whole was stirred at RT for 1-2 h. After completion of the reaction (monitored by LCMS), the reaction mixture was diluted with EtOAc and washed with water, followed by brine, dried over anhydrous Na₂SO₄, filtered and evaporated *in vacuo* to afford the compound of formula **(IX)** as a crude thick oil which was used in the next step without any further purification.

### General Procedure 4

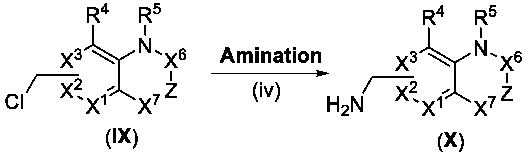

To a stirred solution of a compound of formula **(IX)** (1.0 eq.) in a sealed tube with THF (3mL/mmol) was added ammonia in methanol (6 mL/mmol) at RT. The reaction mixture was stirred at 80 °C for 10-18 h. Progress of the reaction was monitored by TLC and LCMS. After completion of the reaction, the solvent was evaporated and the residue was taken up in 10% MeOH in DCM and the remaining solid was filtered off. The filtrate was evaporated *in vacuo* to give the crude product which was triturated with hexane and diethyl ether to afford the compound of formula **(X)** as a crude solid which was used in the next step without any further purification.

### General Procedure 5

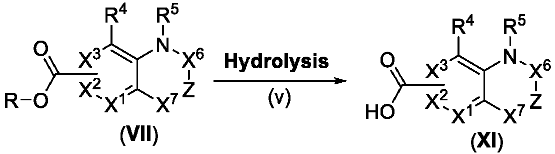

To a stirred solution of ester **(VII)** (1.0 eq.) in a mixture of MeOH or THF (6.5 mL/mmol) and water (0.8 mL/mmol) was added LiOH, NaOH or KOH (2.0 eq.) at RT and the resulting reaction mixture was stirred at RT for 2-16 h. TLC showed complete consumption of the ester **(VII).** The solvents were evaporated *in vacuo* and the resulting residue was washed with ether. The residue was then acidified with 1N HCl to pH 5-6, which resulted in the formation of a precipitate, which was filtered and washed with water and then dried by azeotropic distillation or under reduced pressure at 50-60 °C to afford the desired carboxylic acids of formula **(XI)** (70-85% yield) as solids.

### General Procedure 6

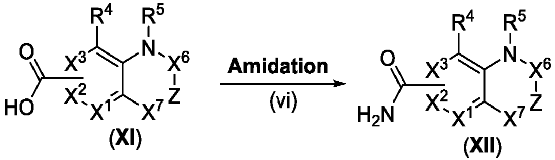

To a stirred solution of acid **(XI)** (1.0 eq.) in DMF (3 mL/mmol) was added HATU (1.2 eq.) and TEA (3.0 eq.) at 0-5 °C. The whole was stirred at RT for 10-15 min. followed by addition of ammonium formate (10.0 eq.). The resulting reaction mixture was stirred at RT for 16-20 h. Progress of the reaction was monitored by LCMS/TLC and after completion of the reaction, the reaction mixture was diluted with water and extracted with EtOAc, the organic layer was washed with brine, dried over anhydrous Na₂SO₄, filtered and evaporated *in vacuo* to afford the compound of formula **(XII)** as crude which was used in the next step without any further purification.

### General Procedure 7

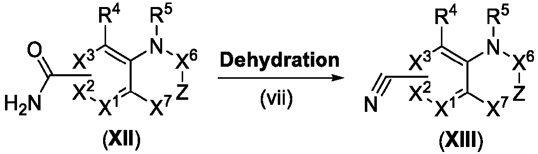

To a stirred solution of amide **(XII)** (1.0 eq.) in THF (3 mL)/mmol was added TEA (5.0 eq.) and trifluoroacetic anhydride (5.0 eq.) at 0-5 °C. The whole was maintained at RT for 1-2 h. Progress of the reaction was monitored by LCMS/TLC and after completion of the reaction, the reaction mixture was quenched with ice-water and extracted with EtOAc, the organic layer was washed with brine, dried over anhydrous Na₂SO₄, filtered and evaporated in vacuo to afford the compound of formula **(XIII)** as crude which was used in the next step without any further purification.

### General Procedure 8

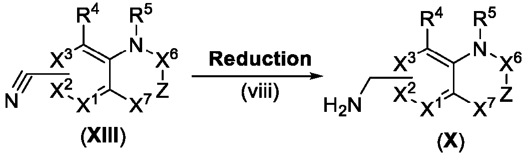

To a stirred solution of a compound of formula **(XIII)** (1.0 eq.) in MeOH (4 mL/mmol) was added (Boc)₂O (2.0 eq.), NiCl₅.5H₂O (0.5 eq.) and NaBH₄ (2.5 eq.) at 5-10 °C and the mixture was maintained at 10-15 °C to RT for 0.5-1 h. After completion of the reaction (monitored by TLC/LCMS), it was diluted with chilled water and the solvent was evaporated, extracted with EtOAc, the organic layer was washed with brine, dried over anhydrous Na₂SO₄, filtered and evaporated *in vacuo* to afford the crude product which was purified by trituration to give the intermediate Boc-protected amine. This was dissolved in 20% TFA in DCM (8 mL/mmol) under an inert atmosphere and stirred at RT for 0.5-1 h. Completion of the reaction was confirmed by LCMS, then the reaction mass was quenched with saturated NaHCO₃ solution (pH~8) and extracted with DCM, the organic layer was washed with brine, dried over anhydrous Na₂SO₄, filtered and evaporated *in vacuo* to afford the compound of formula **(X)** as crude which was used in the next step without any further purification.

### General Procedure 9

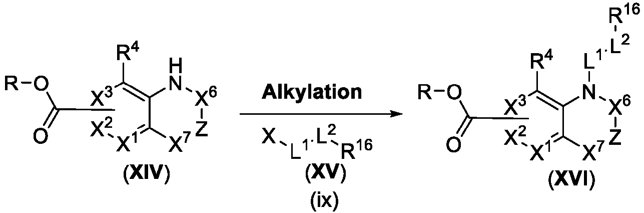

### Option A

To a stirred solution of a compound of formula **(XIV)** (1.0 eq.) in DMF or THF (4 mL/mmol) was added K₂CO₃, Cs₂CO₃, Na₂CO₃, NaOH or NaH (1.1 eq.). In the case where NaOH was used, TBAB (0.1 eq.) was also added as a phase transfer catalyst, followed by addition of a compound of formula **(XV)** (1.05 eq.) and the mixture allowed to stir at RT for 0.5-1 h. The reaction was monitored by TLC. After completion of the reaction the reaction mixture was quenched with a saturated solution of NH₄Cl, diluted with ice-cold water and extracted with EtOAc or MTBE. The organic layers were washed with brine, dried over anhydrous Na₂SO₄ and evaporated *in vacuo* to afford the crude product which was purified by Combi-flash using mixtures of EtOAc in hexanes as eluent to give compounds of formula **(XVI)** (60-80% yield) as colourless oils.

### Option B

Alternatively, to a stirred solution of a compound of formula **(XIV)** (1,0 eq.) in DCM or MeCN or THF (4 mL/mmol) was added TEA or DIPEA (2.0 eq.) or without the base followed by addition of a compound of formula **(XV)** (1.5 eq.) and the whole allowed to stir at RT for 0.5 to 1 h. The progress of the reaction was monitored by TLC. After completion of the reaction, the mixture was diluted with water, extracted with EtOAc, and the combined organic layers were washed with brine and dried over anhydrous Na₂SO₄. The organic layers were evaporated *in vacuo* to obtain the crude product which was purified by Combi-flash using mixtures of EtOAc in hexanes as eluent to afford compounds of formula **(XVI)** (60-80% yield) as colourless oils.

### General Procedure 10

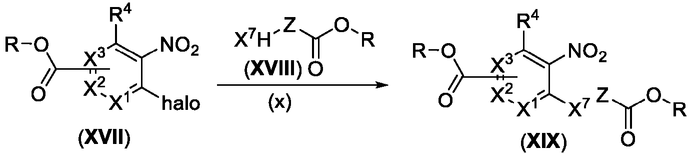

To a stirred solution of a compound of formula **(XVII)** (1.0 eq.) and a suitable nucleophile **(XVIII)** (1.25 eq.) in a suitable solvent, such as 1,4-dioxane, MeCN, DMF or THF (3 mL/mmol), was added dropwise or portionwise a suitable base such as TEA, DBU, NaH or K₂CO₃ (1.5 eq.) with ice bath cooling and the combined mixture allowed to stir at 0-25 °C for 1-16 h. Progress of the reaction was monitored by TLC or LCMS and on completion of the reaction the mixture was quenched with a saturated aqueous solution of NH₄Cl and extracted with EtOAc. The combined organic layers were washed with brine, dried over anhydrous Na₂SO₄ and evaporated *in vacuo* to dryness. The crude compounds of formula **(XIX)** (60-95% yield) obtained as solids were pure enough to be used directly in the next step without any further purification.

### General Procedure 11

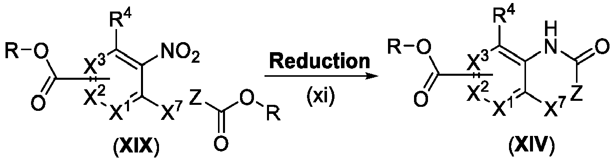

### Option A (Reduction by Fe/Zn-AcOH/HCl/NH4Cl)

To a stirred solution of a compound of formula **(XIX)** (1.0 eq.) in EtOH or MeOH (2 mL/mmol) was added a suitable acid, such as AcOH or aq. HCl (3 mL/mmol) followed by iron powder or zinc powder (4.0 eq.) at RT. In some cases NH₄Cl was also used as a source of hydrogen. The reaction mixture was stirred at 75-85 °C for 1-5 h. The reaction was monitored by TLC or LCMS and after completion the reaction mixture was poured into ice-cold water and filtered through a short celite bed. The filtrate was extracted with EtOAc and then washed with aqueous NaHCO₃ and then brine. The collected organic layers were dried over anhydrous Na₂SO₄ and concentrated *in vacuo* to afford compounds of formula **(XIV)** (60-80% yield) as crude solids, which were used in the next step without any further purification.

### Option B: (Reduction by Sodium dithionate)

To a stirred solution of a compound of formula **(XIX)** (1.0 eq.) in a mixture of either MeCN/H₂O or THF/H₂O (12 mL/mmol, 2:1) was added sodium hydrosulphite (8.0 eq.), *tetra*-butyl ammonium hydrosulphate (0.5 eq.) and K₂CO₃ (6.0 eq.) at RT and the mixture then stirred for 1 h. Progress of the reaction was monitored by TLC and or LCMS. After completion of the reaction the solvents were evaporated *in vacuo* to give an oily liquid which was dissolved in 1N HCl and extracted with EtOAc. The combined organic layers were washed with brine and dried over anhydrous Na₂SO₄. The organics were filtered and evaporated *in vacuo* to give a compound of formula **(XIV)** (80-90% yield) as solids.

### Option C: (Reduction by Pd/C/H₂)

To a stirred solution of a compound of formula **(XIX)** (1.0 eq.) in EtOAc, MeOH or EtOH (9.4 mL/mmol, 120 mL) was added 10% Pd-C (50% w/w in water) (77.8 mg/mmol) under an inert atmosphere at room temperature. The reaction mixture was purged with H₂ gas using balloon pressure and then allowed to further stir for 3-5 h at room temperature. The course of the reaction was monitored by TLC and/or LCMS. After completion of the reaction the mixture was diluted with EtOAc, filtered carefully through a bed of celite and washed with EtOAc 4-5 times until the mother liquor showed no compound remaining by TLC. Then the collected organic layers were dried over anhydrous Na₂SO₄, filtered and concentrated under reduced pressure to give a compound of formula **(XIV)** (80-85 % yield) as semi-solids. The products were pure enough to use in the next step without any further purification.

### General Procedure 12

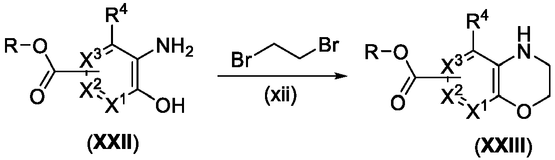

To a stirred solution of a compound of formula **(XXII)** (1.0 eq.) in DMF or THF (1.6 mL/mmol) was added K₂CO₃, Cs₂CO₃, Na₂CO₃, NaOH or NaH (4.0 eq.) at RT and then 1,2-dibromoethane (4.0 eq.) was added and the reaction mass maintained at 80-85 ^{O}C for 10-16 h. Progress of the reaction was monitored by TLC and UPLC-MS which showed formation of the desired product. After completion of the reaction, the reaction mixture was diluted with water and extracted with EtOAc. The combined organics were washed with brine, dried over anhydrous Na₂SO₄ and evaporated *in vacuo* to afford a crude material which was purified by Combi-flash using suitable solvents to afford compounds of formula **(XXIII)** (50-55% yield) as solids.

### General Procedure 13

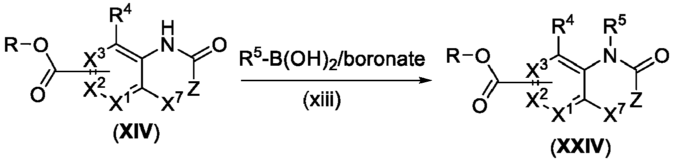

To a stirred solution of a compound of formula **(XIV)** (1.0 eq.) in EDC (1.1 mL/mmol) was added R⁵-B(OH)₂/boronate (1.5 eq.) in EDC or toluene (1.1 mL/mmol), DBU (2.0 eq.) and a solution of Cu(OAc) (2.0 eq.) at RT. The resulting reaction mixture was stirred at RT for 20-24 h. Progress of the reaction was monitored by LCMS and after completion the reaction mixture was diluted with water and extracted with EtOAc. The organic layer was washed with brine, dried over anhydrous Na₂SO₄ and evaporated in *vacuo* to afford the crude material which was purified by Combi-flash using suitable solvents to afford a compound of formula **(XXIV)** (34-40% yield) as a solid.

### General Procedure 14

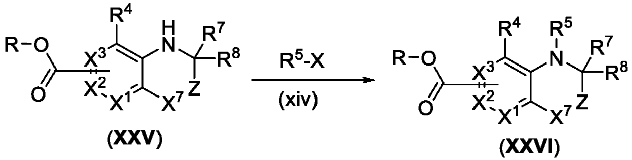

To a stirred solution of a compound of formula **(XXV)** (1.0 eq.) in toluene or dioxane or EDC (6 mL/mmol) was added R⁵-X (where X is a suitable leaving group) (1.5 eq.), cesium carbonate (2.0 eq.) and BINAP (0.2 eq.) at RT. The whole was degassed with nitrogen for 20 min., then palladium acetate (0.1 eq.) was added into the reaction mixture and stirring continued at 100-110 °C for 20-24 h. Progress of the reaction was monitored by UPLC-MS and after completion the reaction mixture was concentrated in *vacuo* to give a crude material which was purified by column chromatography using suitable solvents to afford a compound of formula **(XXVI)** (30-35% yield) as a solid.

### General Procedure 15

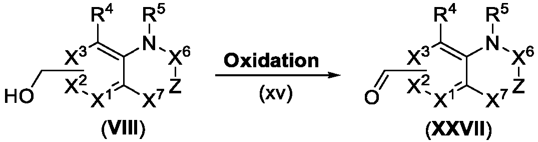

To a stirred solution of a compound of formula **(VIII)** (1.0 eq.) in DCM (6 mL/mmol) was added MnO₂ (10.0 eq.) and the reaction mixture was stirred at room temperature for 10-16 h. Completion of the reaction was confirmed by LCMS and then the reaction mixture was filtered through a celite bed and concentrated under reduced pressure to give the crude product which was purified by silica gel column chromatography using suitable solvents to afford the compound of formula **(XXVII)** (50-60% yield).

### General Procedure 16

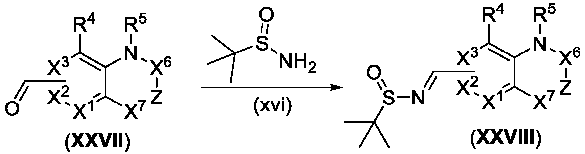

To a stirred solution of a compound of formula **(XXVII)** (1.0 eq.) in THF (8 mL/mmol) was added 2-methylpropane-2-sulfinamide (1.5 eq.) at room temperature. Then titanium isopropoxide (2.0 eq.) was added dropwise to the solution at the same temperature and the reaction mixture was stirred for 15-20 h. Completion of the reaction was confirmed by LCMS, then the reaction mixture was quenched with saturated NaHCO₃ solution and extracted with EtOAc. The combined organic layers were washed with brine solution, dried over anhydrous Na₂SO₄ and evaporated under reduced pressure to give crude material which was purified by silica gel column chromatography using suitable solvents to afford the compound of formula (55-60% yield).

### General Procedure 17

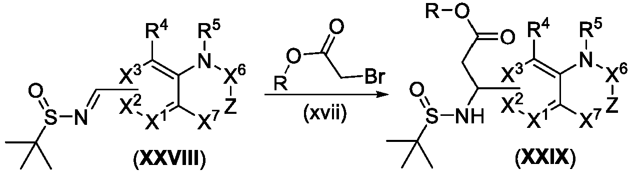

To a stirred suspension of Zn (10. eq.) in THF (10 mL/mmol) was added iodine (0.1 eq.) at room temperature and the whole was heated at reflux for 30 min. Then to the reaction mixture was added a mixture of a compound of formula **(XXVIII)** (1.0 eq.) and alkyl bromoacetate (4.0 eq.) in THF (6 mL/mmol). The resulting reaction mixture was refluxed for 2-3 h. Progress of the reaction was monitored by LCMS and after completion, the reaction was filtered through a celite bed. The filtrate was quenched with water and the organic parts were extracted with EtOAc. The combined organic layers were washed with brine, dried over anhydrous Na₂SO₄and concentrated under reduced pressure to obtain the crude product. The crude was purified by silica gel column chromatography using suitable solvents to afford the title compound (60-65% yield).

### General Procedure 18

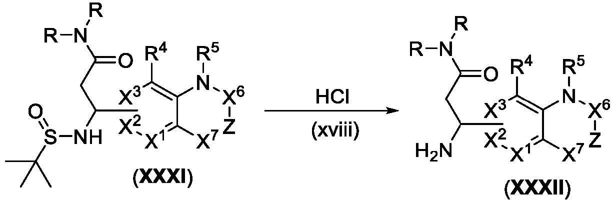

To a stirred solution of a compound of formula **(XXXI)** (1.0 eq.) in EtOH (11 mL/mmol) was added 1.25M HCl in MeOH (2.3 mL/mmol) at 0-5 °C and the whole was stirred for 1-2 h. After completion of the reaction (monitored by LCMS), the excess solvent was evaporated under reduced pressure and the reaction mass was quenched with saturated NaHCO₃ solution and extracted with 10% MeOH-DCM. The combined organic layers were washed with brine, dried over anhydrous Na₂SO₄ and concentrated under reduced pressure to afford the compound of formula **(XXXII)** as crude which was used in the next step without any further purification.

### General Procedure 19

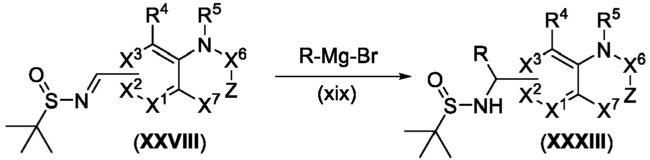

A solution of a compound of formula **(XXVIII)** (prepared according to methods described in General Procedure 16) (1.0 eq.) in THF (6 mL/mmol) was charged by slow addition of R-Mg-Br (3.0 eq.; 1M in diethyl ether) at -78 °C. The mixture was stirred at the same temperature for 1-2 h. After completion of the reaction, the reaction mixture was quenched with a saturated solution of NH₄Cl and extracted with EtOAc. The combined organic layer was washed with brine, dried over anhydrous Na₂SO₄ and concentrated under reduced pressure to obtain the crude product. The crude was purified by silica gel column chromatography using suitable solvents to afford the compound of formula **(XXXIII)** (70-75% yield).

### General Procedure 20

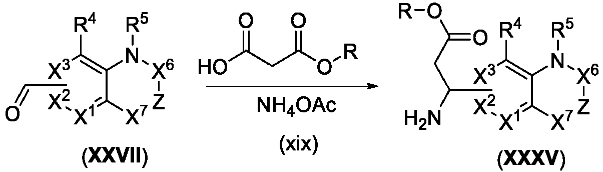

To a stirred solution of a compound of formula **(XXVII)** (1.0 eq.) in MeOH (10 mL/mmol) was added excess NH₄OAc (34 eq.) and 3-alkyl-3-oxopropanoic acid (2.0 eq.) at RT. The whole was allowed to stir at RT for 2-3 h. Then another portion of NH₄OAc (34 eq.) was added and the combined mixture was heated at 80-85 °C for 15-20 h. Progress of the reaction was monitored by TLC/LCMS and after completion of the reaction the solvent was evaporated to give the crude product which was purified by silica gel column chromatography using suitable solvents to afford the compound of formula **(XXXV)** (10-15% yield).

### Examples

Nuclear magnetic resonance (NMR) spectra were in all cases consistent with the proposed structures. Characteristic chemical shifts (δ) are given in parts-per-million downfield from tetramethylsilane (for ¹H-NMR) and upfield from trichloro-fluoromethane (for ¹⁹F NMR) using conventional abbreviations for designation of major peaks: e.g. s, singlet; d, doublet; t, triplet; q, quartet; m, multiplet; br, broad. The following abbreviations have been used for common solvents: CDCl₃, deuterochloroform; d₆-DMSO, deuterodimethylsulphoxide; and CD₃OD, deuteromethanol.

Mass spectra, MS (m/z), were recorded using electrospray ionisation (ESI). Where relevant and unless otherwise stated the m/z data provided are for isotopes ¹⁹F, ³⁵Cl, ⁷⁹Br and ¹²⁷I.

All chemicals, reagents and solvents were purchased from commercial sources and used without further purification. All reactions were performed under an atmosphere of nitrogen unless otherwise noted.

Flash column chromatography was carried out using pre-packed silica gel cartridges in a Combi-Flash platform. Prep-HPLC purification was carried out according to the General purification and analytical methods described above. Thin layer chromatography (TLC) was carried out on Merck silica gel 60 plates (5729). All final compounds were >95% pure as judged by the LCMS or UPLC analysis methods described in the General Purification and Analytical methods above unless otherwise stated.

### Example 1: 1-((4-Benzyl-3,4-dihydro-2H-benzo[b][1,4]thiazin-6-yl)methyl)-3-(1H-indol-6-yl)urea

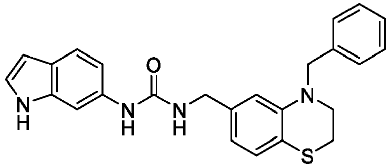

Example 1 was prepared according to the methods described in General Procedures 1-4, 9-11 and the methods described below.

### Preparation 1: (4-Benzyl-3,4-dihydro-2H-benzo[b][1,4]thiazin-6-vl)methanamine

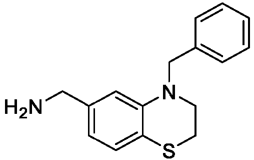

### Step 1: Methyl 4-((2-ethoxy-2-oxoethyl)thio)-3-nitrobenzoate

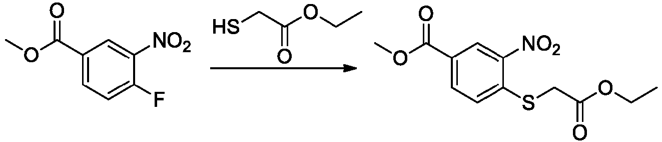

Methyl 4-fluoro-3-nitrobenzoate (10.0 g, 50.2 mmol) was taken up in MeCN (2.0 L) and TEA (7.61 g, 75.38 mmol) was added to the solution. The reaction mixture was cooled to 0-5 °C and ethyl thioglycolate (7.25 g, 62.7 mmol) was added dropwise. The reaction mixture was stirred for 30 min. at ice-cold temperature. It was then diluted with EtOAc and washed with a saturated solution of NH₄Cl and brine. The organic layer was dried over anhydrous Na₂SO₄ and evaporated *in vacuo* to dryness to give the title compound (14.0 g, 46.82 mmol, 93% yield) as a yellow solid, which was pure enough to be used in the next step without any further purification. LCMS m/z: 300.06 [M+H].

### Step 2: Methyl 3-oxo-3,4-dihydro-2H-benzo[b-1,4]thiazine-6-carboxylate

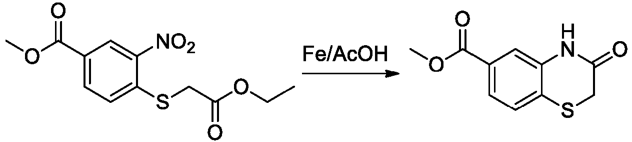

To a stirred solution of methyl 4-((2-ethoxy-2-oxoethyl)thio)-3-nitrobenzoate (Preparation 1, Step 1) (5.0 g, 16.7 mmol) in acetic acid (50 mL) was added iron powder (3.73 g, 66.8 mmol). The resulting reaction mixture was stirred at 80 °C for 3 h. On completion (monitored by TLC), the reaction was cooled to room temperature and poured onto 1N HCl (250 mL) and then stirred for 1 h. The resulting white precipitate was filtered off and washed with water. The residue obtained was re-dissolved in 5% MeOH in DCM (50 mL) and filtered through a bed of celite. The filtrate was evaporated to dryness in *vacuo* to afford the title compound (3.5 g, 15.6 mmol, 91% yield) as a pale yellow solid. LCMS m/z: 222.05 [M-H].

### Step 3: Methyl 4-benzyl-3-oxo-3,4-dihydro-2H-benzo[b][1,4]thiazine-6-carboxylate

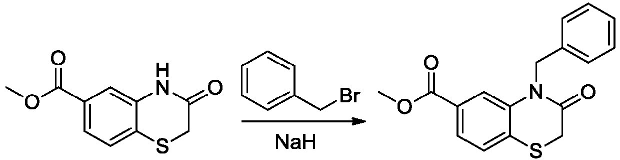

To a stirred solution of methyl 3-oxo-3,4-dihydro-2*H*-benzo[b-1,4]thiazine-6-carboxylate (Preparation 1, Step 2) (5.0 g, 22.2 mmol) in DMF (50 mL) at 0-5 °C was added NaH (0.98 g, 24.4 mmol) portionwise and the whole stirred for another 5-10 min. at the same temperature. Then, benzyl bromide (2.8 mL, 23.3 mmol) was added and the reaction mixture was stirred for 1 h. Completion of the reaction was monitored by TLC and LC-MS. After completion, the reaction mixture was quenched with a saturated solution of NH₄Cl and diluted with ice-cold water. The aqueous reaction mixture was extracted with MTBE and washed with brine. The separated organic layer was then dried over anhydrous Na₂SO₄ and concentrated under reduced pressure to afford the title compound (9.0 g) as a crude pale yellow solid which was used in the next step without any further purification. LCMS m/z: 314.16 [M+H].

### Step 4: (4-Benzyl-3,4-dihydro-2H-benzo[b][1,4]thiazin-6-yl)methanol

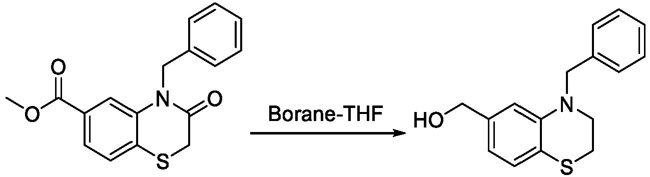

To a stirred solution of methyl 4-benzyl-3-oxo-3,4-dihydro-2H-benzo[b][1,4]thiazine-6-carboxylate (Preparation 1, Step 3) (1.4 g, 4.47 mmol) in THF (15 mL) was added borane-THF complex (13.4 mL, 13.4 mmol; 1M soln in THF) at 0-5 °C and the whole reaction mixture was refluxed for 2 h. UPLC-showed formation of the desired compound and after completion of the reaction, the reaction mixture was cooled to RT and diluted with methanol (20 mL). The resulting mixture was further refluxed for 10 min., then the solvent was evaporated to afford the crude material which was purified by column chromatography to afford the title compound (500 mg) as a white solid. LCMS m/z: 274 [M+H].

### Step-5: 4-Benzyl-6-(chloromethyl)-3,4-dihydro-2H-benzo[b][1,4]thiazine

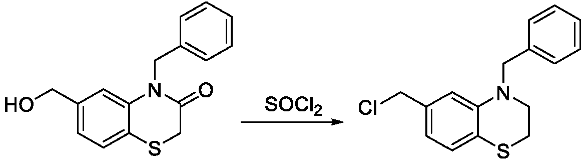

To a stirred solution of 4-benzyl-6-(hydroxymethyl)-2H-benzo[b][1,4]thiazin-3(4H)-one (Preparation 1, Step 4) (900 mg, 3.32 mmol) in DCM (25 mL ) was added a few drops of DMF followed by addition of SOCl₂ (790 mL, 6.63 mmol) at RT. The whole was stirred at RT for 1 h. After completion of the reaction (monitored by LCMS), the reaction mixture was diluted with EtOAc and washed with water, followed by brine, dried over anhydrous Na₂SO₄, filtered and evaporated *in vacuo* to afford the title compound (1.0 g, crude) as a brownish thick oil which was used in the next step without any further purification. LCMS m/z: 290 [M+H].

### Step-6: (4-Benzyl-3,4-dihydro-2H-benzo[b][1,4]thiazin-6-yl)methanamine

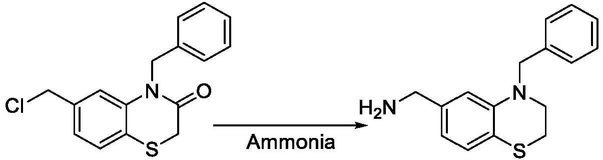

To a stirred solution of 4-benzyl-6-(chloromethyl)-2H-benzo[b][1,4]thiazin-3(4H)-one (Preparation 1, Step 5) (900 mg, 3.11 mmol) in a sealed tube with THF (10 mL) was added ammonia in methanol (20 mL) at RT. The reaction mixture was stirred at 80 °C for 16 h. Progress of the reaction was monitored by TLC and LCMS. After completion of the reaction, the solvent was evaporated and the residue was dissolved in 10% MeOH in DCM and the remaining solid was filtered off. The filtrate was evaporated *in vacuo* to give the crude product which was triturated with hexane and diethyl ether to afford the title compound (450 mg, crude) as a grayish solid which was used in the next step without any further purification. LCMS m/z: 271 [M+H].

### Preparation 2: 1-((4-Benzyl-3,4-dihydro-2H-benzo[b][1,4]thiazin-6-yl)methyl)-3-(1H-indol-6-yl)urea (Example 1)

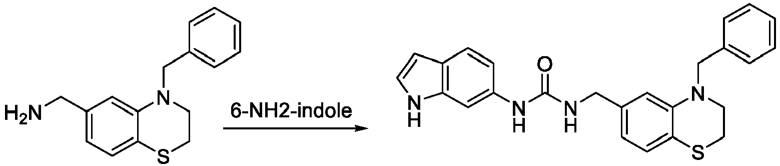

To a stirred solution of 6-NH₂-indole (54 mg, 0.406 mmol) in THF (5 mL) was added triphosgene (54 mg, 0.406 mmol) at 0-5 °C and the temperature was maintained at RT for 1 h. TLC showed completion of the reaction; (4-benzyl-3,4-dihydro-2H-benzo[b][1,4]thiazin-6-yl)methanamine (Preparation 1, Step 6) (100 mg, 0.369 mmol) and TEA (0.176 mL, 1.217 mmol) were then added at RT and the mixture was further stirred at RT for 1 h. Progress of the reaction was monitored by TLC and LCMS and after completion of the reaction it was diluted with EtOAc and washed with water followed by brine, dried over anhydrous Na₂SO₄, filtered and evaporated *in vacuo* to afford the crude product which was purified by prep-HPLC to give the title compound (7 mg, 4.4% yield ) as a faint brownish solid. Purity by HPLC: 97.99%; 1H NMR (400 MHz; DMSO-d₆): δ 3.06 (t, *J =* 5.04 Hz, 2H), 3.63 (t, *J =* 4.8 Hz, 2H), 4.09 (d, *J =* 5.6 Hz, 2H), 4.53 (s, 2H), 6.30 (s, 1H), 6.35-6.40 (m, 1H), 6.52 (d, *J =* 8 Hz, 1H), 7.67 (s, 1H), 6.77 (d, *J =* 8.64 Hz, 1H), 6.93 (d, *J =* 7.88 Hz, 1H), 7.17 (t, *J =* 2.68 HZ, 1H), 7.21-7.23 (m, 1H), 7.28-7.30 (m, 4H), 7.34 (d, *J =* 8.32 Hz, 1H), 7.73 (s, 1H), 8.38 (s, 1H), 10.84 (s, 1H); LCMS m/z: 429.13 [M+H].

### Example 2: 1-((4-Benzyl-3-oxo-3,4-dihydro-2H-benzo[b][1,4]oxazin-7-yl)methyl)-3-(1H-indol-6-yl)urea

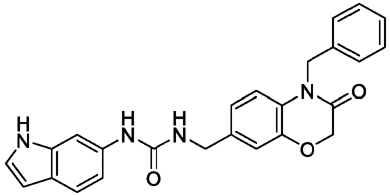

Example 2 was prepared according to the methods described in General Procedures 1-2, 5-11 and the methods described below.

### Preparation 3: 7-(Aminomethyl)-4-benzyl-2H-benzo[b][1,4]oxazin-3(4H)-one

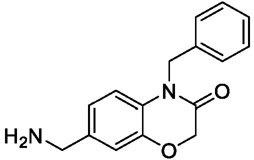

### Step 1: Methyl 3-(2-methoxy-2-oxoethoxy)-4-nitrobenzoate

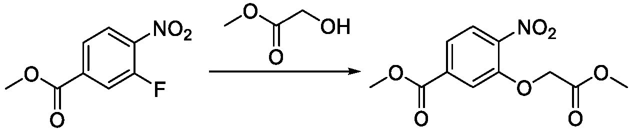

To a stirred solution of of NaH (1.5 g, 376 mmol) in 1,4-dioxane (50 mL) was added methyl 2-hydroxyacetate (3.39 g, 376 mmol) at 5-10 °C and the reaction mixture was maintained at the same temperature for 30 min. followed by addition of commercially available methyl 3-fluoro-4-nitrobenzoate (5.0 g, 251 mmol) in 1,4-dioxane (25 mL) solution. The whole was stirred at RT for 16 h. After completion of the reaction (monitored by LCMS), the reaction mixture was quenched with ice-cold water and stirred for 15 min. The precipitated solid was filtered and washed with water and dried under vacuum to afford the title compound (5.0 g, crude) as a pale yellow solid which was used in the next step without any further purification. LCMS m/z: 269.98 [M+H].

### Step 2: Methyl 3-oxo-3,4-dihydro-2H-benzo[b][1,4]oxazine-7-carboxylate

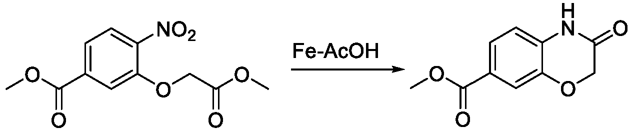

To a stirred solution of methyl 3-(2-methoxy-2-oxoethoxy)-4-nitrobenzoate (Preparation 3, Step 1) (5.0 g, 18.57 mmol) in AcOH (25 mL) was added Fe-powder (4.15 g, 74.304 mmol) at RT. The reaction mixture was stirred at 90 °C for 2 h. The reaction was monitored by LCMS/TLC and after completion the reaction mixture was quenched into ice-cold water (500 mL) and stirred for 30 min. The precipitated solid was filtered and washed with excess water and then dried under vacuum to afford the title compound (3.8 g, crude) as an ash colored solid which was used in the next step without any further purification. LCMS m/z: 207.98 [M+H].

### Step 3: Methyl 4-benzyl-3-oxo-3,4-dihydro-2H-benzo[b][1,4]oxazine-7-carboxylate

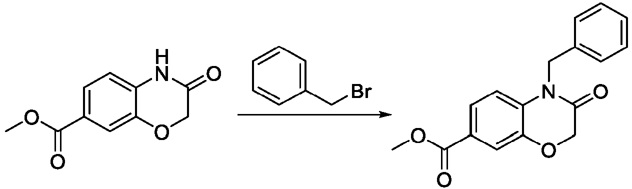

To a stirred solution of methyl 3-oxo-3,4-dihydro-2H-benzo[b][1,4]oxazine-7-carboxylate (Preparation 3, Step 2) (2.0 g, 9.65 mmol) in DMF (20 mL) was added NaH (425 mg, 10.62 mmol) followed by benzyl bromide (1.27 mL, 10.62 mmol) at 0-10 °C. The whole was stirred at 10 °C to RT for 1h. The reaction was monitored by LCMS/TLC and after completion the reaction mixture was diluted with ice cold water, the precipitated solid was filtered off, washed with excess water and dried *in vacuo* to afford the title compound (2.6 g, crude) as a brown solid which was used in the next step without any further purification. LCMS m/z: 298.88 [M+H].

### Step 4: 4-Benzyl-3-oxo-3,4-dihydro-2H-benzo[b][1,4]oxazine-7-carboxylic acid

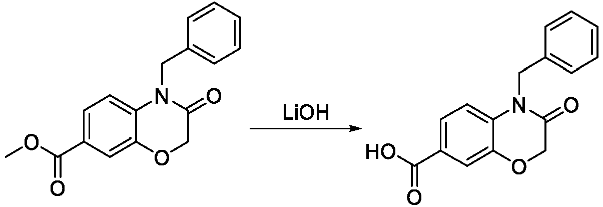

To a stirred solution of methyl 4-benzyl-3-oxo-3,4-dihydro-2H-benzo[b][1,4]oxazine-7-carboxylate (Preparation 3, Step 3) (2.6 g, 8.75 mmol) in THF (30 mL) and MeOH (15 mL) was added a solution of LiOH.H₂O (1.83 g, 43.73 mmol) in water (15 mL) and the mixture was maintained at RT for 24 h. After completion of the reaction, the solvents were evaporated to give a residue which was diluted with water, washed with diethyl ether and the aqueous part was acidified with 6N HCl. The precipitated solid was filtered, washed and dried *in vacuo* to afford the title compound (2.2 g, crude) as an off-white solid which was used in the next step without any further purification. LCMS m/z: 284.02 [M+H].

### Step 5: 4-Benzyl-3-oxo-3,4-dihydro-2H-benzo[b][1,4]oxazine-7-carboxamide

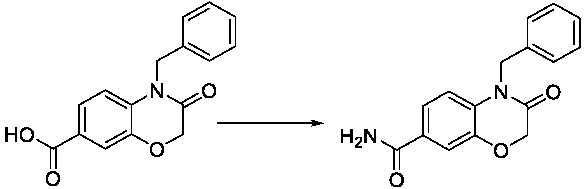

A stirred solution of 4-benzyl-3-oxo-3,4-dihydro-2H-benzo[b][1,4]oxazine-7-carboxylic acid (Preparation 3, Step 4) (500 mg, 1.765 mmol) in DMF (5 mL) was cooled to 0-5 °C followed by addition of HATU (803 mg, 2.12 mmol) and TEA (0.764 mL, 5.23 mmol). The whole was stirred at RT for 10 min. followed by addition of ammonium format (1.1 g, 17.65 mmol) and the whole maintained at RT for 16 h. Progress of the reaction was monitored by LCMS/TLC and after completion of the reaction, the reaction mixture was diluted with water and extracted with EtOAc, the organic layer was washed with brine, dried over anhydrous Na₂SO₄, filtered and evaporated *in vacuo* to afford the title compound (500 mg, crude) as a pale yellow oil which was used in the next step without any further purification. LCMS m/z: 283 [M+H].

### Step 6: 4-Benzyl-3-oxo-3,4-dihydro-2H-benzo[b][1,4]oxazine-7-carbonitrile

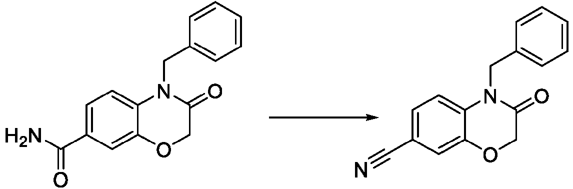

A stirred solution of 4-benzyl-3-oxo-3,4-dihydro-2H-benzo[b][1,4]oxazine-7-carboxamide (Preparation 3, Step 5) (0.5 g, 1.77 mmol) in THF (5 mL) was cooled to o-5 °C followed by addition of TEA (1.28 mL, 8.85 mmol) and trifluoroacetic anhydride (0.744 mL, 5.313 mmol) and the whole was maintained at RT for 1 h. Progress of the reaction was monitored by LCMS/TLC and after completion of the reaction, the reaction mixture was quenched with ice-water and extracted with EtOAc, the organic layer was washed with brine, dried over anhydrous Na₂SO₄, filtered and evaporated in vacuo to afford the title compound (500 mg, crude) as a faint yellow oil which was used in the next step without any further purification. LCMS m/z: 264.98 [M+H].

### Step 7: tert-Butyl ((4-benzyl-3-oxo-3,4-dihydro-2H-benzo[b][1,4]oxazin-7-yl)methyl)carbamate

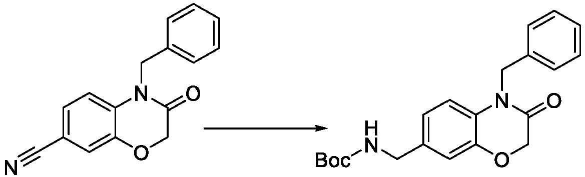

To a stirred solution of 4-benzyl-3-oxo-3,4-dihydro-2H-benzo[b][1,4]oxazine-7-carbonitrile (Preparation 3, Step 6) (0.3 g, 1.14 mmol) in MeOH (5 mL) was added (Boc)₂O (0.496 mL, 2.27 mmol), NiCl₂.5H₂O (135 mg, 0.57 ,mmol) and NaBH₄ (108 mg, 2.84 mmol) at 5-10 °C and the mixture was maintained at 10 °C to RT for 0.5 h. After completion of the reaction (monitored by TLC/LCMS), it was diluted with chilled water and the solvent was evaporated, extracted with EtOAc, the organic layer was washed with brine, dried over anhydrous Na₂SO₄, filtered and evaporated *in vacuo* to afford the crude product which was purified by trituration to give the title compound (300 mg, crude) as an off-white solid which was used in the next step without any further purification. LCMS m/z: 369 [M+H].

### Step 8: 7-(Aminomethyl)-4-benzyl-2H-benzo[b][1,4]oxazin-3(4H)-one

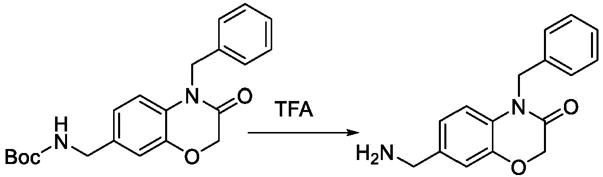

A solution of *tert-*butyl ((4-benzyl-3-oxo-3,4-dihydro-2H-benzo[b][1,4]oxazin-7-yl)methyl)carbamate (Preparation 3, Step 7) (300 mg, 0.81 mmol) in 20% TFA in DCM (10 mL) under an inert atmosphere was stirred at RT for 0.5 h. Completion of the reaction was confirmed by LCMS, then the reaction mass was quenched with saturated NaHCO₃ solution (pH~8) and extracted with DCM, the organic layer was washed with brine, dried over anhydrous Na₂SO₄, filtered and evaporated *in vacuo* to afford the title compound (180 mg, crude) as an off-white solid which was used in the next step without any further purification. LCMS m/z: 269.8 [M+H].

### Preparation 4: 1-((4-Benzyl-3-oxo-3,4-dihydro-2H-benzo[b][1,4]oxazin-7-yl)methyl)-3-(1H-indol-6-yl)urea (Example 2)

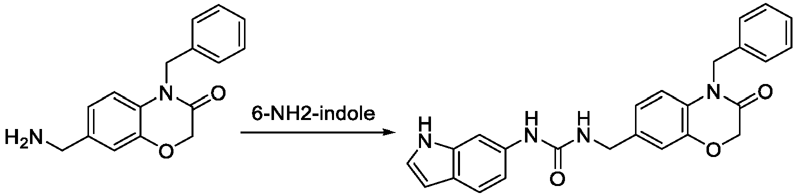

To a stirred solution of 6-amino indole (48 mg, 0.368 mmol) in THF (5 mL) was added triphosgene (50 mg, 0.167 mmol) at 0-5 °C and the mixture was maintained at RT for 1 h. TLC showed completion of the reaction, then 7-(amino methyl)-4-benzyl-2H-benzo[b][1,4]oxazin-3(4H)-one (90 mg, 0.335 mmol) (Preparation 3, Step 8) and TEA (0.16 mL, 1.105 mmol) were added. The resulting reaction mixture was stirred at RT for 1 h. Progress of the reaction was monitored by LCMS/TLC and after completion, the reaction mixture was diluted with EtOAc and washed with water followed by brine, dried over anhydrous Na₂SO₄, filtered and evaporated *in vacuo* to afford the crude product which was purified by prep-HPLC to give the title compound (20 mg, 14% yield) as an off-white solid. Purity by HPLC: 98.72%; 1H NMR (400 MHz; DMSO-d₆): δ 4.21 (d, *J =* 8.84 Hz, 2H), 4.78 (s, 2H), 5.15 (s, 2H), 6.29 (s, 1H), 6.48 (t, *J =* 5.92 Hz, 1H), 6.76-6.78 (m, 1H), 6.89-6.90 (m, 1H), 6.96-7.00 (m, 2H), 7.17 (t, *J =* 2.64 Hz, 1H), 7.22-7.24 (m, 1H), 7.26-7.28 (m, 2H), 7.30 (bs, 1H), 7.32-7.35 (m, 2H), 7.72 (s, 1H), 8.39 (s, 1H), 10.83 (s, 1H); LCMS m/z: 427.08 [M+H].

### Example 3: 1-((4-Benzyl-3,4-dihydro-2H-benzo[b][1,4]oxazin-7-yl)methyl)--3-(1H-indol-6-yl)urea

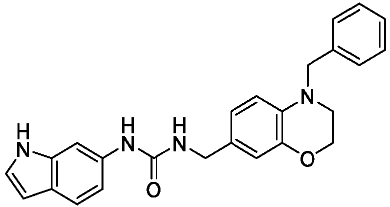

Example 3 was prepared according to the methods described in General Procedures 1-4, 10-11 and the methods described below.

### Preparation 5: (4-Benzyl-3,4-dihydro-2H-benzo[b][1,4]oxazin-7-yl)methanamine

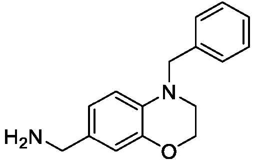

### Step 1: (4-Benzyl-3,4-dihydro-2H-benzo[b][1,4]oxazin-7-yl)methanamine

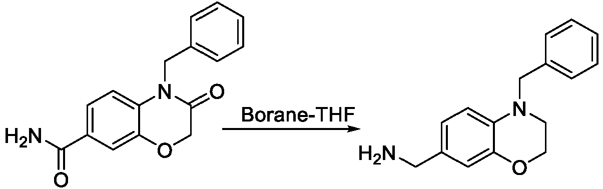

A stirred solution of 4-benzyl-3-oxo-3,4-dihydro-2H-benzo[b][1,4]oxazine-7-carboxamide (Preparation 3, Step 5) (0.2 g, 0.71 mmol) in borane-THF (4.3 mL, 4.25 mmol) was refluxed at 60 °C for 1 h. After completion of the reaction, the reaction mixture was quenched with methanol followed by evaporation of the solvent under vacuum. The reaction mixture was diluted with water and extracted with EtOAc and washed with water followed by brine, dried over anhydrous Na₂SO₄, filtered, and evaporated *in vacuo* to afford the title compound (180 mg, crude) as a faint yellow oil which was used in the next step without any further purification. LCMS m/z: 255.14 [M+H].

### Preparation 6: 1-((4-Benzyl-3,4-dihydro-2H-benzo[b][1,4]oxazin-7-yl)methyl)-3-(1H-indol-6-yl)urea (Example 3)

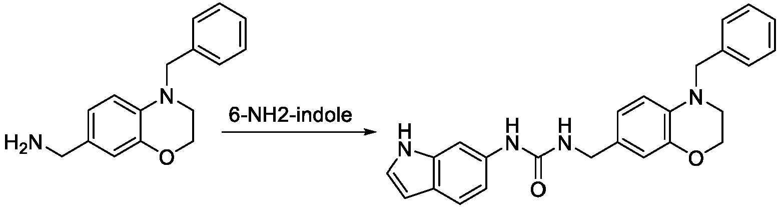

To a stirred solution of 6-amino indole (180 mg, 0.79 mmol) in THF (6 mL) was added triphosgene (117 mg, 0.39 mmol) at 0-5 °C and the mixture was maintained at RT for 1 h. TLC showed completion of the reaction, then (4-benzyl-3,4-dihydro-2H-benzo[b][1,4]oxazin-7-yl)methanamine (Preparation 5, Step 1) (125 mg, 0.95 mmol) and TEA (0.327 mL, 2.36 mmol) were added and the combined mixture was further stirred at RT for 1 h. Progress of the reaction was monitored by LCMS and after completion the reaction mixture was diluted with EtOAc and washed with water followed by brine, dried over anhydrous Na₂SO₄, filtered and evaporated *in vacuo* to give the crude product which was purified by prep-HPLC to afford the title compound (20 mg, 6% yield) as an off-white solid. Purity by HPLC: 98.72%; ¹H NMR: (500 MHz; DMSO-d₆): δ 4.16 (d, *J =* 5.3 Hz, 2H), 4.26 (t, *J =* 4.15 Hz, 2H), 4.52 (S, 2H), 6.35 (s, 1H), 6.39 (t, *J =* 6.15 Hz, 1H), 6.67-6.74 (m, 4H), 6.81-6.83 (dd, *J*1 = 1.6 Hz, *J*2 = 8.4 HZ, 1H), 7.23 (t, *J =* 2.55 Hz, 1H), 7.30-7.41 (m, 7H), 7.81 (s, 1H), 8.38 (s, 1H), 10.90 (s, 1H); LCMS m/z: 385.16 [M+H].

### Example 4: 3-(3-(1H-indol-6-yl)ureido)-3-(4-benzyl-3,4-dihydro-2H-benzo[b][1,4]thiazin-6-yl)-N,N-dimethylpropanamide

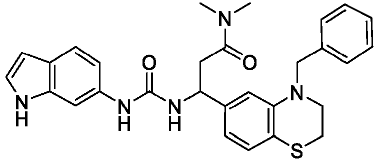

Example 4 was prepared according to the methods described in General Procedures 1-2, 6, 9, 15-18 and the methods described below.

### Preparation 7: 3-(4-Benzyl-3,4-dihydro-2H-benzo[b][1,4]thiazin-6-yl)-3-((tert-butylsulfinyl)amino)propanoic acid

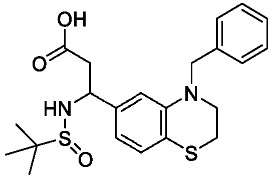

### Step 1: 4-Benzyl-3,4-dihydro-2H-benzo[b][1,4]thiazine-6-carbaldehyde

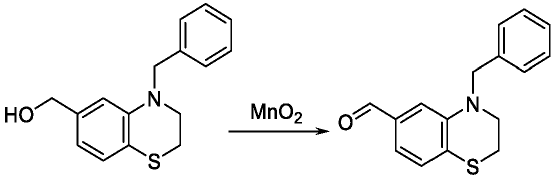

To a stirred solution of (4-benzyl-2,3-dihydro-1,4-benzothiazin-6-yl)methanol (Preparation 1, Step 4) (1.3 g, 4.79 mmol) in DCM (30.0 mL) was added MnO₂ (4.16 g, 47.9 mmol) and the reaction mixture was stirred at room temperature for 16 h. Completion of the reaction was confirmed by LCMS and then the reaction mixture was filtered through a celite bed and concentrated under reduced pressure to give crude product. The crude material was purified by silica gel column chromatography (5-10% EtOAc-hexane) to afford the title compound (700 mg, 54.3% yield) as a yellow solid.

### Step 2: N-((4-Benzyl-3,4-dihydro-2H-benzo[b][1,4]thiazin-6-yl)methylene)-2-methylpropane-2-sulfinamide

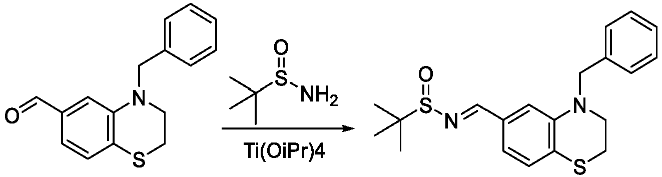

To a stirred solution of 4-benzyl-2,3-dihydro-1,4-benzothiazine-6-carbaldehyde (Preparation 7, Step 1) (1.0 g, 3.71 mmol) in THF (30.0 mL) was added 2-methylpropane-2-sulfinamide (675 mg, 5.57 mmol) at room temperature. Then titanium isopropoxide (2.25 mL, 7.42 mmol) was added dropwise to the solution at the same temperature and the reaction mixture was stirred for 16 h. Completion of the reaction was confirmed by LCMS and then the reaction mixture was quenched with saturated NaHCO₃ solution and extracted with EtOAc (3 x 50 mL). The combined organic layer was washed with brine solution (1 x 30 mL), dried over anhydrous Na₂SO₄ and evaporated under reduced pressure to give crude. The crude material was purified by silica gel column chromatography (10-15% EtOAc-hexane) to afford the title compound (800 mg, 57.9% yield) as a yellow solid. LCMS m/z: 372.2 [M+H].

### Step 3: Ethyl 3-(4-benzyl-3,4-dihydro-2H-benzo[b][1,4]thiazin-6-yl)-3-((tert-butylsulfinyl)amino)propanoate

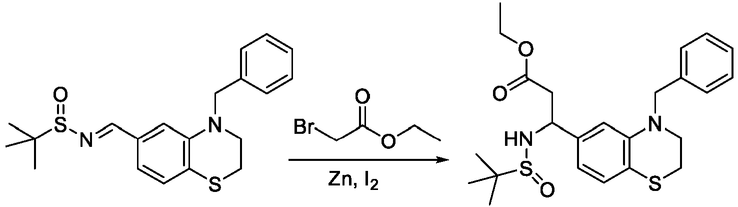

To a stirred suspension of Zn (2.11 g, 32.3 mmol) in THF (30.0 mL) was added iodine (82 mg, 0.32 mmol) at room temperature and the whole was heated at reflux for 30 min. Then, a mixture of N-[(4-benzyl-2,3-dihydro-1,4-benzothiazin-6-yl)methylene]-2-methyl-propane-2-sulfinamide (Preparation 7, Step 2) (1.2 g, 3.23 mmol) and ethyl bromoacetate (1.43 mL, 12.9 mmol) in THF (20 mL) was added into the reaction mixture. The resulting reaction mixture was refluxed for 2 h. Progress of the reaction was monitored by LCMS and after completion, the reaction was filtered through a celite bed. The filtrate was quenched with water (50 mL) and the organic part was extracted with EtOAc (3 x 50 mL). The combined organic layer was washed with brine (1 x 50 mL), dried over anhydrous Na₂SO₄ and concentrated under reduced pressure to give crude product. The crude was purified by silica gel column chromatography (40-60% EtOAc-hexane) to afford the title compound (900 mg, 64.6% yield) as an off-white solid. LCMS m/z: 460.9 [M+H].

### Step 4: 3-(4-Benzyl-3,4-dihydro-2H-benzo[b][1,4]thiazin-6-yl)-3-((tert-butylsulfinyl)amino)propanoic acid

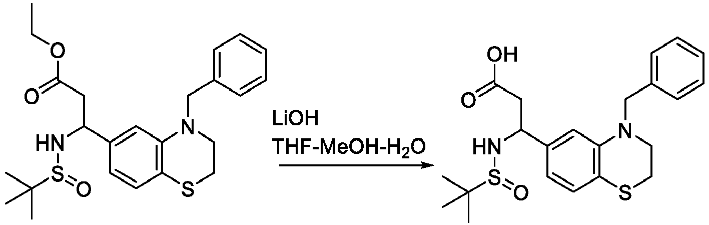

To a stirred solution of ethyl 3-(4-benzyl-2,3-dihydro-1,4-benzothiazin-6-yl)-3-(tert-butylsulfinylamino)propanoate (Preparation 7, Step 3) (1.0 g, 2.17 mmol) in THF:MeOH:H₂O (20 mL, 2:1:1) was added LiOH (104 mg, 4.35 mmol) at room temperature and the reaction mixture was stirred at the same temperature for 2 h. Progress of the reaction was monitored by LCMS and after completion, the solvents were evaporated to give a residue which was diluted with water and acidified with 1N HCl solution to pH 3-5. The product was extracted with 10% MeOH-DCM (3 x 50 mL) and the combined organic layers were washed with brine (1 x 30 mL), dried over anhydrous Na₂SO₄ and concentrated under reduced pressure to obtain the crude product. The crude was purified by silica gel column chromatography (3-5% MeOH-DCM) to afford the title compound (700 mg, 74.4% yield) as an off-white solid. LCMS m/z: 433.2 [M+H].

### Preparation 8: Step 1: 3-(4-Benzyl-3,4-dihydro-2H-benzo[b][1,4]thiazin-6-yl)-3-((tert-butylsulfinyl)amino)-N,N-dimethylpropanamide

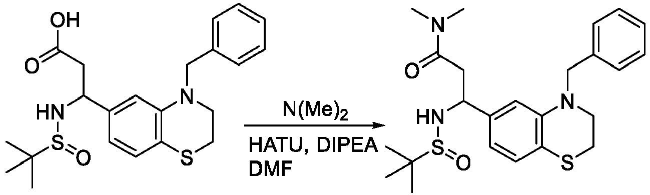

To a stirred solution of 3-(4-benzyl-2,3-dihydro-1,4-benzothiazin-6-yl)-3-(tert-butylsulfinylamino)propanoic acid (Preparation 7, Step 4) (700 mg, 1.62 mmol) in DMF (10 mL) were added HATU (1.23 g, 3.24 mmol), DIPEA (0.85 mL, 4.86 mmol) and dimethyl amine (4.05 mL, 8.1 mmol, 2M in THF) at 0-5 °C and the resulting reaction mixture was stirred at the same temperature for 16 h. Progress of the reaction was monitored by LCMS and after completion, the reaction mixture was quenched with water and extracted with EtOAc (2 x 30 mL). The combined organic layers were washed with ice cold water (5 x 30 mL), brine (1 x 20 mL), dried over anhydrous Na₂SO₄ and concentrated under reduced pressure to obtain the crude product. The crude was purified by silica gel column chromatography (3-5% MeOH-DCM) to afford the title compound (450 mg, 60.4% yield) as an off-white solid. LCMS m/z: 460.4 [M+H].

### Step 2: 3-Amino-3-(4-benzyl-3,4-dihydro-2H-benzo[b][1,4]thiazin-6-yl)-N,N-dimethylpropanamide

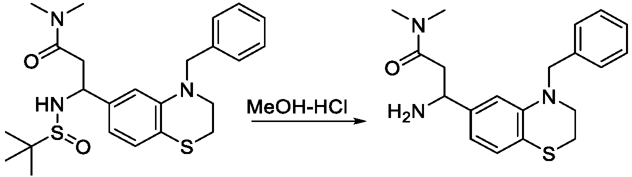

To a stirred solution of 3-(4-benzyl-2,3-dihydro-1,4-benzothiazin-6-yl)-3-(tert-butylsulfinylamino)-N,N-dimethyl-propanamide (Preparation 8, Step 1) (400 mg, 0.87 mmol) in EtOH (10.0 mL) was added 1.25M HCl in MeOH (2.09 mL, 2.61 mmol) at 0-5 °C and the whole was stirred for 1 h. After completion of the reaction (monitored by LCMS), the excess solvent was evaporated under reduced pressure and the reaction mass was quenched with saturated NaHCO₃ solution and extracted with 10% MeOH-DCM acetate (3 x 50 mL). The combined organic layers were washed with brine (1 x 30 mL), dried over anhydrous Na₂SO₄ and concentrated under reduced pressure to afford the title compound (300 mg, crude). The crude was used in the next step without any further purification. LCMS m/z: 356.38 [M+H].

### Step 3: 3-(3-(1H-Indol-6-yl)ureido)-3-(4-benzyl-3,4-dihydro-2H-benzo[b][1,4]thiazin-6-yl)-N,N-dimethylpropanamide (Example 4)

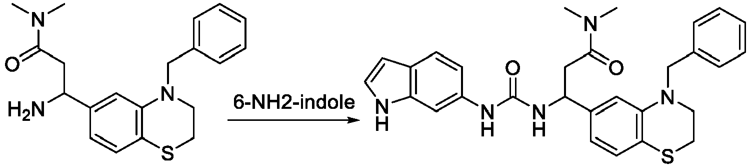

To a stirred solution of 6-amino indole (123 mg, 0.93 mmol) in THF (5 mL) was added p-nitrophenyl chloroformate (255 mg, 1.67 mmol) at 0-5 °C and the whole was stirred at room temperature for 3 h. Then to the reaction mixture was added TEA (0.58 mL, 4.23 mmol) and 3-amino-3-(4-benzyl-2,3-dihydro-1,4-benzothiazin-6-yl)-N,N-dimethyl-propanamide (Preparation 8, Step 2) (300 mg, 0.85 mmol) at the same temperature and the combined mixture was stirred for another 2 h. Progress of the reaction was monitored by LCMS and after completion, the reaction mixture was quenched with water and extracted with EtOAc (3 x 20 mL). Then the combined organic layers were washed with brine solution (1 x 20 mL), dried over anhydrous Na₂SO₄ and concentrated under reduced pressure to obtain the crude product. The crude was purified by silica gel column chromatography (3-5% acetone-DCM) to afford the title compound (150 mg, 34.6% yield) as an off-white solid. Purity by HPLC: 96.47%; ¹H NMR: (400 MHz; DMSO-d₆): δ 2.55-2.60 (m, 2H), 2.71 (s, 3H), 2.78 (s, 3H), 3.04 (t*, J* = 5.12 Hz, 2H), 3.61 (t, *J =* 4.8 Hz, 2H), 4.53 (s, 2H), 4.93-4.95 (m, 1H), 6.27 (bs, 1H), 6.54-6.59 (m, 2H), 6.68 (s, 1H), 6.73-6.75 (dd, *J*1 = 1.28 Hz, *J*2 = 8.32 Hz, 1H), 6.89 (d, *J =* 7.92 Hz, 1H), 7.14 (t, *J =* 2.52 Hz, 1H), 7.19-7.22 (m, 1H), 7.29-7.33 (m, 5H), 7.70 (bs, 1H), 8.45 (s, 1H), 10.81 (s, 1H); LCMS m/z: 514.52 [M+H].

### Chiral separation of Example 4

Racemic compound 3-(3-(1H-Indol-6-yl)ureido)-3-(4-benzyl-3,4-dihydro-2H-benzo[b][1,4]thiazin-6-yl)-N,N-dimethylpropanamide **(Example 4**) (50 mg) was subjected to chiral separation to afford two enantiomers

Enantiomer 1: **Example 5** 8 mg, HPLC purity 96.2%; ¹H NMR: (400 MHz; DMSO-d₆): δ 2.55-2.59 (m, 2H), 2.71 (s, 3H), 2.80 (s, 3H), 3.04 (s, 2H), 3.61 (m, 2H), 4.54 (s, 2H), 4.93 (t, *J =* 5.04 Hz, 1H), 6.27 (bs, 1H), 6.54-6.59 (m, 2H), 6.68 (s, 1H), 6.74 (d, *J =* 5.12 Hz, 1H), 6.89 (d, *J* = 8.08 Hz, 1H), 7.14 (bs, 1H), 7.20-7.22 (m, 1H), 7.29-7.33 (m, 5H), 7.70 (bs, 1H), 8.45 (s, 1H), 10.80 (s, 1H); LCMS m/z: 514.51 [M+H].

Enantiomer 2: **Example 6** 7 mg, HPLC purity 94.36%; ¹H NMR: (400 MHz; DMSO-d₆): δ 2.55-2.59 (m, 2H), 2.71 (s, 3H), 2.76 (s, 3H), 3.05 (d, *J* = 4.96 Hz, 2H), 3.61 (d, *J* = 4.36 Hz, 2H), 4.53 (s, 2H), 4.94 (t, *J* = 7.68 Hz, 1H), 6.27 (bs, 1H), 6.54-6.59 (m, 2H), 6.68 (s, 1H), 6.74 (d, *J* = 4.52 Hz, 1H), 6.89 (d, *J* = 7.96 Hz, 1H), 7.14 (bs, 1H), 7.21-7.22 (m, 1H), 7.29-7.33 (m, 5H), 7.70 (bs, 1H), 8.44 (s, 1H), 10.80 (s, 1H); LCMS m/z: 514.51 [M+H].

### Chiral separation method:

| | |
|---|---|
| Column | Chiralpak IA (250 X 20 mm) 5u; |
| Solubility | MeOH |
| Wave length | 240 nm |
| Mobile Phase | Hexane/EtOH/DCM: 70/15/15 |
| Run time | 20 min; |
| Flow rate | 25 g/min. |

### Examples 7 and 8: 1-(1-(4-Benzyl-3,4-dihydro-2H-benzo[b][1,4]oxazin-6-yl)-3,4-dihydroxybutyl)-3-(1H-indol-6-yl)urea

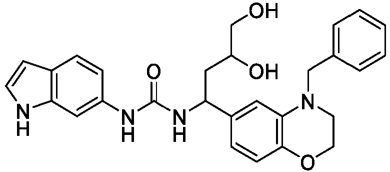

Examples 7 and 8 was prepared according to the methods described in General Procedures 1-2, 9, 15-16, 19 and the methods described below.

### Preparation 9: 1-(1-(4-Benzyl-3,4-dihydro-2H-benzo[b][1,4]oxazin-6-yl)but-3-en-1-yl)-3-(1H-indol-6-yl)urea

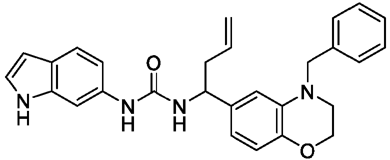

### Step 1: N-(1-(4-Benzyl-3,4-dihydro-2H-benzo[b][1,4]oxazin-6-yl)but-3-en-1-yl)-2-methylpropane-2-sulfinamide

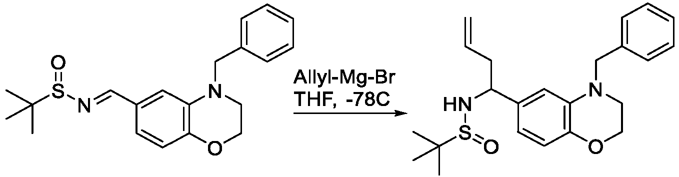

A solution of N-((4-benzyl-3,4-dihydro-2H-benzo[b][1,4]oxazin-6-yl)methylene)-2-methylpropane-2-sulfinamide (prepared according to the methods described for the synthesis of Preparation 7, Step 2) (300 mg, 0.84 mmol) in THF (5 mL) was treated by slow addition of allyl-Mg-Br (2.53 mL, 2.53 mmol, 1M in diethyl ether) at -78 °C. The mixture was stirred at the same temperature for 1 h. After completion of the reaction, the reaction mixture was quenched with a saturated solution of NH₄Cl and extracted with EtOAc (2 x 30 mL). The combined organic layers were washed with brine (1 x 20 mL), dried over anhydrous Na₂SO₄ and concentrated under reduced pressure to obtain the crude product. The crude was purified by silica gel column chromatography (30-50% EtOAc-hexane) to afford the title compound (250 mg, 74.4% yield) as an off-white solid. LCMS m/z: 399.2 [M+H].

### Step 2: 1-(4-Benzyl-3,4-dihydro-2H-benzo[b][1,4]oxazin-6-yl)but-3-en-1-amine

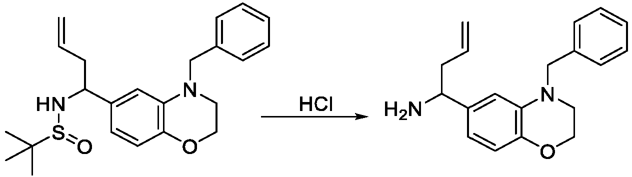

To a stirred solution of N-[1-(4-benzyl-2,3-dihydro-1,4-benzoxazin-6-yl)but-3-enyl]-2-methyl-propane-2-sulfinamide (Preparation 9, Step 1) (250 mg, 0.70 mmol) in EtOH (5.0 mL) was added 1.25M HCl in MeOH (1.68 mL, 2.11 mmol) at 0-5 °C and the whole was stirred for 3 h. After completion of the reaction, the excess solvent was concentrated under reduced pressure and the reaction mixture was quenched with NaHCO₃ solution and extracted with EtOAc (3 x 50 mL). Then the combined organic layers were washed with brine solution (1 x 30 mL), dried over anhydrous Na₂SO₄ and concentrated under reduced pressure to obtain the crude. The crude was purified by silica gel column chromatography (3-5% MeOH-DCM) to afford the title compound (150 mg, 72.6% yield) as an off-white solid.

### Step 3: 1-(1-(4-Benzyl-3,4-dihydro-2H-benzo[b][1,4]oxazin-6-yl)but-3-en-1-yl)-3-(1H-indol-6-yl)urea

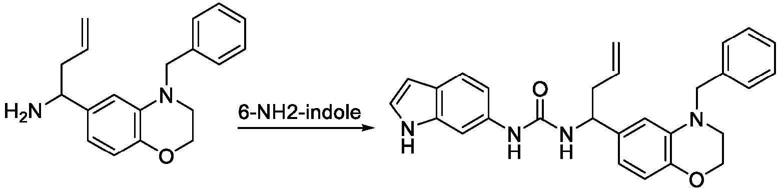

To a stirred solution of 6-amino indole (74.1 mg, 0.56 mmol) in THF (3 mL) was added p-nitrophenyl chloroformate (154 mg, 0.77 mmol) at 0-5 °C and the whole was stirred at room temperature for 3 h. Then to the reaction mixture was added TEA (0.35 mL, 2.55 mmol) and 1-(4-benzyl-2,3-dihydro-1,4-benzoxazin-6-yl)but-3-en-1-amine (Preparation 9, Step 2) (150 mg, 0.51 mmol) at the same temperature and the combined mixture was stirred for another 2 h. The reaction was monitored by LCMS. The reaction mixture was quenched with water and extracted with EtOAc (3 x 20 mL). Then the combined organic layers were washed with brine solution (1 x 20 mL), dried over anhydrous Na₂SO₄ and concentrated under reduced pressure to obtain the crude product. The crude was purified by silica gel column chromatography (3-5% acetone-DCM) to afford the title compound (100 mg, 43.3% yield) as an off-white solid. LCMS m/z: 453.49 [M+H].

### Preparation 10: 1-(1-(4-benzyl-3,4-dihydro-2H-benzo[b][1,4]oxazin-6-yl)-3,4-dihydroxybutyl)-3-(1H-indol-6-yl)urea

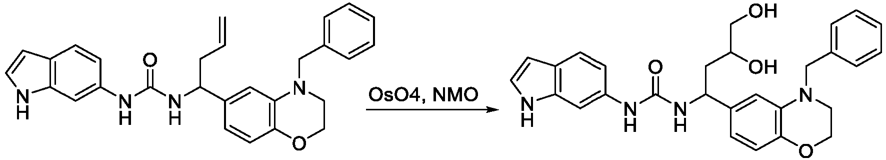

To a stirred solution of 1-[1-(4-benzyl-2,3-dihydro-1,4-benzoxazin-6-yl)but-3-enyl]-3-(1H-indol-6-yl)urea (Preparation 9, Step 3) (100 mg, 0.22 mmol) in acetone:H₂O (3 mL, 9:1) were added N-methylmorpholine N-oxide (51.8 mg, 0.44 mmol) and OsO₄ (5.63 mg, 0.02 mmol) at 0-5 °C and the whole was stirred at the same temperature for 2 h. Progress of the reaction was monitored by LCMS and after completion of the reaction, the excess solvent was evaporated *in vacuo* to give a residue which was diluted with water and extracted with EtOAc (3 x 20 mL). Then the combined organic layers were washed with brine solution (1 x 20 mL), dried over anhydrous Na₂SO₄ and concentrated under reduced pressure to obtain the title compound (50 mg, crude) as a brown solid. LCMS m/z: 487.52 [M+H].

### Chiral separation of 1-(1-(4-benzyl-3,4-dihydro-2H-benzo[b][1,4]oxazin-6-yl)-3,4-dihydroxybutyl)-3-(1H-indol-6-yl)urea (Preparation 10)

Racemic compound 1-(1-(4-benzyl-3,4-dihydro-2H-benzo[b][1,4]oxazin-6-yl)-3,4-dihydroxybutyl)-3-(1H-indol-6-yl)urea (Preparation 10) (50 mg) was subjected to normal phase chiral HPLC separation to afford two enantiomers as light brown solids.

Enantiomer 1: **Example 7**. 4 mg, HPLC purity 98.28%; ¹H NMR: (400 MHz; DMSO-d₆): δ 1.62 (bs, 1H), 1.70 (bs, 1H), 3.22-3.29 (m, 4H), 4.14 (s, 2H), 4.43-4.47 (m, 4H), 4.69 (d, *J =* 6.08 Hz, 1H), 6.22 (s, 1H), 6.36 (d, *J =* 7.72 Hz, 1H), 6.49 (d, *J =* 8.56 Hz, 1H), 6.63 (d, *J =* 8.04 Hz, 1H), 6.71 (d, *J =* 8.52 Hz, 1H), 6.78 (s, 1H), 7.14 (s, 1H), 7.22-7.23 (m, 1H), 7.30-7.33 (m, 5H), 7.69 (s, 1H), 8.15 (s, 1H), 10.80 (s, 1H); LCMS m/z: 487.4 [M+H].

Enantiomer 2: **Example 8.** 5 mg, HPLC purity 98.26%; ¹H NMR: (400 MHz; DMSO-d₆): δ 1.47 (bs, 1H), 1.70-1.73 (bs, 1H), 3.19-3.23 (m, 3H), 3.50 (bs, 1H), 4.15 (s, 2H), 4.43-4.45 (m, 3H), 4.61 (d, *J =* 4.64 Hz, 1H), 4.77 (bs, 1H), 6.28 (s, 1H), 6.40 (d, *J =* 8.36 Hz, 1H), 6.48 (d, *J =* 8.2 Hz, 1H), 6.63 (d, *J =* 8.36 Hz, 1H), 6.72-6.74 (m, 2H), 7.15 (s, 1H), 7.22 (d, J = 6.32 Hz, 1H), 7.27-7.34 (m, 5H), 7.70 (s, 1H), 8.30 (s, 1H), 10.81 (s, 1H); LCMS m/z: 487.4 [M+H].

### Chiral separation method:

| | |
|---|---|
| Column | Chiralpak IA (250 x 20 mm) 5u; |
| Solubility | MeOH |
| Wave length | 240 nm |
| Mobile Phase | Hexane/EtOH/DCM: 70/15/15 |
| Run time | 20 min; |
| Flow rate | 25 g/min. |

### Preparation 11: 1-(1-(4-Benzyl-3,4-dihydro-2H-benzo[b][1,4]thiazin-6-yl)but-3-en-1-yl)-3-(1H-indol-6-yl)urea (Example 9)

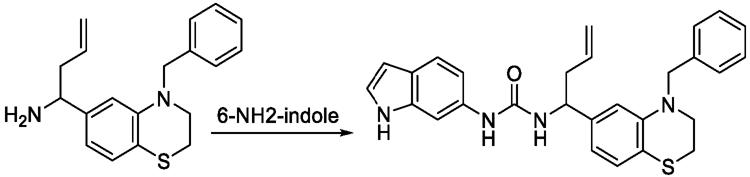

To a stirred solution of 6-amino indole (32.8 mg, 0.25 mmol) in dry THF (3.0 ml) was added TEA (0.09 ml, 0.68 mmol) and 4-nitrophenyl chloroformate (68.2 mg, 0.34 mmol) at 0-5 °C. The resulting reaction mixture was stirred for 30 min. at 0-5 °C and then 1-(4-benzyl-3, 4-dihydro-2H-benzo[b][1,4]thiazin-6-yl)but-3-en-1-amine (prepared according to methods described for the synthesis of Preparation 9, Step 2) (70.0 mg, 0.23 mmol ) added at 0-5 °C. The whole was stirred for 16 h under a nitrogen atmosphere. Progress of the reaction was monitored by TLC and LCMS and after completion of the reaction, the solvents were evaporated under reduced pressure, diluted with water (20 mL) and extracted with EtOAc (2 x 20 mL). The combined organic layers were washed with water (2 x 20 mL) followed by brine (1 x 20 mL), dried over anhydrous Na₂SO₄ and evaporated under reduced pressure to obtain the crude product which was purified by Combi-flash to afford the title compound (13 mg, 12.3% yield) as a brown solid. Purity by HPLC: 94.01%; ¹H NMR: (400 MHz; DMSO-d₆): δ 2.31-2.40 (m, 2H), 2.97 (d, *J* = 2.68 Hz, 2H), 3.63 (s, 2H), 4.55 (bs, 3H), 4.91-4.97 (m, 2H), 5.52-5.57 (m, 1H), 6.28-6.32 (m, 2H), 6.51 (d, *J =* 8.32 Hz, 1H), 6.63 (s, 1H), 6.71 (d, *J* = 8.04 Hz, 1H), 6.91 (d, *J* = 7.72 Hz, 1H), 7.15 (s, 1H), 7.21 (bs, 1H), 7.29-7.34 (m, 5H), 7.68 (s, 1H), 8.22 (s, 1H), 10.80 (s, 1H); LCMS m/z: 469.27 [M+H].

### Example 10: 3-(3-(1H-Indol-6-yl)ureido)-3-(4-benzyl-3,4-dihydro-2H-benzo[b][1,4]thiazin-6-yl)propanoic acid

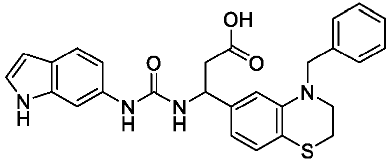

Example 10 was prepared according to the methods described in General Procedures 1-2, 5, 9, 15, 20 and the methods described below.

### Preparation 12; Step 1: Methyl 3-amino-3-(4-benzyl-3,4-dihydro-2H-benzo[b][1,4]thiazin-6-yl)propanoate

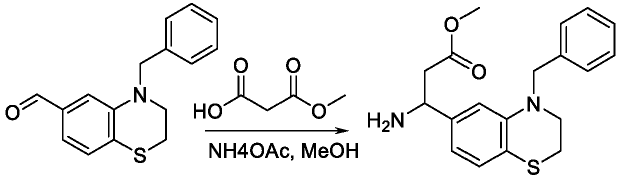

4-Benzyl-2,3-dihydro-1,4-benzothiazine-6-carbaldehyde (Preparation 7, Step 1) (600 mg, 2.23 mmol) was dissolved in MeOH (25 mL). Then NH₄OAc (515.79 mg, 77.08 mmol) and 3-methoxy-3-oxopropanoic acid (0.47 ml, 4.46 mmol) were added to the reaction mixture at RT. The whole was allowed to stir at RT for 2 h. Then another portion of NH40Ac (515.79 mg, 77.08 mmol) was added and the combined mixture was heated at 80 °C for 16 h. Progress of the reaction was monitored by TLC and LCMS and after completion of the reaction the solvent was evaporated to obtain the crude product which was purified by silica gel column chromatography (5% MeOH-DCM) to afford the title compound (100 mg, 13.1% yield) as a yellow solid. LCMS m/z: 343.66 [M+H].

### Step 2: Methyl 3-(3-(1H-indol-6-yl)ureido)-3-(4-benzyl-3,4-dihydro-2H-benzo[b][1,4]thiazin-6-yl)propanoate

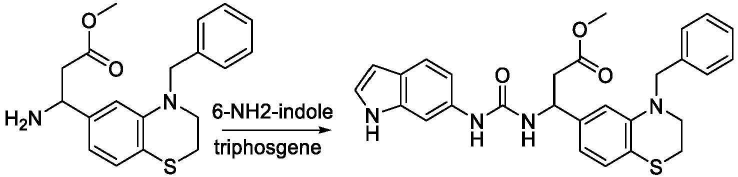

To a reaction mixture of methyl 3-amion-3-(4-benzyl-3,4-dihydro-2H-benzo[b][1,4]thiazin-6-yl)propanoate (Preparation 12, Step 1) (100 mg, 0.29 mmol) and TEA 0.13 ml, 0.87 mmol) was added a solution of triphosgene (87 mg, 0.29 mmol) in DCM (2mL) at 0-5 °C under a nitrogen atmosphere. The resulting mixture was stirred at 0-5 °C for 30 min. then a solution of 6-amino indole (48 mg, 0.292mmol) in DCM (5 mL) was added at 0-5 °C dropwise and the mixture was stirred at room temperature for 16 h. Progress of the reaction was monitored by TLC and LCMS and after completion of the reaction the mixture was quenched with saturated aqueous NaHCO₃ solution and extracted with EtOAc (2 x 20 mL). The combined organic layers were washed with water (2 x 20 mL), brine (2 x 20 mL), dried over anhydrous Na₂SO₄and concentrated *in vacuo* to give the crude product which was purified by silica gel column chromatography (60-70% EtOAc-hexane) to afford the title compound (40 mg, 28% yield) as yellow solid. LCMS m/z: 501.3 [M+H].

### Step 3: 3-(3-(1H-indol-6-yl)ureido)-3-(4-benzyl-3,4-dihydro-2H-benzo[b][1,4]thiazin-6-yl)propanoic acid (Example 10)

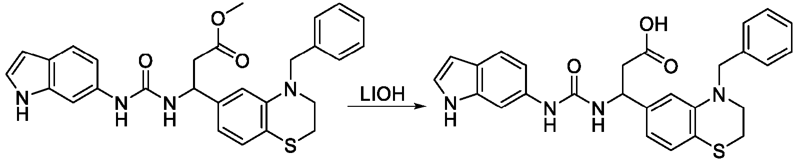

To a stirred solution of methyl 3-(3-(1H-indol-6-yl)ureido)-3-(4-benzyl-3,4-dihydro-2H-benzo[b][1,4]thiazin-6-yl)propanoate (Preparation 12, Step 2) (40 mg, 0.08 mmol) in a mixture of MeOH-THF-H₂O (10 mL, 3:1:1) was added LiOH (4.8 mg, 0.2 mmol) and the resulting solution was stirred at RT for 16 h. After completion of the reaction (monitored by LCMS) the reaction mass was acidified using 3N HCl to pH 5 and extracted with EtOAc (2 x 10 mL). The combined organic layers were washed with brine (2 x 10 mL), dried over anhydrous Na₂SO₄and concentrated under vacuum to give the crude product which was purified by RP HPLC to afford the title compound (6 mg, 15% yield) as an off-white solid. Purity by HPLC: 97.72%; ¹H NMR: (400 MHz; DMSO-d₆): δ 2.55-2.57 (m, 2H), 3.03 (t, *J =* 5.12 Hz, 2H), 3.59 (t, *J =* 4.84 Hz, 2H), 4.53 (s, 2H), 4.88-4.93 (m, 1H), 6.28 (s, 1H), 6.54-6.58 (m, 2H), 6.74 (t, *J =* 8.76 Hz, 2H), 6.63 (s, 1H), 6.91 (d, *J =* 7.88 Hz, 1H), 7.15 (t, *J =* 2.6 Hz, 1H), 7.22-7.34 (m, 5H), 7.69 (s, 1H), 8.35 (s, 1H), 10.81 (s, 1H); LCMS m/z: 487 [M+H].

### Example 11: 1-(1-(4-Benzyl-3,4-dihydro-2H-benzo[b][1,4]thiazin-6-yl)-2-hydroxyethyl)-3-(1H-indol-6-yl)urea

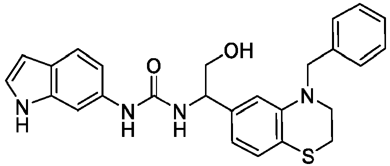

Example 11 was prepared according to the methods described in General Procedures 1-2, 5, 9 and the methods described below.

### Preparation 13: Methyl 2-amino-2-(4-benzyl-3,4-dihydro-2H-benzo[b][1,4]thiazin-6-yl)acetate

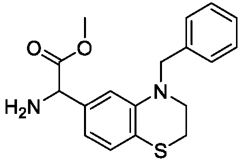

### Step 1: Methyl 2-(4-benzyl-3,4-dihydro-2H-benzo[b][1,4]thiazin-6-yl)-2-((diphenylmethylene)amino)acetate

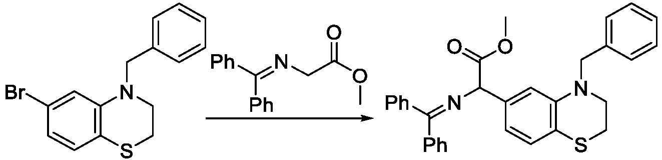

To a stirred solution of 4-benzyl-6-bromo-3,4-dihydro-2H-benzo[b][1,4]thiazine (prepared from commercially available 6-bromo-2H-benzo[b][1,4]thiazin-3(4H)-one by reduction of cyclic amide followed by benzylation as described in General Procedures 2 and 9) (360 mg, 1.12 mmol) in toluene (8 mL) was added methyl 2-((diphenylmethylene)amino)acetate (313 mg, 1.23 mmol), K₃PO₄ (716 mg, 3.34 mmol) and bis(tri-tert-butylphosphine)palladium(o) (5 mg, 0.01 mmol). The resulting reaction mixture was degassed with N₂ gas for 10 min. After this time, the whole reaction mixture was capped and stirred at 100 °C for 20 h. After completion of the reaction (monitored by LCMS) the reaction mixture was quenched with water and extracted with EtOAc (2 x 50 mL). The combined organic layers were washed with brine solution (1 x 50 mL), dried over anhydrous Na₂SO₄ and evaporated in vacuo to afford the title compound (200 mg, crude) as a reddish gummy oil. LCMS m/z: 493.2 [M+H].

### Step 2: Methyl 2-amino-2-(4-benzyl-3,4-dihydro-2H-benzo[b][1,4]thiazin-6-yl)acetate

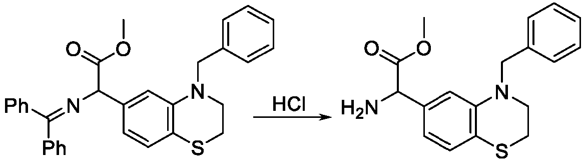

A solution of methyl 2-(4-benzyl-3,4-dihydro-2H-benzo[b][1,4]thiazin-6-yl)-2-((diphenylmethylene)amino)acetate (Preparation 13, Step-1) (840 mg, 1.70 mmol) in 4M HCl in dioxane (4.2 mL, 17.0 mmol) was stirred at room temperature for 4 h. After completion of the reaction, the solvent was evaporated and the crude residue was neutralised with NaHCO₃ solution and extracted with 10% MeOH/DCM (4 x 100 mL). The combined organic layers were washed with brine solution (1 x 50 mL), dried over anhydrous Na₂SO₄ and evaporated in vacuo to afford the title compound (400 mg, 72% yield) as a gummy solid. LCMS m/z: 329.2 [M+H].

### Preparation 14; Step 1: Methyl 2-(3-(1H-indol-6-yl)ureido)-2-(4-benzyl-3,4-dihydro-2H-benzo[b][1,4]thiazin-6-yl)acetate

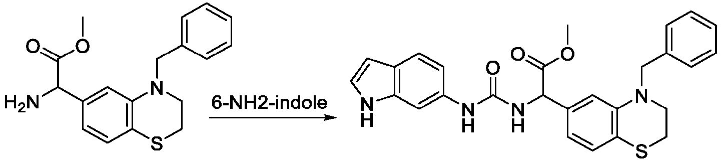

To a stirred solution of 6-amino indole (80.51 mg, 0.60 mmol) in dry THF (6.0 mL) was added TEA (0.255 mL, 1.82 mmol) and 4-nitrophenylchloroformate (184 mg, 0.91 mmol) at 0-5 °C. The whole reaction was stirred at the same temperature for 30 min. TLC confirmed that the starting material had been consumed. Then methyl 2-amino-2-(4-benzyl-3,4-dihydro-2H-benzo[b][1,4]thiazin-6-yl)acetate (Preparation 13, Step 2) (200 mg, 0.60 mmol) was added into the reaction mixture at 0-5 °C and stirring was continued overnight. TLC showed one polar spot, then product formation was confirmed by LCMS. On completion of the reaction the reaction mixture was evaporated to give a residue which was diluted with water (20 mL) and extracted with EtOAC (2 x 20 mL). The combined organic layers were washed with brine (1 x 20 mL), dried over anhydrous Na₂SO₄ and filtered. The filtrate was evaporated under reduced pressure to obtain the crude. The crude was purified by Combi-flash (1-2% acetone-DCM) to afford the title compound (53 mg, 18% yield) as a brown solid. LCMS m/z: 487.2 [M+H].

### Step 2: 1-(1-(4-Benzyl-3,4-dihydro-2H-benzo[b][1,4]thiazin-6-yl)-2-hydroxyethyl)-3-(1H-indol-6-yl)urea (Example 11)

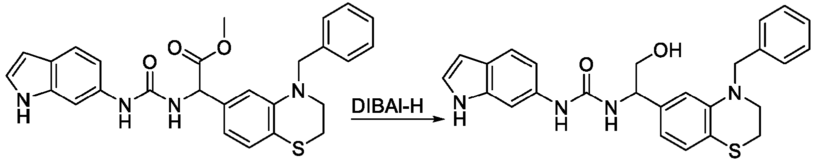

To a stirred solution of methyl 2-(3-(1H-indol-6-yl)ureido)-2-(4-benzyl-3,4-dihydro-2H-benzo[b][1,4]thiazin-6-yl)acetate (Preparation 14, Step 1) (70 mg, 0.14 mmol) in THF (2 mL) was added DIBAl-H (0.29 mL, 0.29 mmol, 1M in toluene) dropwise at 0-5 °C. The reaction mixture was stirred at the same temperature for 2 h. The reaction mixture was then quenched by dropwise addition ofa saturated solution of Rochelle salt at 0-5 °C and the resulting solution was stirred for 1 h at the same temperature. The reaction mass was filtered through a celite bed. The celite bed was washed with EtOAc. The filtrate was extracted with EtOAc (2 x 20 mL). The combined organic layers were washed with brine (1 x 20 mL), dried over anhydrous Na₂SO₄ and concentrated under reduced pressure to obtain the crude product which was purified by reverse phase prep-HPLC to afford the title compound (14 mg, 21% yield) as an off-white solid. Purity by HPLC: 99.34%; ¹H NMR: (400 MHz; DMSO-d₆): δ 3.04-3.06 (m, 2H), 3.40-3.44 (m, 1H), 3.48-3.52 (m, 1H), 3.59-3.61 (m, 2H), 4.51-4.53 (m, 3H), 4.83 (t, *J =* 5.2 Hz, 1H), 6.28 (s, 1H), 6.42 (d, *J =* 7.88 Hz, 1H), 6.53 (d, *J =* 7.96 Hz, 1H), 6.68 (s, 1H), 6.71-6.74 (dd, *J1* = 1.6 Hz, *J2* = 8.48 Hz, 1H), 7.91 (d, *J =* 7.88 Hz, 1H), 7.15 (t, *J =* 2.6 Hz, 1H), 7.1-7.23 (m, 1H), 7.29-7.34 (m, 5H), 7.68 (s, 1H), 8.38 (s, 1H), 10.80 (s, 1H); LCMS m/z: 459.2 [M+H].

### Chiral separation of 1-(1-(4-Benzyl-3,4-dihydro-2H-benzo[b][1,4]thiazin-6-yl)-2-hydroxyethyl)-3-(1H-indol-6-yl)urea (Example 11)

Racemic 1-(1-(4-benzyl-3,4-dihydro-2H-benzo[b][1,4]thiazin-6-yl)-2-hydroxyethyl)-3-(1H-indol-6-yl)urea (Preparation 14, Step 2) (14 mg) was subjected to normal phase chiral HPLC separation to afford two enantiomers.

Enantiomer 1: **Example 12.** 4.22 mg, HPLC purity 92.71%; ¹H NMR: (400 MHz; DMSO-d₆): δ 3.04 (t, *J =* 5.56 Hz, 2H), 3.41-3.42 (m, 1H), 3.48-3.51 (m, 1H), 3.59-3.61 (m, 2H), 4.53 (s, 3H), 4.87 (bs, 1H), 6.28 (d, *J =* 2.56 Hz, 1H), 6.54 (t, *J =* 8.92 Hz, 2H), 6.69 (s, 1H), 6.74 (d, *J =* 8.16 Hz, 1H), 6.90 (d, *J =* 7.84 Hz, 1H), 7.15 (d, *J =* 2.8 Hz, 1H), 7.20-7.21 (m, 1H), 7.27-7.34 (m, 5H), 7.68 (s, 1H), 8.49 (s, 1H), 10.80 (s, 1H); LCMS m/z: 459.15 [M+H].

Enantiomer 2: **Example 13.** 5.12 mg, HPLC purity 91.26%; ¹H NMR: (400 MHz; DMSO-d₆): δ 3.04 (t, *J =* 4.68 Hz, 2H), 3.41-3.42 (m, 1H), 3.48-3.50 (m, 1H), 3.59-3.60 (m, 2H), 4.53 (s, 3H), 4.87 (bs, 1H), 6.28 (bs, 1H), 6.53 (d, *J =* 7.76 Hz, 2H), 6.68 (s, 1H), 6.73 (d, *J =* 8.56 Hz, 1H), 6.91 (d, *J =* 7.84 Hz, 1H), 7.14 (bs, 1H), 7.20-7.21 (m, 1H), 7.29-7.34 (m, 5H), 7.68 (s, 1H), 8.47 (s, 1H), 10.80 (s, 1H); LCMS m/z: 487.4 [M+H].

### Chiral separation method:

| | |
|---|---|
| Column | Chiralpak IA (250 x 21 mm) 5u; |
| Solubility | MeOH |
| Wave length | 240 nm |
| Mobile Phase | Hexane/EtOH/DCM: 50/25/25 |
| Run time | 15 min; |
| Flow rate | 21 mL/min. |

### Example 14: 1-(1H-Indol-6-yl)-3-((4-phenyl-3,4-dihydro-2H-benzo[b][1,4]thiazin-6-yl)methyl)urea

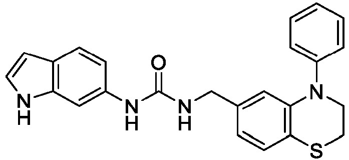

Example 14 was prepared according to the methods described in General Procedures 1-4, 9, 14 and the methods described below.

### Preparation 15: (4-Phenyl-3,4-dihydro-2H-benzo[b][1,4]thiazin-6-yl)methanamine

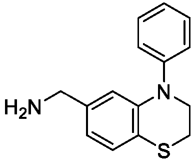

### Step 1: Methyl 3,4-dihydro-2H-benzo[b][1,4]thiazine-6-carboxylate

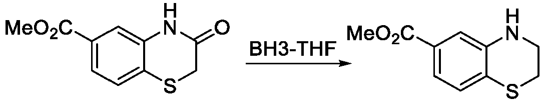

To a stirred solution of methyl 3-oxo-3,4-dihydro-2H-benzo[b][1,4]thiazine-6-carboxylate (Preparation 1, Step 2) (2.5 g, 11 mmol) in dry THF was added borane-THF (22.4 mL, 22.42 mmol, 1M in THF) at 0-5 °C and the resulting solution was allowed to stir at room temperature for 3 h. After completion of the reaction the reaction mixture was quenched with methanol and concentrated under vacuum to obtain the crude compound which was purified by Combi-flash chromatography to afford the title compound (1.8 g, 77% yield) as an off-white solid. LCMS m/z: 210.0 [M+H].

### Step 2: Methyl 4-phenyl-3,4-dihydro-2H-benzo[b][1,4]thiazine-6-carboxylate

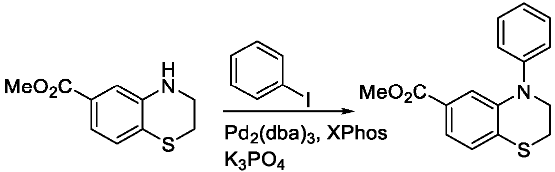

To a degassed solution of methyl 3,4-dihydro-2H-benzo[b][1,4]thiazine-6-carboxylate (Preparation 15, Step 1) (850 mg, 4.06 mmol) in toluene was added iodobenzene (0.907 mL, 8.12 mmol), K₃PO₄ (2.58 g, 12.2 mmol), X-Phos (194 mg, 0.40 mmol) and Pd₂(dba)₃ (372.5 mg, 0.40 mmol). The resulting reaction mixture was heated at 100 °C for 16 h under a nitrogen atmosphere. Progress of the reaction was monitored by LCMS and after completion the reaction mass was filtered through a celite bed and concentrated under reduced pressure to obtain the crude. The crude was purified by Combi-flash chromatography to afford the title compound (500 mg, 47% yield) as a gummy solid. LCMS m/z: 286.24 [M+H].

### Step 3: 4-Phenyl-3,4-dihydro-2H-benzo[b][1,4]thiazine-6-carboxylic acid

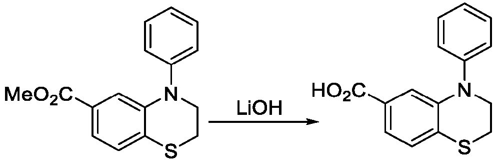

To a stirred solution of methyl 4-phenyl-3,4-dihydro-2H-benzo[b][1,4]thiazine-6-carboxylate (Preparation 15, Step 2) (1.0 g, 3.50 mmol) in a mixture of THF:MeOH:H₂O (50 mL 2:2:1) was added lithium hydroxide monohydrate (737 mg, 17.54 mmol) and the resulting mixture was stirred at room temperature for 16 h. After completion of the reaction the solvents were removed under vacuum and the reaction mass was quenched with 1N aq HCl at 0-5 °C. The neutralised reaction mixture was extracted with 10% MeOH-DCM, washed with brine, dried over anhydrous Na₂SO₄and concentrated under vacuum to afford the title compound (800 mg, 84% yield) as a white solid. LCMS m/z: 270.0 [M+H].

### Step 4: 4-Phenyl-3,4-dihydro-2H-benzo[b][1,4]thiazine-6-carboxamide

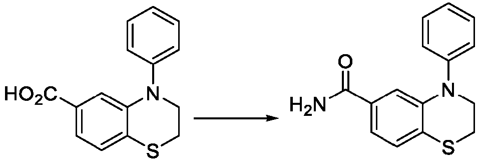

To a stirred solution of 4-phenyl-3,4-dihydro-2H-benzo[b][1,4]thiazine-6-carboxylic acid (Preparation 15, Step 3) (400 mg, 1.47 mmol) in DCM (7 mL) was added oxalylchloride (0.25 ml, 2.95 mmol) at 0-5 °C and the reaction mixture was stirred at the same temperature for 2 h. 0.5M NH₃ in dioxane (8.84 mL) was added dropwise to the reaction mixture at 0-5 °C. The whole reaction mixture was stirred at room temperature overnight. After completion of the reaction the solvents were evaporated and the crude reaction mass was taken up in EtOAc (25 mL). the organic part was washed with water (2 x 15 mL), brine (2 x 15 mL), dried over anhydrous Na₂SO₄ and concentrated under vacuum to afford the title compound (350 mg, 88% yield) as an off-white solid. LCMS m/z: 271.22 [M+H].

### Step 5: (4-Phenyl-3,4-dihydro-2H-benzo[b][1,4]thiazin-6-yl)methanamine

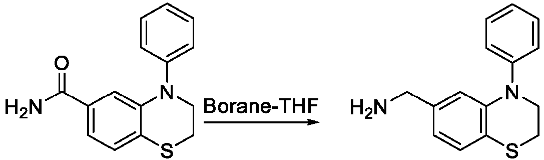

To a stirred solution of 4-phenyl-3,4-dihydro-2H-benzo[b][1,4]thiazine-6-carboxamide (Preparation 15, Step 4) (200 mg, 0.74 mmol) in THF (5 mL) was added borane-THF (1M solution in THF, 4.2 mL) at 0-5 °C and the reaction mixture was heated to reflux for 3 h. the reaction was monitored by LCMS and after completion the reaction mixture was cooled in an ice bath, quenched with methanol and concentrated under vacuum to dryness. The crude product was purified by Combi-flash column chromatography to afford the title compound (100 mg, 52.7% yield) as an off-white solid. GCMS m/z: 256.1.

### Preparation 15: 1-(1H-Indol-6-yl)-3-((4-phenyl-3,4-dihydro-2H-benzo[b][1,4]thiazin-6-yl)methyl)urea (Example 14)

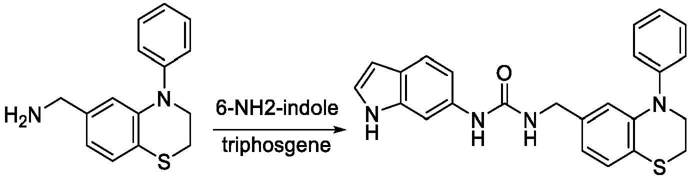

To a stirred solution of (4-phenyl-3,4-dihydro-2H-benzo[b][1,4]thiazin-6-yl)methanamine (Preparation 15, Step 5) (55 mg, 0.21 mmol) in DCM (1 mL) was added TEA (0.09 mL, 0.64 mmol) and a solution of triphosgene (0.63 mg, 0.21 mmol) in DCM (1 mL) at 0-5 °C under a nitrogen atmosphere. The whole was stirred at the same temperature for 1 h then 6-amino indole (22.68 mg, 0.17 mmol) was added to the reaction mixture and the resulting solution was stirred at room temperature for 1 h. After completion (monitored by LCMS), the solvents were removed under vacuum and the crude product was purified by Combi-flash chromatography to afford the title compound (14 mg, 15% yield) as an off-white solid. HPLC purity 99.44%; ¹H NMR: (400 MHz; DMSO-d₆): δ 3.08 (t, *J =* 5.12 Hz, 2H), 3.87-3.87 (m, 2H), 4.07 (d, *J =* 5.8 Hz, 2H), 6.28 (s, 1H), 6.33 (t, *J =* 5.88 Hz, 1H), 6.72-6.77 (m, 3H), 7.01 (t, *J =* 7.32 Hz, 1H), 7.07 (d, *J =* 7.96 Hz, 1H), 7.12 (d, *J =* 7.72 Hz, 2H), 7.16 (t, *J =* 2.52 Hz, 1H), 7.27-7.34 (m, 3H), 7.67 (s, 1H), 8.26 (s, 1H), 10.81 (s, 1H); LCMS m/z: 425.09 [M+H].

### Examples 15-37

The examples in the table below were prepared according to the above methods used to make Examples 1-14 as described in General Procedures 1-20 using the appropriate amines. Purification was as stated in the aforementioned methods

| **Ex** | **Structure** | **IUPAC** | **LCMS [M+H]** | **Purity (%)** |
|---|---|---|---|---|
| | | **Name** | | |
| 1 | 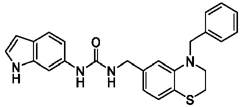 | 1-((4-benzyl-3,4-dihydro-2H-benzo[b][1,4]thiazin-6-yl)methyl)-3-(1H-indol-6-yl)urea | 429.13 | 97.99 |
| 2 | 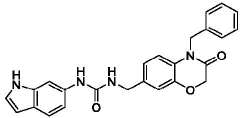 | 1-((4-benzyl-3-oxo-3,4-dihydro-2H-benzo[b][1,4]oxazin-7-yl)methyl)-3-(1H-indol-6-yl)urea | 427.08 | 98.72 |
| 15 | 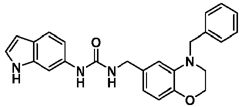 | 1-((4-benzyl-3,4-dihydro-2H-benzo[b][1,4]oxazin-6-yl)methyl)-3-(1H-indol-6-yl)urea | 413.15 | 99.05 |
| 16 | 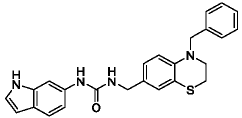 | 1-((4-benzyl-3,4-dihydro-2H-benzo[b][1,4]thiazin-7-yl)methyl)-3-(1H-indol-6-yl)urea | 427.21 | 98.13 |
| 17 | 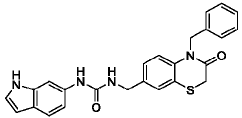 | 1-((4-benzyl-3-oxo-3,4-dihydro-2H-benzo[b][1,4]thiazin-7-yl)methyl)-3-(1H-indol-6-yl)urea | 443.10 | 95.98 |
| 18 | 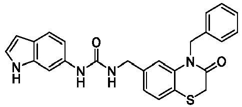 | 1-((4-benzyl-3-oxo-3,4-dihydro-2H-benzo[b][1,4]thiazin-6-yl)methyl)-3-(1H-indol-6-yl)urea | 443.14 | 96.06 |
| 3 | 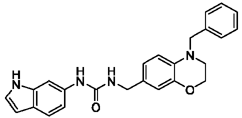 | 1-((4-benzyl-3,4-dihydro-2H-benzo[b][1,4]oxazin-7-yl)methyl)-3-(1H-indol-6-yl)urea | 411.22 [M-H] | 98.49 |
| 19 | 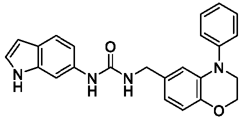 | 1-(1H-indol-6-yl)-3-((4-phenyl-3,4-dihydro-2H-benzo[b][1,4]oxazin-6-yl)methyl)urea | 399.1 | 98.4 |
| 10 | 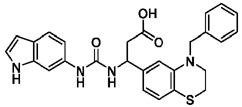 | 3-(3-(1H-indol-6-yl)ureido)-3-(4-benzyl-3,4-dihydro-2H-benzo[b][1,4]thiazin-6-yl)propanoic acid | 487.0 | 97.72 |
| 14 | 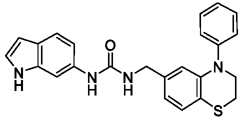 | 1-(1H-indol-6-yl)-3-((4-phenyl-3,4-dihydro-2H-benzo[b][1,4]thiazin-6-yl)methyl)urea | 415.09 | 99.44 |
| 20 | 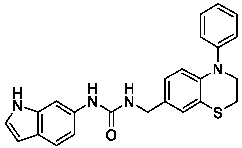 | 1-(1H-indol-6-yl)-3-((4-phenyl-3,4-dihydro-2H-benzo[b][1,4]thiazin-7-yl)methyl)urea | 415.4 | 91.69 |
| 21 | 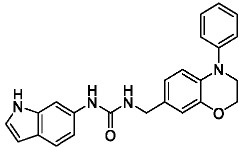 | 1-(1H-indol-6-yl)-3-((4-phenyl-3,4-dihydro-2H-benzo[b][1,4]oxazin-7-yl)methyl)urea | 399.20 | 98.05 |
| 11 | 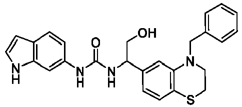 | 1-(1-(4-benzyl-3,4-dihydro-2H-benzo[b][1,4]thiazin-6-yl)-2-hydroxyethyl)-3-(1H-indol-6-yl)urea | 459.21 | 99.34 |
| 9 | 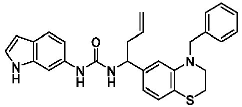 | 1-(1-(4-benzyl-3,4-dihydro-2H-benzo[b][1,4]thiazin-6-yl)but-3-en-1-yl)-3-(1H-indol-6-yl)urea | 469.27 | 94.01 |
| 22 | 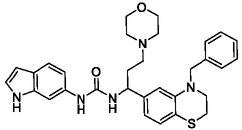 | 1-(1-(4-benzyl-3,4-dihydro-2H-benzo[b][1,4]thiazin-6-yl)-3-morpholinopropyl)-3-(1H-indol-6-yl)urea | 542.28 | 98.08 |
| 23 | 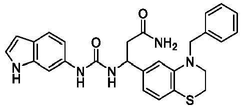 | 3-(3-(1H-indol-6-yl)ureido)-3-(4-benzyl-3,4-dihydro-2H-benzo[b][1,4]thiazin-6-yl)propanamide | 486.25 | 99.21 |
| 24 | 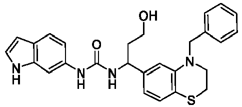 | 1-(1-(4-benzyl-3,4-dihydro-2H-benzo[b][1,4]thiazin-6-yl)-3-hydroxypropyl)-3-(1H-indol-6-yl)urea | 473.25 | 99.52 |
| 4 | 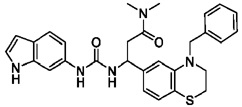 | 3-(3-(1H-indol-6-yl)ureido)-3-(4-benzyl-3,4-dihydro-2H-benzo[b][1,4]thiazin-6-yl)-N,N-dimethylpropanamide | 514.52 | 96.47 |
| 25 | 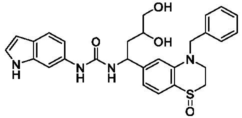 | 1-(1-(4-benzyl-1-oxido-3,4-dihydro-2H-benzo[b][1,4]thiazin-6-yl)-3,4-dihydroxybutyl)-3-(1H-indol-6-yl)urea | 519.28 | 99.88 |
| 26 | 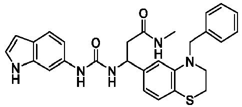 | 3-(3-(1H-indol-6-yl)ureido)-3-(4-benzyl-3,4-dihydro-2H-benzo[b][1,4]thiazin-6-yl)-N-methylpropanamide | 500.4 | 99.0 |
| 5 | 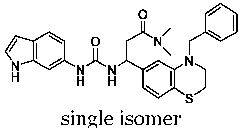 | 3-(3-(1H-indol-6-yl)ureido)-3-(4-benzyl-3,4-dihydro-2H-benzo[b][1,4]thiazin-6-yl)-N,N-dimethylpropanamide | 514.51 | 96.42 |
| 6 | 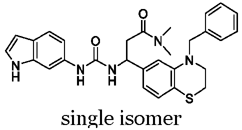 | 3-(3-(1H-indol-6-yl)ureido)-3-(4-benzyl-3,4-dihydro-2H-benzo[b][1,4]thiazin-6-yl)-N,N-dimethylpropanamide | 514.51 | 94.36 |
| 7 | 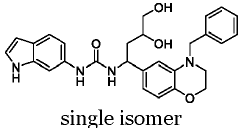 | 1-(1-(4-benzyl-3,4-dihydro-2H-benzo[b][1,4]oxazin-6-yl)-3,4-dihydroxybutyl)-3-(1H-indol-6-yl)urea | 487.4 | 98.28 |
| 8 | 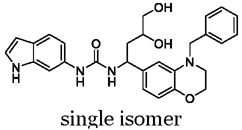 | 1-(1-(4-benzyl-3,4-dihydro-2H-benzo[b][1,4]oxazin-6-yl)-3,4-dihydroxybutyl)-3-(1H-indol-6-yl)urea | 487.4 | 98.26 |
| 12 | 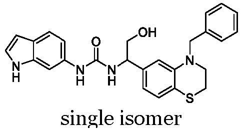 | 1-(1-(4-benzyl-3,4-dihydro-2H-benzo[b][1,4]thiazin-6-yl)-2-hydroxyethyl)-3-(1H-indol-6-yl)urea | 459.15 | 92.71 |
| 13 | 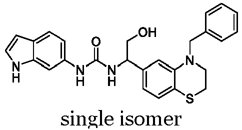 | 1-(1-(4-benzyl-3,4-dihydro-2H-benzo[b][1,4]thiazin-6-yl)-2-hydroxyethyl)-3-(1H-indol-6-yl)urea | 459.15 | 91.26 |
| 27 | 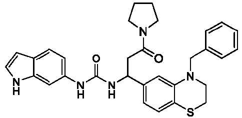 | 1-(1-(4-benzyl-3,4-dihydro-2H-benzo[b][1,4]thiazin-6-yl)-3-oxo-3-(pyrrolidin-1-yl)propyl)-3-(1H-indol-6-yl)urea | 540.4 | 99.64 |
| 28 | 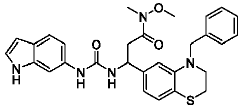 | 3-(3-(1H-indol-6-yl)ureido)-3-(4-benzyl-3,4-dihydro-2H-benzo[b][1,4]thiazin-6-yl)-N-methoxy-N-methylpropanamide | 530.20 | 95.01 |
| 29 | 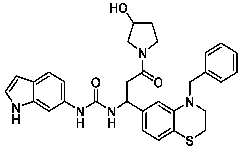 | 1-(1-(4-benzyl-3,4-dihydro-2H-benzo[b][1,4]thiazin-6-yl)-3-(3-hydroxypyrrolidin-1-yl)-3-oxopropyl)-3-(1H-indol-6-yl)urea | 556.4 | 99.27 |
| 30 | 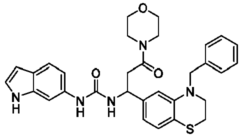 | 1-(1-(4-benzyl-3,4-dihydro-2H-benzo[b][1,4]thiazin-6-yl)-3-morpholino-3-oxopropyl)-3-(1H-indol-6-yl)urea | 556.4 | 99.9 |
| 31 | 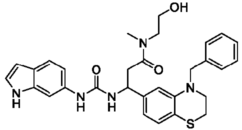 | 3-(3-(1H-indol-6-yl)ureido)-3-(4-benzyl-3,4-dihydro-2H-benzo[b][1,4]thiazin-6-yl)-N-(2-hydroxyethyl)-N-methylpropanamide | 544.4 | 99.55 |
| 32 | 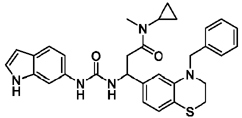 | 3-(3-(1H-indol-6-yl)ureido)-3-(4-benzyl-3,4-dihydro-2H-benzo[b][1,4]thiazin-6-yl)-N-cyclopropyl-N-methylpropanamide | 540.4 | 99.18 |
| 33 | 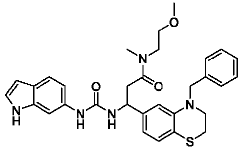 | 3-(3-(1H-indol-6-yl)ureido)-3-(4-benzyl-3,4-dihydro-2H-benzo[b][1,4]thiazin-6-yl)-N-(2-methoxyethyl)-N-methylpropanamide | 558.4 | 98.85 |
| 34 | 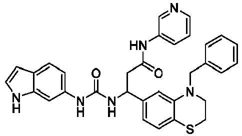 | 3-(3-(1H-indol-6-yl)ureido)-3-(4-benzyl-3,4-dihydro-2H-benzo[b][1,4]thiazin-6-yl)-N-(pyridin-3-yl)propanamide | 563.36 | 98.89 |
| 35 | 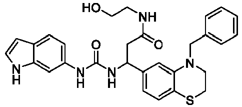 | 3-(3-(1H-indol-6-yl)ureido)-3-(4-benzyl-3,4-dihydro-2H-benzo[b][1,4]thiazin-6-yl)-N-(2-hydroxyethyl)propanamide | 530.37 | 99.47 |
| 36 | 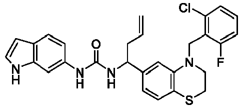 | 1-(1-(4-(2-chloro-6-fluorobenzyl)-3,4-dihydro-2H-benzo[b][1,4]thiazin-6-yl)but-3-en-1-yl)-3-(1H-indol-6-yl)urea | 521.28 | 99.02 |
| 37 | 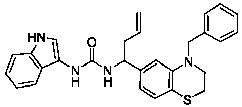 | 1-(1-(4-benzyl-3,4-dihydro-2H-benzo[b][1,4]thiazin-6-yl)but-3-en-1-yl)-3-(1H-indol-3-yl)urea | 469.29 | 99.86 |

### Biological assay

### Reporter gene expression assay in THP-1 cells

THP1-Dual^{™} cells (Invivogen) were derived from the human THP-1 monocyte cell line by stable integration of two inducible reporter constructs. As a result, THP1-Dual^{™} cells allow the simultaneous study of the IRF pathway, by assessing the activity of a secreted luciferase (Lucia) and the NF-κB pathway, by monitoring the activity of secreted SEAP. 5 x 10⁴ THP1-Dual^{™} cells were seeded in 384-well plates in growth medium and preincubated with novel compounds for 10 minutes followed by stimulation with 5 µM 2',3'-cGAMP. After 20hr of stimulation the supernatant was removed and the IRF pathway reporter protein was readily measured in the cell culture supernatant using QUANTI-Luc^{™} (Invivogen), a luciferase detection reagent on a Spectramax i3X luminometer.

In the tables below, IC₅₀ value ranges for exemplary compounds are given. The IC₅₀ ranges are indicated as "A" for values less than or equal to 1 µM, "B" for values greater than 1 µM and less than or equal to 10 µM, and "C" for values greater than 10 µM.

### Activity data

| **Ex.** | **THP-1 (HAQ)** | **Ex.** | **THP-1 (HAQ)** |
|---|---|---|---|
| | **Activity** | | **Activity** |
| 1 | A | 26 | B |
| 2 | B | 5 | B |
| 15 | A | 6 | B |
| 16 | A | 7 | C |
| 17 | A | 8 | C |
| 18 | B | 12 | A |
| 3 | A | 13 | A |
| 19 | A | 27 | B |
| 10 | C | 28 | B |
| 14 | A | 29 | C |
| 20 | B | 30 | B |
| 21 | A | 31 | B |
| 11 | A | 32 | C |
| 9 | A | 33 | B |
| 22 | C | 34 | C |
| 23 | A | 35 | B |
| 24 | C | 36 | A |
| 4 | B | 37 | B |
| 25 | C | | |

## Claims

1. A compound of formula (I): 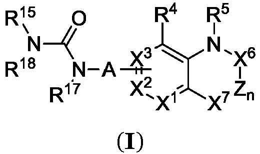
, wherein X¹ is CR¹;
X² is CR₂;
X³ is CR³;
X⁶ is C=O or CR⁷R⁸;
Z is CR⁹R¹⁰;
X⁷ is S, SO or O;
A is an optionally substituted C₁-C₆ alkylene, an optionally substituted C₂-C₆ alkenylene or an optionally substituted C₂-C₆ alkynylene;
n is 1;
R¹, R⁴ and R⁸ are each independently selected from the group consisting of H, halogen, OR¹³, CN, CONR¹³R¹⁴, NR¹³R¹⁴, NR¹³COR¹⁴, optionally substituted C₁-C₆ alkyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl and optionally substituted mono or bicyclic 3 to 8 membered heterocycle;
R⁹ and R¹⁰ are each independently selected from the group consisting of H, halogen, OR¹³, CN, CONR¹³R¹⁴, NR¹³R¹⁴, NR¹³COR¹⁴, optionally substituted C₁-C₆ alkyl, optionally substituted C₂-C₆ alkenyl or optionally substituted C₂-C₆ alkynyl;
one of R² and R³ is -A-NR¹⁷-C(O)-NR¹⁸-R¹⁵ and the other of R² and R³ is selected from the group consisting of H, halogen, OR¹³, CN, COOR₁₃, CONR¹³R¹⁴, NR¹³R¹⁴, NR¹³COR¹⁴, optionally substituted C₁-C₆ alkyl, optionally substituted mono or bicyclic C₃-C₆ cycloalkyl mono or bicyclic optionally substituted C₆-C₁₂ aryl, mono or bicyclic optionally substituted 5 to 10 membered heteroaryl and optionally substituted mono or bicyclic 3 to 8 membered heterocycle;
R⁵ is selected from the group consisting of H, CONR¹³R¹⁴, optionally substituted C₁-C₆ alkyl, optionally substituted C₁-C₆ alkylsulfonyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl, optionally substituted mono or bicyclic 3 to 8 membered heterocycle and L¹-L²-R¹⁶;
R⁷ is selected from the group consisting of H, halogen, CONR¹³R¹⁴, optionally substituted C₁-C₆ alkyl, optionally substituted mono or bicyclic C₃-C₆ cycloalkyl, optionally substituted C₂-C₆ alkenyl, optionally substituted C₂-C₆ alkynyl, mono or bicyclic optionally substituted C₆-C₁₂ aryl, mono or bicyclic optionally substituted 5 to 10 membered heteroaryl, optionally substituted mono or bicyclic 3 to 8 membered heterocycle and L¹-L²-R¹⁶;
wherein a maximum of one of R⁵ and R⁷ is -L¹-L²-R¹⁶;
R¹³ and R¹⁴ are each independently selected from the group consisting of H, halogen, OH, CN, COOH, CONH₂, NH₂, NHCOH, optionally substituted C₁-C₆ alkyl, optionally substituted C₁-C₆ alkylsulfonyl, optionally substituted mono or bicyclic C₃-C₆ cycloalkyl, optionally substituted C₂-C₆ alkenyl, optionally substituted C₂-C₆ alkynyl, optionally substituted C₁-C₆ alkoxy, optionally substituted C₁-C₆ alkoxycarbonyl group, mono or bicyclic optionally substituted C₆-C₁₂ aryl, mono or bicyclic optionally substituted 5 to 10 membered heteroaryl, optionally substituted mono or bicyclic 3 to 8 membered heterocycle, optionally substituted aryloxy, optionally substituted heteroaryloxy and optionally substituted heterocyclyloxy;
L¹ is absent or an optionally substituted C₁-C₆ alkylene, an optionally substituted C₂-C₆ alkenylene, an optionally substituted C₂-C₆ alkynylene, O, S, S=O, SO₂ or NR¹⁹;
L² is absent or an optionally substituted C₁-C₆ alkylene, an optionally substituted C₂-C₆ alkenylene, an optionally substituted C₂-C₆ alkynylene, O, S, S=O, SO₂ or NR¹⁹;
R¹⁵ is optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle;
R¹⁶ is optionally substituted C₁-C₆ alkyl, optionally substituted C₂-C₆ alkenyl, optionally substituted C₂-C₆ alkynyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle; and
R¹⁷ to R¹⁹ are independently H, an optionally substituted C₁-C₆ alkyl or an optionally substituted C₂-C₆ alkenyl;
or a pharmaceutically acceptable complex, salt, solvate, tautomeric form or polymorphic form thereof; and
wherein the compound is not

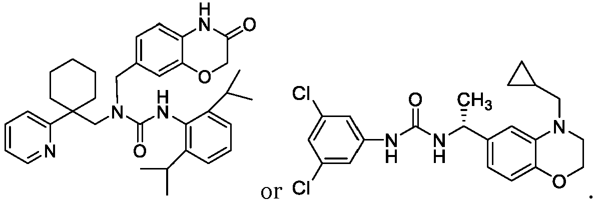

2. The compound according to claim 1, wherein R¹ is H, halogen, OH, CN or optionally substituted C₁-C₆ alkyl.

3. The compound according to any preceding claim, wherein one of R² and R³ is -A-NR¹⁷-C(O)-NR¹⁸-R¹⁵ and the other of R² and R³ is H, halogen, OH, CN, COOR₁₃, CONR¹³R¹⁴, NR¹³R¹⁴, NR¹³COR¹⁴ or optionally substituted C₁-C₆ alkyl, and R¹³ and R¹⁴ are each independently selected from the group consisting of H, optionally substituted C₁-C₃ alkyl, optionally substituted C₂-C₃ alkenyl and optionally substituted C₂-C₃ alkynyl; and preferably wherein one of R² and R³ is -A-NR¹⁷-C(O)-NR¹⁸-R¹⁵ and the other of R² and R³ is H, halogen, OH, CN, CONR¹³R¹⁴, NR¹³R¹⁴ or C₁-C₃ alkyl, and R¹³ and R¹⁴ are each independently selected from the group consisting of H, C₁-C₃ alkyl, C₂-C₃ alkenyl and C₂-C₃ alkynyl.

4. The compound according to any preceding claim, wherein A is an optionally substituted C₁-C₆ alkylene, an optionally substituted C₂-C₆ alkenylene or an optionally substituted C₂-C₆ alkynylene, wherein the alkylene, alkenylene or alkynylene is unsubstituted or substituted with one or more of halogen, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optionally substituted C₆-C₁₂ aryl, optionally substituted 5 to 10 membered heteroaryl, optionally substituted C₃-C₆ cycloalkyl or optionally substituted 3 to 8 membered heterocycle; and preferably wherein A is -CH₂-, 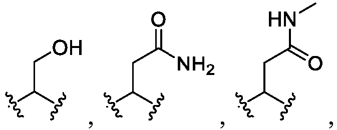 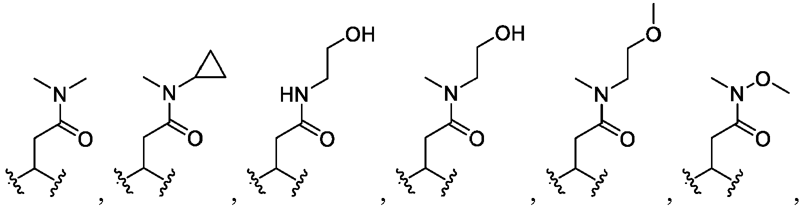 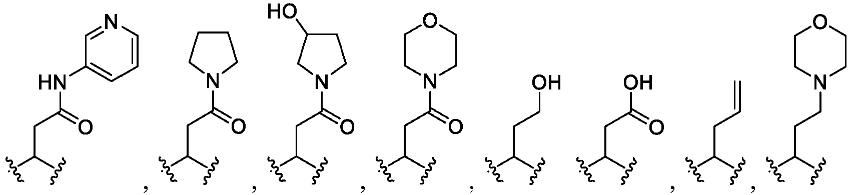 or

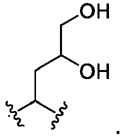

5. The compound according to any preceding claim, wherein R⁴ is H, halogen, OH, CN or optionally substituted C₁-C₆ alkyl.

6. The compound according to any preceding claim, wherein R⁵ is -L¹-L²-R¹⁶, and optionally wherein:
- L¹ is absent, an optionally substituted C₁-C₃ alkylene, an optionally substituted C₂-C₃ alkenylene or an optionally substituted C₂-C₃ alkynylene;
- L² is absent or O, S, S=O, SO₂ or NR¹⁹; and/or
- R¹⁶ is optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle.

7. The compound according to any one of claims 1 to 5, wherein R⁵ is optionally substituted C₁-C₆ alkyl.

8. The compound according to any preceding claim, wherein X⁶ is CR⁷R⁸ and R⁷ and R⁸ are independently H, halogen, OH, CONR¹³R¹⁴ or optionally substituted C₁-C₆ alkyl.

9. The compound according to any one of claims 1 to 7, wherein X⁶ is CO.

10. The compound according to any preceding claim, wherein:
- X⁷ is S, SO or O;
- R⁹ and R¹⁰ are independently H, halogen, OR¹³, CN, CONR¹³R¹⁴, NR¹³R¹⁴, NR¹³COR¹⁴, optionally substituted C₁-C₃ alkyl, optionally substituted C₂-C₃ alkenyl or optionally substituted C₂-C₃ alkynyl; and
- R¹³ and R¹⁴ are independently H, optionally substituted C₁-C₃ alkyl, optionally substituted C₂-C₃ alkenyl or optionally substituted C₂-C₃ alkynyl.

11. The compound according to claim 1, wherein the compound is:
1-((4-Benzyl-3,4-dihydro-2H-benzo[b][1,4]thiazin-6-yl)methyl)-3-(1H-indol-6-yl)urea;
1-((4-Benzyl-3-oxo-3,4-dihydro-2H-benzo[b][1,4]oxazin-7-yl)methyl)-3-(1H-indol-6-yl)urea;
1-((4-Benzyl-3,4-dihydro-2H-benzo[b][1,4]oxazin-7-yl)methyl)-3-(1H-indol-6-yl)urea;
3-(3-(1H-indol-6-yl)ureido)-3-(4-benzyl-3,4-dihydro-2H-benzo[b][1,4]thiazin-6-yl)-N,N-dimethylpropanamide;
(S)-3-(3-(1H-indol-6-yl)ureido)-3-(4-benzyl-3,4-dihydro-2H-benzo[b][1,4]thiazin-6-yl)-N,N-dimethylpropanamide;
(R)-3-(3-(1H-indol-6-yl)ureido)-3-(4-benzyl-3,4-dihydro-2H-benzo[b][1,4]thiazin-6-yl)-N,N-dimethylpropanamide;
1-(1-(4-Benzyl-3,4-dihydro-2H-benzo[b][1,4]oxazin-6-yl)-3,4-dihydroxybutyl)-3-(1H-indol-6-yl)urea;
1-(1-(4-Benzyl-3,4-dihydro-2H-benzo[b][1,4]thiazin-6-yl)but-3-en-1-yl)-3-(1H-indol-6-yl)urea;
3-(3-(1H-Indol-6-yl)ureido)-3-(4-benzyl-3,4-dihydro-2H-benzo[b][1,4]thiazin-6-yl)propanoic acid;
1-(1-(4-Benzyl-3,4-dihydro-2H-benzo[b][1,4]thiazin-6-yl)-2-hydroxyethyl)-3-(1H-indol-6-yl)urea;
(R)-1-(1-(4-benzyl-3,4-dihydro-2H-benzo[b][1,4]thiazin-6-yl)-2-hydroxyethyl)-3-(1H-indol-6-yl)urea;
(S)-1-(1-(4-benzyl-3,4-dihydro-2H-benzo[b][1,4]thiazin-6-yl)-2-hydroxyethyl)-3-(1H-indol-6-yl)urea;
1-(1H-Indol-6-yl)-3-((4-phenyl-3,4-dihydro-2H-benzo[b][1,4]thiazin-6-yl)methyl)urea;
1-((4-benzyl-3,4-dihydro-2H-benzo[b][1,4]oxazin-6-yl)methyl)-3-(1H-indol-6-yl)urea;
1-((4-benzyl-3,4-dihydro-2H-benzo[b][1,4]thiazin-7-yl)methyl)-3-(1H-indol-6-yl)urea;
1-((4-benzyl-3-oxo-3,4-dihydro-2H-benzo[b][1,4]thiazin-7-yl)methyl)-3-(1H-indol-6-yl)urea;
1-((4-benzyl-3-oxo-3,4-dihydro-2H-benzo[b][1,4]thiazin-6-yl)methyl)-3-(1H-indol-6-yl)urea;
1-(1H-indol-6-yl)-3-((4-phenyl-3,4-dihydro-2H-benzo[b][1,4]oxazin-6-yl)methyl)urea;
1-(1H-indol-6-yl)-3-((4-phenyl-3,4-dihydro-2H-benzo[b][1,4]thiazin-7-yl)methyl)urea;
1-(1H-indol-6-yl)-3-((4-phenyl-3,4-dihydro-2H-benzo[b][1,4]oxazin-7-yl)methyl)urea;
1-(1-(4-benzyl-3,4-dihydro-2H-benzo[b][1,4]thiazin-6-yl)-3-morpholinopropyl)-3-(1H-indol-6-yl)urea;
3-(3-(1H-indol-6-yl)ureido)-3-(4-benzyl-3,4-dihydro-2H-benzo[b][1,4]thiazin-6-yl)propanamide;
1-(1-(4-benzyl-3,4-dihydro-2H-benzo[b][1,4]thiazin-6-yl)-3-hydroxypropyl)-3-(1H-indol-6-yl)urea;
1-(1-(4-benzyl-1-oxido-3,4-dihydro-2H-benzo[b][1,4]thiazin-6-yl)-3,4-dihydroxybutyl)-3-(1H-indol-6-yl)urea;
3-(3-(1H-indol-6-yl)ureido)-3-(4-benzyl-3,4-dihydro-2H-benzo[b][1,4]thiazin-6-yl)-N-methylpropanamide;
1-(1-(4-benzyl-3,4-dihydro-2H-benzo[b][1,4]thiazin-6-yl)-3-oxo-3-(pyrrolidin-1-yl)propyl)-3-(1H-indol-6-yl)urea;
3-(3-(1H-indol-6-yl)ureido)-3-(4-benzyl-3,4-dihydro-2H-benzo[b][1,4]thiazin-6-yl)-N-methoxy-N-methylpropanamide;
1-(1-(4-benzyl-3,4-dihydro-2H-benzo[b][1,4]thiazin-6-yl)-3-(3-hydroxypyrrolidin-1-yl)-3-oxopropyl)-3-(1H-indol-6-yl)urea;
1-(1-(4-benzyl-3,4-dihydro-2H-benzo[b][1,4]thiazin-6-yl)-3-morpholino-3-oxopropyl)-3-(1H-indol-6-yl)urea;
3-(3-(1H-indol-6-yl)ureido)-3-(4-benzyl-3,4-dihydro-2H-benzo[b][1,4]thiazin-6-yl)-N-(2-hydroxyethyl)-N-methylpropanamide;
3-(3-(1H-indol-6-yl)ureido)-3-(4-benzyl-3,4-dihydro-2H-benzo[b][1,4]thiazin-6-yl)-N-cyclopropyl-N-methylpropanamide;
3-(3-(1H-indol-6-yl)ureido)-3-(4-benzyl-3,4-dihydro-2H-benzo[b][1,4]thiazin-6-yl)-N-(2-methoxyethyl)-N-methylpropanamide;
3-(3-(1H-indol-6-yl)ureido)-3-(4-benzyl-3,4-dihydro-2H-benzo[b][1,4]thiazin-6-yl)-N-(pyridin-3-yl)propanamide;
3-(3-(1H-indol-6-yl)ureido)-3-(4-benzyl-3,4-dihydro-2H-benzo[b][1,4]thiazin-6-yl)-N-(2-hydroxyethyl)propanamide;
1-(1-(4-(2-chloro-6-fluorobenzyl)-3,4-dihydro-2H-benzo[b][1,4]thiazin-6-yl)but-3-en-1-yl)-3-(1H-indol-6-yl)urea; or
1-(1-(4-benzyl-3,4-dihydro-2H-benzo[b][1,4]thiazin-6-yl)but-3-en-1-yl)-3-(1H-indol-3-yl)urea.

12. A pharmaceutical composition comprising a compound according to any preceding claim, or a pharmaceutically acceptable salt, solvate, tautomeric form or polymorphic form thereof, and a pharmaceutically acceptable vehicle.

13. A compound, as defined in any one of claims 1 to 11, or a pharmaceutically acceptable complex, salt, solvate, tautomeric form or polymorphic form thereof, of a pharmaceutical composition, as defined by claim 12, for use as a medicament.

14. A compound of formula (**I**): 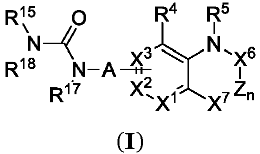
, wherein X¹ is CR¹;
X² is CR² and X³ is CR³;
X⁶ is C=O or CR⁷R⁸;
Z is CR⁹R¹⁰;
X⁷ is S, SO or O;
A is an optionally substituted C₁-C₆ alkylene, an optionally substituted C₂-C₆ alkenylene or an optionally substituted C₂-C₆ alkynylene;
n is 1;
R¹, R⁴ and R⁸ are each independently selected from the group consisting of H, halogen, OR¹³, CN, CONR¹³R¹⁴, NR¹³R¹⁴, NR¹³COR¹⁴, optionally substituted C₁-C₆ alkyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl and optionally substituted mono or bicyclic 3 to 8 membered heterocycle;
R⁹ and R¹⁰ are each independently selected from the group consisting of H, halogen, OR¹³, CN, CONR¹³R¹⁴, NR¹³R¹⁴, NR¹³COR¹⁴, optionally substituted C₁-C₆ alkyl, optionally substituted C₂-C₆ alkenyl or optionally substituted C₂-C₆ alkynyl;
one of R² and R³ is -A-NR¹⁷-C(O)-NR¹⁸-R¹⁵ and the other of R² and R³ is selected from the group consisting of H, halogen, OR¹³, CN, COOR₁₃, CONR¹³R¹⁴, NR¹³R¹⁴, NR¹³COR¹⁴, optionally substituted C₁-C₆ alkyl, optionally substituted mono or bicyclic C₃-C₆ cycloalkyl mono or bicyclic optionally substituted C₆-C₁₂ aryl, mono or bicyclic optionally substituted 5 to 10 membered heteroaryl and optionally substituted mono or bicyclic 3 to 8 membered heterocycle;
R⁵ is selected from the group consisting of H, CONR¹³R¹⁴, optionally substituted C₁-C₆ alkyl, optionally substituted C₁-C₆ alkylsulfonyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl, optionally substituted mono or bicyclic 3 to 8 membered heterocycle and L¹-L²-R¹⁶;
R⁷ is selected from the group consisting of H, halogen, CONR¹³R¹⁴, optionally substituted C₁-C₆ alkyl, optionally substituted mono or bicyclic C₃-C₆ cycloalkyl, optionally substituted C₂-C₆ alkenyl, optionally substituted C₂-C₆ alkynyl, mono or bicyclic optionally substituted C₆-C₁₂ aryl, mono or bicyclic optionally substituted 5 to 10 membered heteroaryl, optionally substituted mono or bicyclic 3 to 8 membered heterocycle and L¹-L²-R¹⁶;
wherein a maximum of one of R⁵ and R7 is -L¹-L²-R¹⁶;
R¹³ and R¹⁴ are each independently selected from the group consisting of H, halogen, OH, CN, COOH, CONH₂, NH₂, NHCOH, optionally substituted C₁-C₆ alkyl, optionally substituted C₁-C₆ alkylsulfonyl, optionally substituted mono or bicyclic C₃-C₆ cycloalkyl, optionally substituted C₂-C₆ alkenyl, optionally substituted C₂-C₆ alkynyl, optionally substituted C₁-C₆ alkoxy, optionally substituted C₁-C₆ alkoxycarbonyl group, mono or bicyclic optionally substituted C₆-C₁₂ aryl, mono or bicyclic optionally substituted 5 to 10 membered heteroaryl, optionally substituted mono or bicyclic 3 to 8 membered heterocycle, optionally substituted aryloxy, optionally substituted heteroaryloxy and optionally substituted heterocyclyloxy;
L¹ is absent or an optionally substituted C₁-C₆ alkylene, an optionally substituted C₂-C₆ alkenylene, an optionally substituted C₂-C₆ alkynylene, O, S, S=O, SO₂ or NR¹⁹;
L² is absent or an optionally substituted C₁-C₆ alkylene, an optionally substituted C₂-C₆ alkenylene, an optionally substituted C₂-C₆ alkynylene, O, S, S=O, SO₂ or NR¹⁹;
R¹⁵ is optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle;
R¹⁶ is optionally substituted C₁-C₆ alkyl, optionally substituted C₂-C₆ alkenyl, optionally substituted C₂-C₆ alkynyl, optionally substituted mono or bicyclic C₃-C₆ cycloalkyl, mono or bicyclic optionally substituted C₆-C₁₂ aryl, mono or bicyclic optionally substituted 5 to 10 membered heteroaryl or optionally substituted mono or bicyclic 3 to 8 membered heterocycle; and
R¹⁷ to R¹⁹ are independently H, an optionally substituted C₁-C₆ alkyl or an optionally substituted C₂-C₆ alkenyl;
or a pharmaceutically acceptable complex, salt, solvate, tautomeric form or polymorphic form thereof,
or a pharmaceutical composition comprising a compound of formula (I), or a pharmaceutically acceptable complex, salt, solvate, tautomeric form or polymorphic form thereof, and a pharmaceutically acceptable vehicle,
for use in treating, ameliorating or preventing a disease selected from liver fibrosis, fatty liver disease, non-alcoholic steatohepatitis (NASH), pulmonary fibrosis, lupus, sepsis, rheumatoid arthritis (RA), type I diabetes, STING-associated vasculopathy with onset in infancy (SAVI), Aicardi-Goutieres syndrome (AGS), familial chilblain lupus (FCL), systemic lupus erythematosus (SLE), retinal vasculopathy, neuroinflammation, systemic inflammatory response syndrome, pancreatitis, cardiovascular disease, renal fibrosis, stroke and age-related macular degeneration (AMD)

15. The compound or composition for use according to claim 14, wherein the disease is fibrosis, and the fibrosis is selected from the group consisting of liver fibrosis, pulmonary fibrosis or renal fibrosis; or wherein the disease is fatty liver disease, and the fatty liver disease is non-alcoholic (or simple) fatty liver or non-alcoholic steatohepatitis (NASH).

## Patentansprüche

1. Verbindung der Formel (I): 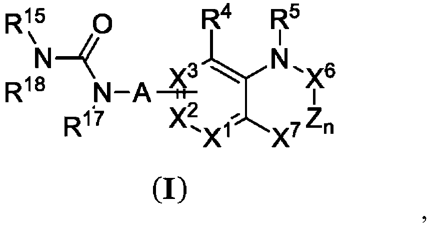
wobei X¹ CR¹ ist;
X² CR² ist;
X³ CR³ ist;
X⁶ C=O oder CR⁷R⁸ ist;
Z CR⁹R¹⁰ ist;
X⁷ S, SO oder O ist;
A ein optional substituiertes C₁-C₆-Alkylen, ein optional substituiertes C₂-C₆-Alkenylen oder ein optional substituiertes C₂-C₆-Alkinylen ist;
n 1 ist;
R¹, R⁴ und R⁸ jeweils unabhängig ausgewählt sind aus der Gruppe bestehend aus H, Halogen, OR¹³, CN, CONR¹³R¹⁴, NR¹³R¹⁴, NR¹³COR¹⁴, optional substituiertem C₁-C₆-Alkyl, optional substituiertem mono- oder bicyclischen C₃-C₆-Cycloalkyl, mono- oder bicyclischem optional substituierten C₆-C₁₂-Aryl, mono- oder bicyclischem optional substituierten 5- bis 10-gliedrigen Heteroaryl und optional substituiertem mono- oder bicyclischen 3- bis 8-gliedrigen Heterocyclus;
R⁹ und R¹⁰ jeweils unabhängig ausgewählt sind aus der Gruppe bestehend aus H, Halogen, OR¹³, CN, CONR¹³R¹⁴, NR¹³R¹⁴, NR¹³COR¹⁴, optional substituiertem C₁-C₆-Alkyl, optional substituiertem C₂-C₆-Alkenyl oder optional substituiertem C₂-C₆-Alkinyl;
eines von R² und R³ -A-NR¹⁷-C(O)-NR¹⁸-R¹⁵ ist und das andere von R² und R³ ausgewählt ist aus der Gruppe bestehend aus H, Halogen, OR¹³, CN, COOR¹³, CONR¹³R¹⁴, NR¹³R¹⁴, NR¹³COR¹⁴, optional substituiertem C₁-C₆-Alkyl, optional substituiertem mono- oder bicyclischen C₃-C₆-Cycloalkyl, mono- oder bicyclischem optional substituierten C₆-C₁₂-Aryl, mono- oder bicyclischem optional substituierten 5- bis 10-gliedrigen Heteroaryl und optional substituiertem mono- oder bicyclischen 3- bis 8-gliedrigen Heterocyclus;
R⁵ ausgewählt ist aus der Gruppe bestehend aus H, CONR¹³R¹⁴, optional substituiertem C₁-C₆-Alkyl, optional substituiertem C₁-C₆-Alkylsulfonyl, optional substituiertem mono- oder bicyclischen C₃-C₆-Cycloalkyl, mono- oder bicyclischem optional substituierten C₆-C₁₂-Aryl, mono- oder bicyclischem optional substituierten 5- bis 10-gliedrigen Heteroaryl, optional substituiertem mono- oder bicyclischen 3- bis 8-gliedrigen Heterocyclus und L¹-L²-R¹⁶;
R⁷ ausgewählt ist aus der Gruppe bestehend aus H, Halogen, CONR¹³R¹⁴, optional substituiertem C₁-C₆-Alkyl, optional substituiertem mono- oder bicyclischen C₃-C₆-Cycloalkyl, optional substituiertem C₂-C₆-Alkenyl, optional substituiertem C₂-C₆-Alkinyl, mono- oder bicyclischem optional substituierten C₆-C₁₂-Aryl, mono- oder bicyclischem optional substituierten 5- bis 10-gliedrigen Heteroaryl, optional substituiertem mono- oder bicyclischen 3- bis 8-gliedrigen Heterocyclus und L¹-L²-R¹⁶;
wobei ein Maximum von einem von R⁵ und R⁷ -L¹-L²-R¹⁶ ist;
R¹³ und R¹⁴ jeweils unabhängig ausgewählt sind aus der Gruppe bestehend aus H, Halogen, OH, CN, COOH, CONH₂, NH₂, NHCOH, optional substituiertem C₁-C₆-Alkyl, optional substituiertem C₁-C₆-Alkylsulfonyl, optional substituiertem mono- oder bicyclischen C₃-C₆-Cycloalkyl, optional substituiertem C₂-C₆-Alkenyl, optional substituiertem C₂-C₆-Alkinyl, optional substituiertem C₁-C₆-Alkoxy, optional substituierter C₁-C₆-Alkoxycarbonylgruppe, mono- oder bicyclischem optional substituierten C₆-C₁₂-Aryl, mono- oder bicyclischem optional substituierten 5- bis 10-gliedrigen Heteroaryl, optional substituiertem mono- oder bicyclischen 3-bis 8-gliedrigen Heterocyclus, optional substituiertem Aryloxy, optional substituiertem Heteroaryloxy und optional substituiertem Heterocyclyloxy;
L¹ fehlt oder ein optional substituiertes C₁-C₆-Alkylen, ein optional substituiertes C₂-C₆-Alkenylen, ein optional substituiertes C₂-C₆-Alkinylen, O, S, S=O, SO₂ oder NR¹⁹ ist;
L² fehlt oder ein optional substituiertes C₁-C₆-Alkylen, ein optional substituiertes C₂-C₆-Alkenylen, ein optional substituiertes C₂-C₆-Alkinylen, O, S, S=O, SO₂ oder NR¹⁹ ist;
R¹⁵ optional substituiertes mono- oder bicyclisches C₃-C₆-Cycloalkyl, mono- oder bicyclisches optional substituiertes C₆-C₁₂-Aryl, mono- oder bicyclisches optional substituiertes 5- bis 10-gliedriges Heteroaryl oder optional substituierter mono- oder bicyclischer 3- bis 8-gliedriger Heterocyclus ist;
R¹⁶ optional substituiertes C₁-C₆-Alkyl, optional substituiertes C₂-C₆-Alkenyl, optional substituiertes C₂-C₆-Alkinyl, optional substituiertes mono- oder bicyclisches C₃-C₆-Cycloalkyl, mono- oder bicyclisches optional substituiertes C₆-C₁₂-Aryl, mono- oder bicyclisches optional substituiertes 5- bis 10-gliedriges Heteroaryl oder optional substituierter mono- oder bicyclischer 3- bis 8-gliedriger Heterocyclus ist; und
R¹⁷ bis R¹⁹ unabhängig H, ein optional substituiertes C₁-C₆-Alkyl oder ein optional substituiertes C₂-C₆-Alkenyl sind;
oder pharmazeutisch verträglicher Komplex, pharmazeutisch verträgliches Salz, Solvat, pharmazeutisch verträgliche tautomere Form oder polymorphe Form davon; und
wobei die Verbindung nicht 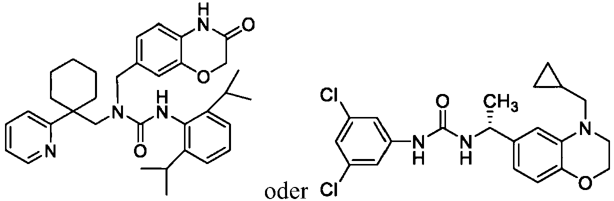 ist.

2. Verbindung nach Anspruch 1, wobei R¹ H, Halogen, OH, CN oder optional substituiertes C₁-C₆-Alkyl ist.

3. Verbindung nach einem vorhergehenden Anspruch, wobei eines von R² und R³ -A-NR¹⁷-C(O)-NR¹⁸-R¹⁵ ist und das andere von R² und R³ H, Halogen, OH, CN, COOR¹³, CONR¹³R¹⁴, NR¹³R¹⁴, NR¹³COR¹⁴ oder optional substituiertes C₁-C₆-Alkyl ist, und R¹³ und R¹⁴ jeweils unabhängig ausgewählt sind aus der Gruppe bestehend aus H, optional substituiertem C₁-C₃-Alkyl, optional substituiertem C₂-C₃-Alkenyl und optional substituiertem C₂-C₃-Alkinyl; und wobei bevorzugt eines von R² und R³ -A-NR¹⁷-C(O)-NR¹⁸-R¹⁵ ist und das andere von R² und R³ H, Halogen, OH, CN, CONR¹³R¹⁴, NR¹³R¹⁴ oder C₁-C₃-Alkyl ist, und R¹³ und R¹⁴ jeweils unabhängig ausgewählt sind aus der Gruppe bestehend aus H, C₁-C₃-Alkyl, C₂-C₃-Alkenyl und C₂-C₃-Alkinyl.

4. Verbindung nach einem vorhergehenden Anspruch, wobei A ein optional substituiertes C₁-C₆-Alkylen, ein optional substituiertes C₂-C₆-Alkenylen oder ein optional substituiertes C₂-C₆-Alkinylen ist, wobei das Alkylen, Alkenylen oder Alkinylen unsubstituiert oder substituiert ist mit einem oder mehreren von Halogen, OR²⁰, CN, Oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), optional substituiertem C₆-C₁₂-Aryl, optional substituiertem 5- bis 10-gliedrigen Heteroaryl, optional substituiertem C₃-C₆-Cycloalkyl oder optional substituiertem 3- bis 8-gliedrigen Heterocyclus; und wobei bevorzugt A wie folgt ist: -CH₂-, 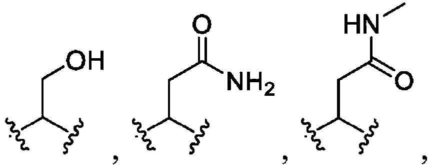 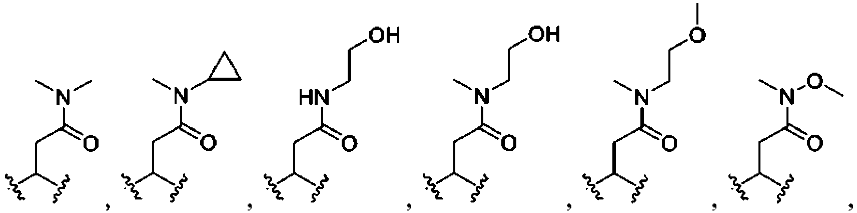 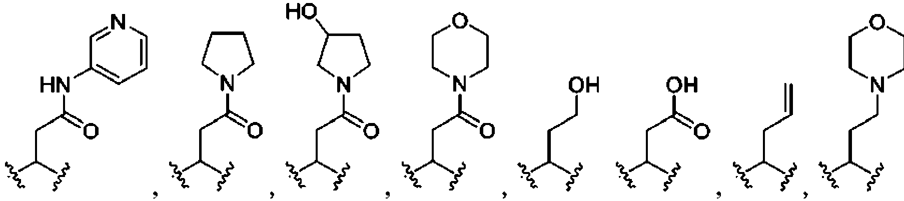 oder

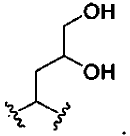

5. Verbindung nach einem vorhergehenden Anspruch, wobei R⁴ H, Halogen, OH, CN oder optional substituiertes C₁-C₆-Alkyl ist.

6. Verbindung nach einem vorhergehenden Anspruch, wobei R⁵ -L¹-L²-R¹⁶ ist und wobei optional:
- L¹ fehlt, ein optional substituiertes C₁-C₃-Alkylen, ein optional substituiertes C₂-C₃-Alkenylen oder ein optional substituiertes C₂-C₃-Alkinylen ist;
- L² fehlt oder O, S, S=O, SO₂ oder NR¹⁹ ist; und/oder
- R¹⁶ optional substituiertes mono- oder bicyclisches C₃-C₆-Cycloalkyl, mono- oder bicyclisches optional substituiertes C₆-C₁₂-Aryl, mono- oder bicyclisches optional substituiertes 5- bis 10-gliedriges Heteroaryl oder optional substituierter mono- oder bicyclischer 3- bis 8-gliedriger Heterocyclus ist.

7. Verbindung nach einem von Anspruch 1 bis 5, wobei R⁵ optional substituiertes C₁-C₆-Alkyl ist.

8. Verbindung nach einem vorhergehenden Anspruch, wobei X⁶ CR⁷R⁸ ist und R⁷ und R⁸ unabhängig H, Halogen, OH, CONR¹³R¹⁴ oder optional substituiertes C₁-C₆-Alkyl sind.

9. Verbindung nach einem von Anspruch 1 bis 7, wobei X⁶ CO ist.

10. Verbindung nach einem vorhergehenden Anspruch, wobei:
- X⁷ S, SO oder O ist;
- R⁹ und R¹⁰ unabhängig H, Halogen, OR¹³, CN, CONR¹³R¹⁴, NR¹³R¹⁴, NR¹³COR¹⁴, optional substituiertes C₁-C₃-Alkyl, optional substituiertes C₂-C₃-Alkenyl oder optional substituiertes C₂-C₃-Alkinyl sind; und
- R¹³ und R¹⁴ unabhängig H, optional substituiertes C₁-C₃-Alkyl, optional substituiertes C₂-C₃-Alkenyl oder optional substituiertes C₂-C₃-Alkinyl sind.

11. Verbindung nach Anspruch 1, wobei die Verbindung wie folgt ist:
1-((4-Benzyl-3,4-dihydro-2H-benzo[b][1,4]thiazin-6-yl)methyl)-3-(1H-indol-6-yl)harnstoff;
1-((4-Benzyl-3-oxo-3,4-dihydro-2H-benzo[b][1,4]oxazin-7-yl)methyl)-3-(1H-indol-6-yl)harnstoff;
1-((4-Benzyl-3,4-dihydro-2H-benzo[b][1,4]oxazin-7-yl)methyl)-3-(1H-indol-6-yl)harnstoff;
3-(3-(1H-Indol-6-yl)ureido)-3-(4-benzyl-3,4-dihydro-2H-benzo[b][1,4]thiazin-6-yl)-N,N-dimethylpropanamid;
(S)-3-(3-(1H-Indol-6-yl)ureido)-3-(4-benzyl-3,4-dihydro-2H-benzo[b][1,4]thiazin-6-yl)-N,N-dimethylpropanamid;
(R)-3-(3-(1H-Indol-6-yl)ureido)-3-(4-benzyl-3,4-dihydro-2H-benzo[b][1,4]thiazin-6-yl)-N,N-dimethylpropanamid;
1-(1-(4-Benzyl-3,4-dihydro-2H-benzo[b][1,4]oxazin-6-yl)-3,4-dihydroxybutyl)-3-(1H-indol-6-yl)harnstoff;
1-(1-(4-Benzyl-3,4-dihydro-2H-benzo[b][1,4]thiazin-6-yl)but-3-en-1-yl)-3-(1H-indol-6-yl)harnstoff;
3-(3-(1H-Indol-6-yl)ureido)-3-(4-benzyl-3,4-dihydro-2H-benzo[b][1,4]thiazin-6-yl)propansäure;
1-(1-(4-Benzyl-3,4-dihydro-2H-benzo[b][1,4]thiazin-6-yl)-2-hydroxyethyl)-3-(1H-indol-6-yl)harnstoff;
(R)-1-(1-(4-Benzyl-3,4-dihydro-2H-benzo[b][1,4]thiazin-6-yl)-2-hydroxyethyl)-3-(1H-indol-6-yl)harnstoff;
(S)-1-(1-(4-Benzyl-3,4-dihydro-2H-benzo[b][1,4]thiazin-6-yl)-2-hydroxyethyl)-3-(1H-indol-6-yl)harnstoff;
1-(1H-Indol-6-yl)-3-((4-phenyl-3,4-dihydro-2H-benzo[b][1,4]thiazin-6-yl)methyl)harnstoff;
1-((4-Benzyl-3,4-dihydro-2H-benzo[b][1,4]oxazin-6-yl)methyl)-3-(1H-indol-6-yl)harnstoff;
1-((4-Benzyl-3,4-dihydro-2H-benzo[b][1,4]thiazin-7-yl)methyl)-3-(1H-indol-6-yl)harnstoff;
1-((4-Benzyl-3-oxo-3,4-dihydro-2H-benzo[b][1,4]thiazin-7-yl)methyl)-3-(1H-indol-6-yl)harnstoff;
1-((4-Benzyl-3-oxo-3,4-dihydro-2H-benzo[b][1,4]thiazin-6-yl)methyl)-3-(1H-indol-6-yl)harnstoff;
1-(1H-Indol-6-yl)-3-((4-phenyl-3,4-dihydro-2H-benzo[b][1,4]oxazin-6-yl)methyl)harnstoff;
1-(1H-Indol-6-yl)-3-((4-phenyl-3,4-dihydro-2H-benzo[b][1,4]thiazin-7-yl)methyl)harnstoff;
1-(1H-Indol-6-yl)-3-((4-phenyl-3,4-dihydro-2H-benzo[b][1,4]oxazin-7-yl)methyl)harnstoff;
1-(1-(4-Benzyl-3,4-dihydro-2H-benzo[b][1,4]thiazin-6-yl)-3-morpholinopropyl)-3-(1H-indol-6-yl)harnstoff;
3-(3-(1H-Indol-6-yl)ureido)-3-(4-benzyl-3,4-dihydro-2H-benzo[b][1,4]thiazin-6-yl)propanamid;
1-(1-(4-Benzyl-3,4-dihydro-2H-benzo[b][1,4]thiazin-6-yl)-3-hydroxypropyl)-3-(1H-indol-6-yl)harnstoff;
1-(1-(4-Benzyl-1-oxido-3,4-dihydro-2H-benzo[b][1,4]thiazin-6-yl)-3,4-dihydroxybutyl)-3-(1H-indol-6-yl)harnstoff;
3-(3-(1H-Indol-6-yl)ureido)-3-(4-benzyl-3,4-dihydro-2H-benzo[b][1,4]thiazin-6-yl)-N-methylpropanamid;
1-(1-(4-Benzyl-3,4-dihydro-2H-benzo[b][1,4]thiazin-6-yl)-3-oxo-3-(pyrrolidin-1-yl)propyl)-3-(1H-indol-6-yl)harnstoff;
3-(3-(1H-Indol-6-yl)ureido)-3-(4-benzyl-3,4-dihydro-2H-benzo[b][1,4]thiazin-6-yl)-N-methoxy-N-methylpropanamid;
1-(1-(4-Benzyl-3,4-dihydro-2H-benzo[b][1,4]thiazin-6-yl)-3-(3-hydroxypyrrolidin-1-yl)-3-oxopropyl)-3-(1H-indol-6-yl)harnstoff;
1-(1-(4-Benzyl-3,4-dihydro-2H-benzo[b][1,4]thiazin-6-yl)-3-morpholino-3-oxopropyl)-3-(1H-indol-6-yl)harnstoff;
3-(3-(1H-Indol-6-yl)ureido)-3-(4-benzyl-3,4-dihydro-2H-benzo[b][1,4]thiazin-6-yl)-N-(2-hydroxyethyl)-N-methylpropanamid;
3-(3-(1H-Indol-6-yl)ureido)-3-(4-benzyl-3,4-dihydro-2H-benzo[b][1,4]thiazin-6-yl)-N-cyclopropyl-N-methylpropanamid;
3-(3-(1H-Indol-6-yl)ureido)-3-(4-benzyl-3,4-dihydro-2H-benzo[b][1,4]thiazin-6-yl)-N-(2-methoxyethyl)-N-methylpropanamid;
3-(3-(1H-Indol-6-yl)ureido)-3-(4-benzyl-3,4-dihydro-2H-benzo[b][1,4]thiazin-6-yl)-N-(pyridin-3-yl)propanamid;
3-(3-(1H-Indol-6-yl)ureido)-3-(4-benzyl-3,4-dihydro-2H-benzo[b][1,4]thiazin-6-yl)-N-(2-hydroxyethyl)propanamid;
1-(1-(4-(2-Chlor-6-fluorbenzyl)-3,4-dihydro-2H-benzo[b][1,4]thiazin-6-yl)but-3-en-1-yl)-3-(1H-indol-6-yl)harnstoff; oder
1-(1-(4-Benzyl-3,4-dihydro-2H-benzo[b][1,4]thiazin-6-yl)but-3-en-1-yl)-3-(1H-indol-3-yl)harnstoff.

12. Pharmazeutische Zusammensetzung, umfassend eine Verbindung nach einem vorhergehenden Anspruch, oder ein pharmazeutisch verträgliches Salz, Solvat, eine pharmazeutisch verträgliche tautomere Form oder polymorphe Form davon und ein pharmazeutisch verträgliches Vehikel.

13. Verbindung wie in einem der Ansprüche 1 bis 11 definiert, oder pharmazeutisch verträglicher Komplex, pharmazeutisch verträgliches Salz, Solvat, pharmazeutisch verträgliche tautomere Form oder polymorphe Form davon, einer pharmazeutischen Zusammensetzung wie durch Anspruch 12 definiert zur Verwendung als Medikament.

14. Verbindung der Formel **(I):** 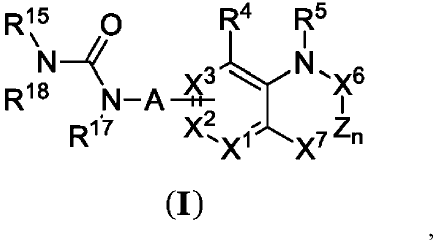
wobei X¹ CR¹ ist;
X² CR² ist und X³ CR³ ist;
X⁶ C=O oder CR⁷R⁸ ist;
Z CR⁹R¹⁰ ist;
X⁷ S, SO oder O ist;
A ein optional substituiertes C₁-C₆-Alkylen, ein optional substituiertes C₂-C₆-Alkenylen oder ein optional substituiertes C₂-C₆-Alkinylen ist;
n 1 ist;
R¹, R⁴ und R⁸ jeweils unabhängig ausgewählt sind aus der Gruppe bestehend aus H, Halogen, OR¹³, CN, CONR¹³R¹⁴, NR¹³R¹⁴, NR¹³COR¹⁴, optional substituiertem C₁-C₆-Alkyl, optional substituiertem mono- oder bicyclischen C₃-C₆-Cycloalkyl, mono- oder bicyclischem optional substituierten C₆-C₁₂-Aryl, mono- oder bicyclischem optional substituierten 5- bis 10-gliedrigen Heteroaryl und optional substituiertem mono- oder bicyclischen 3- bis 8-gliedrigen Heterocyclus;
R⁹ und R¹⁰ jeweils unabhängig ausgewählt sind aus der Gruppe bestehend aus H, Halogen, OR¹³, CN, CONR¹³R¹⁴, NR¹³R¹⁴, NR¹³COR¹⁴, optional substituiertem C₁-C₆-Alkyl, optional substituiertem C₂-C₆-Alkenyl oder optional substituiertem C₂-C₆-Alkinyl;
eines von R² und R³ -A-NR¹⁷-C(O)-NR¹⁸-R¹⁵ ist und das andere von R² und R³ ausgewählt ist aus der Gruppe bestehend aus H, Halogen, OR¹³, CN, COOR¹³, CONR¹³R¹⁴, NR¹³R¹⁴, NR¹³COR¹⁴, optional substituiertem C₁-C₆-Alkyl, optional substituiertem mono- oder bicyclischen C₃-C₆-Cycloalkyl, mono- oder bicyclischem optional substituierten C₆-C₁₂-Aryl, mono- oder bicyclischem optional substituierten 5- bis 10-gliedrigen Heteroaryl und optional substituiertem mono- oder bicyclischen 3- bis 8-gliedrigen Heterocyclus;
R⁵ ausgewählt ist aus der Gruppe bestehend aus H, CONR¹³R¹⁴, optional substituiertem C₁-C₆-Alkyl, optional substituiertem C₁-C₆-Alkylsulfonyl, optional substituiertem mono- oder bicyclischen C₃-C₆-Cycloalkyl, mono- oder bicyclischem optional substituierten C₆-C₁₂-Aryl, mono- oder bicyclischem optional substituierten 5- bis 10-gliedrigen Heteroaryl, optional substituiertem mono- oder bicyclischen 3- bis 8-gliedrigen Heterocyclus und L¹-L²-R¹⁶;
R⁷ ausgewählt ist aus der Gruppe bestehend aus H, Halogen, CONR¹³R¹⁴, optional substituiertem C₁-C₆-Alkyl, optional substituiertem mono- oder bicyclischen C₃-C₆-Cycloalkyl, optional substituiertem C₂-C₆-Alkenyl, optional substituiertem C₂-C₆-Alkinyl, mono- oder bicyclischem optional substituierten C₆-C₁₂-Aryl, mono- oder bicyclischem optional substituierten 5- bis 10-gliedrigen Heteroaryl, optional substituiertem mono- oder bicyclischen 3- bis 8-gliedrigen Heterocyclus und L¹-L²-R¹⁶_{;}
wobei ein Maximum von einem von R⁵ und R⁷ -L¹-L²-R¹⁶ ist;
R¹³ und R¹⁴ jeweils unabhängig ausgewählt sind aus der Gruppe bestehend aus H, Halogen, OH, CN, COOH, CONH₂, NH₂, NHCOH, optional substituiertem C₁-C₆-Alkyl, optional substituiertem C₁-C₆-Alkylsulfonyl, optional substituiertem mono- oder bicyclischen C₃-C₆-Cycloalkyl, optional substituiertem C₂-C₆-Alkenyl, optional substituiertem C₂-C₆-Alkinyl, optional substituiertem C₁-C₆-Alkoxy, optional substituierter C₁-C₆-Alkoxycarbonylgruppe, mono- oder bicyclischem optional substituierten C₆-C₁₂-Aryl, mono- oder bicyclischem optional substituierten 5- bis 10-gliedrigen Heteroaryl, optional substituiertem mono- oder bicyclischen 3-bis 8-gliedrigen Heterocyclus, optional substituiertem Aryloxy, optional substituiertem Heteroaryloxy und optional substituiertem Heterocyclyloxy;
L¹ fehlt oder ein optional substituiertes C₁-C₆-Alkylen, ein optional substituiertes C₂-C₆-Alkenylen, ein optional substituiertes C₂-C₆-Alkinylen, O, S, S=O, SO₂ oder NR¹⁹ ist;
L² fehlt oder ein optional substituiertes C₁-C₆-Alkylen, ein optional substituiertes C₂-C₆-Alkenylen, ein optional substituiertes C₂-C₆-Alkinylen, O, S, S=O, SO₂ oder NR¹⁹ ist;
R¹⁵ optional substituiertes mono- oder bicyclisches C₃-C₆-Cycloalkyl, mono- oder bicyclisches optional substituiertes C₆-C₁₂-Aryl, mono- oder bicyclisches optional substituiertes 5- bis 10-gliedriges Heteroaryl oder optional substituierter mono- oder bicyclischer 3- bis 8-gliedriger Heterocyclus ist;
R¹⁶ optional substituiertes C₁-C₆-Alkyl, optional substituiertes C₂-C₆-Alkenyl, optional substituiertes C₂-C₆-Alkinyl, optional substituiertes mono- oder bicyclisches C₃-C₆-Cycloalkyl, mono- oder bicyclisches optional substituiertes C₆-C₁₂-Aryl, mono- oder bicyclisches optional substituiertes 5- bis 10-gliedriges Heteroaryl oder optional substituierter mono- oder bicyclischer 3- bis 8-gliedriger Heterocyclus ist; und
R¹⁷ bis R¹⁹ unabhängig H, ein optional substituiertes C₁-C₆-Alkyl oder ein optional substituiertes C₂-C₆-Alkenyl sind;
oder pharmazeutisch verträglicher Komplex, pharmazeutisch verträgliches Salz, Solvat, pharmazeutisch verträgliche tautomere Form oder polymorphe Form davon,
oder eine pharmazeutische Zusammensetzung, umfassend eine Verbindung der Formel (I) oder einen pharmazeutisch verträglichen Komplex, ein pharmazeutisch verträgliches Salz, Solvat, eine pharmazeutisch verträgliche tautomere Form oder polymorphe Form davon und ein pharmazeutisch verträgliches Vehikel,
zur Verwendung beim Behandeln, Lindern oder Verhindern einer Erkrankung ausgewählt aus Leberfibrose, Fettlebererkrankung, nichtalkoholischer Steatohepatitis (NASH), Lungenfibrose, Lupus, Sepsis, rheumatoider Arthritis (RA), Typ-I-Diabetes, STING-assoziierter Vaskulopathie mit Beginn im Säuglingsalter (SAVI), Aicardi-Goutieres-Syndrom (AGS), familiärem Chilblain-Lupus (FCL), systemischem Lupus erythematodes (SLE), retinaler Vaskulopathie, Neuroentzündung, systemischem Entzündungsreaktionssyndrom, Pankreatitis, kardiovaskulärer Erkrankung, Nierenfibrose, Schlaganfall und altersbedingter Makuladegeneration (AMD).

15. Verbindung oder Zusammensetzung zur Verwendung nach Anspruch 14, wobei die Erkrankung Fibrose ist und die Fibrose ausgewählt ist aus der Gruppe bestehend aus Leberfibrose, Lungenfibrose oder Nierenfibrose; oder wobei die Erkrankung Fettlebererkrankung ist und die Fettlebererkrankung nichtalkoholische (oder einfache) Fettleber oder nichtalkoholische Steatohepatitis (NASH) ist.

## Revendications

1. Composé de formule (I) : 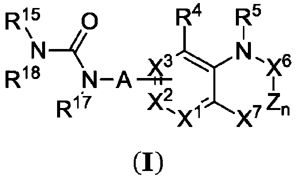
, dans lequel X¹ est CR¹ ;
X² est CR² ;
X³ est CR³ ;
X⁶ est C=O ou CR⁷R⁸ ;
Z est CR⁹R¹⁰ ;
X⁷ est S, SO ou O ;
A est un alkylène en C₁-C₆ éventuellement substitué, un alcénylène en C₂-C₆ éventuellement substitué ou un alcynylène en C₂-C₆ éventuellement substitué ;
n est 1 ;
R¹, R⁴ et R⁸ sont chacun indépendamment choisis dans le groupe constitué de H, halogène, OR¹³, CN, CONR¹³R¹⁴, NR¹³R¹⁴, NR¹³COR¹⁴, alkyle en C₁-C₆ éventuellement substitué, cycloalkyle en C₃-C₆ mono- ou bicyclique éventuellement substitué, aryle en C₆-C₁₂ mono- ou bicyclique éventuellement substitué, hétéroaryle à 5 à 10 chaînons mono- ou bicyclique éventuellement substitué et hétérocycle à 3 à 8 chaînons mono- ou bicyclique éventuellement substitué ;
R⁹ et R¹⁰ sont chacun indépendamment choisis dans le groupe constitué de H, halogène, OR¹³, CN, CONR¹³R¹⁴, NR¹³R¹⁴, NR¹³COR¹⁴, alkyle en C₁-C₆ éventuellement substitué, alcényle en C₂-C₆ éventuellement substitué ou alcynyle en C₂-C₆ éventuellement substitué ;
l'un de R² et R³ est -A-NR¹⁷-C(O)-NR¹⁸-R¹⁵ et l'autre de R² et R³ est choisi dans le groupe constitué de H, halogène, OR¹³, CN, COOR¹³, CONR¹³R¹⁴, NR¹³R¹⁴, NR¹³COR¹⁴, alkyle en C₁-C₆ éventuellement substitué, cycloalkyle en C₃-C₆ mono- ou bicyclique éventuellement substitué, aryle en C₆-C₁₂ mono- ou bicyclique éventuellement substitué, hétéroaryle à 5 à 10 chaînons mono- ou bicyclique éventuellement substitué et hétérocycle à 3 à 8 chaînons mono- ou bicyclique éventuellement substitué ;
R⁵ est choisi dans le groupe constitué de H, CONR¹³R¹⁴, alkyle en C₁-C₆ éventuellement substitué, alkylsulfonyle en C₁-C₆ éventuellement substitué, cycloalkyle en C₃-C₆ mono- ou bicyclique éventuellement substitué, aryle en C₆-C₁₂ mono- ou bicyclique éventuellement substitué, hétéroaryle à 5 à 10 chaînons mono- ou bicyclique éventuellement substitué, hétérocycle à 3 à 8 chaînons mono- ou bicyclique éventuellement substitué et L¹-L²-R¹⁶ ;
R⁷ est choisi dans le groupe constitué de H, halogène, CONR¹³R¹⁴, alkyle en C₁-C₆ éventuellement substitué, cycloalkyle en C₃-C₆ mono- ou bicyclique éventuellement substitué, alcényle en C₂-C₆ éventuellement substitué, alcynyle en C₂-C₆ éventuellement substitué, aryle en C₆-C₁₂ mono- ou bicyclique éventuellement substitué, hétéroaryle à 5 à 10 chaînons mono- ou bicyclique éventuellement substitué, hétérocycle à 3 à 8 chaînons mono- ou bicyclique éventuellement substitué et L¹-L²-R¹⁶ ;
dans lequel un maximum de l'un de R⁵ et R⁷ est -L¹-L²-R¹⁶ ;
R¹³ et R¹⁴ sont chacun indépendamment choisis dans le groupe constitué de H, halogène, OH, CN, COOH, CONH₂, NH₂, NHCOH, alkyle en C₁-C₆ éventuellement substitué, alkylsulfonyle en C₁-C₆ éventuellement substitué, cycloalkyle en C₃-C₆ mono- ou bicyclique éventuellement substitué, alcényle en C₂-C₆ éventuellement substitué, alcynyle en C₂-C₆ éventuellement substitué, alkoxy en C₁-C₆ éventuellement substitué, groupe alkoxycarbonyle en C₁-C₆ éventuellement substitué, aryle en C₆-C₁₂ mono- ou bicyclique éventuellement substitué, hétéroaryle à 5 à 10 chaînons mono- ou bicyclique éventuellement substitué, hétérocycle à 3 à 8 chaînons mono- ou bicyclique éventuellement substitué, aryloxy éventuellement substitué, hétéroaryloxy éventuellement substitué et hétérocyclyloxy éventuellement substitué ;
L¹ est absent ou un alkylène en C₁-C₆ éventuellement substitué, un alcénylène en C₂-C₆ éventuellement substitué, un alcynylène en C₂-C₆ éventuellement substitué, O, S, S=O, SO₂ ou NR¹⁹ ;
L² est absent ou un alkylène en C₁-C₆ éventuellement substitué, un alcénylène en C₂-C₆ éventuellement substitué, un alcynylène en C₂-C₆ éventuellement substitué, O, S, S=O, SO₂ ou NR¹⁹ ;
R¹⁵ est un cycloalkyle en C₃-C₆ mono- ou bicyclique éventuellement substitué, un aryle en C₆-C₁₂ mono- ou bicyclique éventuellement substitué, un hétéroaryle à 5 à 10 chaînons mono- ou bicyclique éventuellement substitué ou un hétérocycle à 3 à 8 chaînons mono- ou bicyclique éventuellement substitué ;
R¹⁶ est un alkyle en C₁-C₆ éventuellement substitué, un alcényle en C₂-C₆ éventuellement substitué, un alcynyle en C₂-C₆ éventuellement substitué, un cycloalkyle en C₃-C₆ mono- ou bicyclique éventuellement substitué, un aryle en C₆-C₁₂ mono- ou bicyclique éventuellement substitué, un hétéroaryle à 5 à 10 chaînons mono- ou bicyclique éventuellement substitué ou un hétérocycle à 3 à 8 chaînons mono- ou bicyclique éventuellement substitué ; et
R¹⁷ à R¹⁹ sont indépendamment H, un alkyle en C₁-C₆ éventuellement substitué ou un alcényle en C₂-C₆ éventuellement substitué ;
ou un complexe, un sel, un solvate, une forme tautomère ou une forme polymorphe pharmaceutiquement acceptable de celui-ci ; et
ledit composé n'est pas

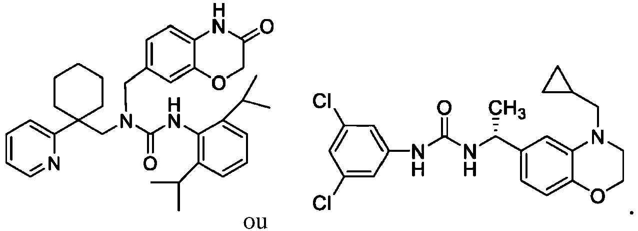

2. Composé selon la revendication 1, dans lequel R¹ est H, halogène, OH, CN ou alkyle en C₁-C₆ éventuellement substitué.

3. Composé selon l'une quelconque des revendications précédentes, dans lequel l'un de R² et R³ est -A-NR¹⁷-C(O)-NR¹⁸-R¹⁵ et l'autre de R² et R³ est H, halogène, OH, CN, COOR¹³, CONR¹³R¹⁴, NR¹³R¹⁴, NR¹³COR¹⁴ ou alkyle en C₁-C₆ éventuellement substitué, et R¹³ et R¹⁴ sont chacun indépendamment choisis dans le groupe constitué de H, alkyle en C₁-C₃ éventuellement substitué, alcényle en C₂-C₃ éventuellement substitué et alcynyle en C₂-C₃ éventuellement substitué ; et de préférence dans lequel l'un de R² et R³ est -A-NR¹⁷-C(O)-NR¹⁸-R¹⁵ et l'autre de R² et R³ est H, halogène, OH, CN, CONR¹³R¹⁴, NR¹³R¹⁴ ou alkyle en C₁-C₃, et R¹³ et R¹⁴ sont chacun indépendamment choisis dans le groupe constitué de H, alkyle en C₁-C₃, alcényle en C₂-C₃ et alcynyle en C₂-C₃.

4. Composé selon l'une quelconque des revendications précédentes, dans lequel A est un alkylène en C₁-C₆ éventuellement substitué, un alcénylène en C₂-C₆ éventuellement substitué ou un alcynylène en C₂-C₆ éventuellement substitué, l'alkylène, l'alcénylène ou l'alcynylène étant non substitué ou substitué par un ou plusieurs parmi halogène, OR²⁰, CN, oxo, C(O)R²⁰, COOR²⁰, OC(O)R²⁰, CONR²⁰R²¹, NR²⁰R²¹, NR²⁰C(O)R²¹, =NOR²⁰, SR²⁰, SO₂R²⁰, OSO₂R²⁰, SO₂NR²⁰R²¹, OP(O)(OR²⁰)(OR²¹), aryle en C₆-C₁₂ éventuellement substitué, hétéroaryle à 5 à 10 chaînons éventuellement substitué, cycloalkyle en C₃-C₆ éventuellement substitué ou hétérocycle à 3 à 8 chaînons éventuellement substitué ; et de préférence dans lequel A est -CH₂-, 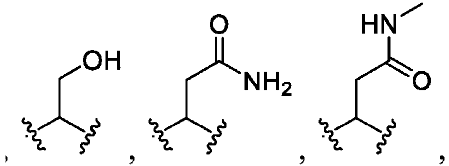 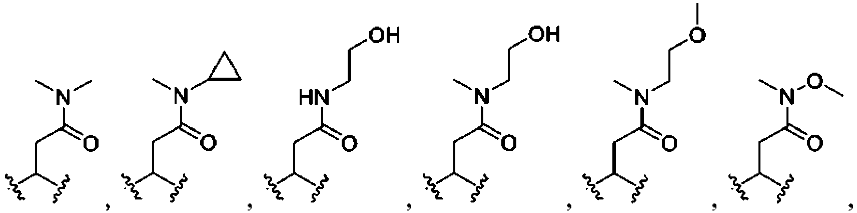 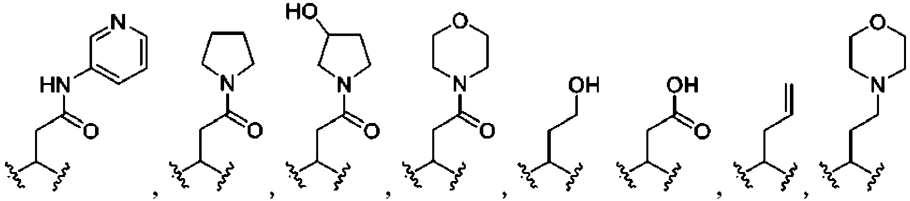 ou

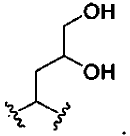

5. Composé selon l'une quelconque des revendications précédentes, dans lequel R⁴ est H, halogène, OH, CN ou alkyle en C₁-C₆ éventuellement substitué.

6. Composé selon l'une quelconque des revendications précédentes, dans lequel R⁵ est -L¹-L²-R¹⁶, et éventuellement dans lequel :
- L¹ est absent, un alkylène en C₁-C₃ éventuellement substitué, un alcénylène en C₂-C₃ éventuellement substitué ou un alcynylène en C₂-C₃ éventuellement substitué ;
- L² est absent ou O, S, S=O, SO₂ ou NR¹⁹ ; et/ou
- R¹⁶ est cycloalkyle en C₃-C₆ mono- ou bicyclique éventuellement substitué, aryle en C₆-C₁₂ mono- ou bicyclique éventuellement substitué, hétéroaryle à 5 à 10 chaînons mono- ou bicyclique éventuellement substitué ou hétérocycle à 3 à 8 chaînons mono- ou bicyclique éventuellement substitué.

7. Composé selon l'une quelconque des revendications 1 à 5, dans lequel R⁵ est alkyle en C₁-C₆ éventuellement substitué.

8. Composé selon l'une quelconque des revendications précédentes, dans lequel X⁶ est CR⁷R⁸ et R⁷ et R⁸ sont indépendamment H, halogène, OH, CONR¹³R¹⁴ ou alkyle en C₁-C₆ éventuellement substitué.

9. Composé selon l'une quelconque des revendications 1 à 7, dans lequel X⁶ est CO.

10. Composé selon l'une quelconque des revendications précédentes, dans lequel :
- X⁷ est S, SO ou O ;
- R⁹ et R¹⁰ sont indépendamment H, halogène, OR¹³, CN, CONR¹³R¹⁴, NR¹³R¹⁴, NR¹³COR¹⁴, alkyle en C₁-C₃ éventuellement substitué, alcényle en C₂-C₃ éventuellement substitué ou alcynyle en C₂-C₃ éventuellement substitué ; et
- R¹³ et R¹⁴ sont indépendamment H, alkyle en C₁-C₃ éventuellement substitué, alcényle en C₂-C₃ éventuellement substitué ou alcynyle en C₂-C₃ éventuellement substitué.

11. Composé selon la revendication 1, ledit composé étant :
1-((4-Benzyl-3,4-dihydro-2H-benzo[b][1,4]thiazin-6-yl)méthyl)-3-(1H-indol-6-yl)urée ;
1-((4-Benzyl-3-oxo-3,4-dihydro-2H-benzo[b][1,4]oxazin-7-yl)méthyl)-3-(1H-indol-6-yl)urée ;
1-((4-Benzyl-3,4-dihydro-2H-benzo[b][1,4]oxazin-7-yl)méthyl)-3-(1H-indol-6-yl)urée ;
3-(3-(1H-indol-6-yl)uréido)-3-(4-benzyl-3,4-dihydro-2H-benzo[b][1,4]thiazin-6-yl)-N,N-diméthylpropanamide ;
(S)-3-(3-(1H-indol-6-yl)uréido)-3-(4-benzyl-3,4-dihydro-2H-benzo[b][1,4]thiazin-6-yl)-N,N-diméthylpropanamide ;
(R)-3-(3-(1H-indol-6-yl)uréido)-3-(4-benzyl-3,4-dihydro-2H-benzo[b][1,4]thiazin-6-yl)-N,N-diméthylpropanamide ;
1-(1-(4-Benzyl-3,4-dihydro-2H-benzo[b][1,4]oxazin-6-yl)-3,4-dihydroxybutyl)-3-(1H-indol-6-yl)urée ;
1-(1-(4-Benzyl-3,4-dihydro-2H-benzo[b][1,4]thiazin-6-yl)but-3-en-1-yl)-3-(1H-indol-6-yl)urée ; Acide 3-(3-(1H-indol-6-yl)uréido)-3-(4-benzyl-3,4-dihydro-2H-benzo[b][1,4]thiazin-6-yl)propanoïque ;
1-(1-(4-Benzyl-3,4-dihydro-2H-benzo[b][1,4]thiazin-6-yl)-2-hydroxyéthyl)-3-(1H-indol-6-yl)urée ;
(R)-1-(1-(4-benzyl-3,4-dihydro-2H-benzo[b][1,4]thiazin-6-yl)-2-hydroxyéthyl)-3-(1H-indol-6-yl)urée ;
(S)-1-(1-(4-benzyl-3,4-dihydro-2H-benzo[b][1,4]thiazin-6-yl)-2-hydroxyéthyl)-3-(1H-indol-6-yl)urée;
1-(1H-Indol-6-yl)-3-((4-phényl-3,4-dihydro-2H-benzo[b][1,4]thiazin-6-yl)méthyl)urée ;
1-((4-benzyl-3,4-dihydro-2H-benzo[b][1,4]oxazin-6-yl)méthyl)-3-(1H-indol-6-yl)urée ;
1-((4-benzyl-3,4-dihydro-2H-benzo[b][1,4]thiazin-7-yl)méthyl)-3-(1H-indol-6-yl)urée ;
1-((4-benzyl-3-oxo-3,4-dihydro-2H-benzo[b][1,4]thiazin-7-yl)méthyl)-3-(1H-indol-6-yl)urée ;
1-((4-benzyl-3-oxo-3,4-dihydro-2H-benzo[b][1,4]thiazin-6-yl)méthyl)-3-(1H-indol-6-yl)urée ;
1-(1H-indol-6-yl)-3-((4-phényl-3,4-dihydro-2H-benzo[b][1,4]oxazin-6-yl)méthyl)urée ;
1-(1H-indol-6-yl)-3-((4-phényl-3,4-dihydro-2H-benzo[b][1,4]thiazin-7-yl)méthyl)urée ;
1-(1H-indol-6-yl)-3-((4-phényl-3,4-dihydro-2H-benzo[b][1,4]oxazin-7-yl)méthyl)urée ;
1-(1-(4-benzyl-3,4-dihydro-2H-benzo[b][1,4]thiazin-6-yl)-3-morpholinopropyl)-3-(1H-indol-6-yl)urée ;
3-(3-(1H-indol-6-yl)uréido)-3-(4-benzyl-3,4-dihydro-2H-benzo[b][1,4]thiazin-6-yl)propanamide ;
1-(1-(4-benzyl-3,4-dihydro-2H-benzo[b][1,4]thiazin-6-yl)-3-hydroxypropyl)-3-(1H-indol-6-yl)urée ;
1-(1-(4-benzyl-1-oxido-3,4-dihydro-2H-benzo[b][1,4]thiazin-6-yl)-3,4-dihydroxybutyl)-3-(1H-indol-6-yl)urée ;
3-(3-(1H-indol-6-yl)uréido)-3-(4-benzyl-3,4-dihydro-2H-benzo[b][1,4]thiazin-6-yl)-N-méthylpropanamide ;
1-(1-(4-benzyl-3,4-dihydro-2H-benzo[b][1,4]thiazin-6-yl)-3-oxo-3-(pyrrolidin-1-yl)propyl)-3-(1H-indol-6-yl)urée ;
3-(3-(1H-indol-6-yl)uréido)-3-(4-benzyl-3,4-dihydro-2H-benzo[b][1,4]thiazin-6-yl)-N-méthoxy-N-méthylpropanamide ;
1-(1-(4-benzyl-3,4-dihydro-2H-benzo[b][1,4]thiazin-6-yl)-3-(3-hydroxypyrrolidin-1-yl)-3-oxopropyl)-3-(1H-indol-6-yl)urée ;
1-(1-(4-benzyl-3,4-dihydro-2H-benzo[b][1,4]thiazin-6-yl)-3-morpholino-3-oxopropyl)-3-(1H-indol-6-yl)urée ;
3-(3-(1H-indol-6-yl)uréido)-3-(4-benzyl-3,4-dihydro-2H-benzo[b][1,4]thiazin-6-yl)-N-(2-hydroxyéthyl)-N-méthylpropanamide ;
3-(3-(1H-indol-6-yl)uréido)-3-(4-benzyl-3,4-dihydro-2H-benzo[b][1,4]thiazin-6-yl)-N-cyclopropyl-N-méthylpropanamide ;
3-(3-(1H-indol-6-yl)ureido)-3-(4-benzyl-3,4-dihydro-2H-benzo[b][1,4]thiazin-6-yl)-N-(2-méthoxyéthyl)-N-méthylpropanamide ;
3-(3-(1H-indol-6-yl)uréido)-3-(4-benzy1-3,4-dihydro-2H-benzo[b][1,4]thiazin-6-yl)-N-(pyridin-3-yl)propanamide ;
3-(3-(1H-indol-6-yl)uréido)-3-(4-benzyl-3,4-dihydro-2H-benzo[b][1,4]thiazin-6-yl)-N-(2-hydroxyéthyl)propanamide ;
1-(1-(4-(2-chloro-6-fluorobenzyl)-3,4-dihydro-2H-benzo[b][1,4]thiazin-6-yl)but-3-en-1-yl)-3-(1H-indol-6-yl)urée ; ou
1-(1-(4-benzyl-3,4-dihydro-2H-benzo[b][1,4]thiazin-6-yl)but-3-en-1-yl)-3-(1H-indol-3-yl)urée.

12. Composition pharmaceutique comprenant un composé selon l'une quelconque des revendications précédentes, ou sel, solvate, forme tautomère ou forme polymorphe pharmaceutiquement acceptable de celui-ci, et véhicule pharmaceutiquement acceptable.

13. Composé tel que défini dans l'une quelconque des revendications 1 à 11, ou complexe, sel, solvate, forme tautomère ou forme polymorphe pharmaceutiquement acceptable de celui-ci, d'une composition pharmaceutique telle que définie par la revendication 12, destiné à être utilisé comme médicament.

14. Composé de formule (I) : 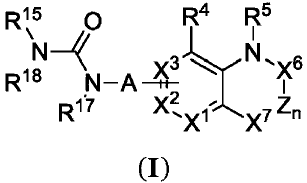
, dans lequel X¹ est CR¹ ;
X² est CR² et X³ est CR³ ;
X⁶ est C=O ou CR⁷R⁸ ;
Z est CR⁹R¹⁰ ;
X⁷ est S, SO ou O ;
A est un alkylène en C₁-C₆ éventuellement substitué, un alcénylène en C₂-C₆ éventuellement substitué ou un alcynylène en C₂-C₆ éventuellement substitué ;
n est 1 ;
R¹, R⁴ et R⁸ sont chacun indépendamment choisis dans le groupe constitué de H, halogène, OR¹³, CN, CONR¹³R¹⁴, NR¹³R¹⁴, NR¹³COR¹⁴, alkyle en C₁-C₆ éventuellement substitué, cycloalkyle en C₃-C₆ mono- ou bicyclique éventuellement substitué, aryle en C₆-C₁₂ mono- ou bicyclique éventuellement substitué, hétéroaryle à 5 à 10 chaînons mono- ou bicyclique éventuellement substitué et hétérocycle à 3 à 8 chaînons mono- ou bicyclique éventuellement substitué ;
R⁹ et R¹⁰ sont chacun indépendamment choisis dans le groupe constitué de H, halogène, OR¹³, CN, CONR¹³R¹⁴, NR¹³R¹⁴, NR¹³COR¹⁴, alkyle en C₁-C₆ éventuellement substitué, alcényle en C₂-C₆ éventuellement substitué ou alcynyle en C₂-C₆ éventuellement substitué ;
l'un de R² et R³ est -A-NR¹⁷-C(O)-NR¹⁸-R¹⁵ et l'autre de R² et R³ est choisi dans le groupe constitué de H, halogène, OR¹³, CN, COOR¹³, CONR¹³R¹⁴, NR¹³R¹⁴, NR¹³COR¹⁴, alkyle en C₁-C₆ éventuellement substitué, cycloalkyle en C₃-C₆ mono- ou bicyclique éventuellement substitué, aryle en C₆-C₁₂ mono- ou bicyclique éventuellement substitué, hétéroaryle à 5 à 10 chaînons mono- ou bicyclique éventuellement substitué et hétérocycle à 3 à 8 chaînons mono- ou bicyclique éventuellement substitué ;
R⁵ est choisi dans le groupe constitué de H, CONR¹³R¹⁴, alkyle en C₁-C₆ éventuellement substitué, alkylsulfonyle en C₁-C₆ éventuellement substitué, cycloalkyle en C₃-C₆ mono- ou bicyclique éventuellement substitué, aryle en C₆-C₁₂ mono- ou bicyclique éventuellement substitué, hétéroaryle à 5 à 10 chaînons mono- ou bicyclique éventuellement substitué, hétérocycle à 3 à 8 chaînons mono- ou bicyclique éventuellement substitué et L¹-L²-R¹⁶ ;
R⁷ est choisi dans le groupe constitué de H, halogène, CONR¹³R¹⁴, alkyle en C₁-C₆ éventuellement substitué, cycloalkyle en C₃-C₆ mono- ou bicyclique éventuellement substitué, alcényle en C₂-C₆ éventuellement substitué, alcynyle en C₂-C₆ éventuellement substitué, aryle en C₆-C₁₂ mono- ou bicyclique éventuellement substitué, hétéroaryle à 5 à 10 chaînons mono- ou bicyclique éventuellement substitué, hétérocycle à 3 à 8 chaînons mono- ou bicyclique éventuellement substitué et L¹-L²-R¹⁶ ;
dans lequel un maximum de l'un de R⁵ et R⁷ est -L¹-L²-R¹⁶ ;
R¹³ et R¹⁴ sont chacun indépendamment choisis dans le groupe constitué de H, halogène, OH, CN, COOH, CONH₂, NH₂, NHCOH, alkyle en C₁-C₆ éventuellement substitué, alkylsulfonyle en C₁-C₆ éventuellement substitué, cycloalkyle en C₃-C₆ mono- ou bicyclique éventuellement substitué, alcényle en C₂-C₆ éventuellement substitué, alcynyle en C₂-C₆ éventuellement substitué, alkoxy en C₁-C₆ éventuellement substitué, groupe alkoxycarbonyle en C₁-C₆ éventuellement substitué, aryle en C₆-C₁₂ mono- ou bicyclique éventuellement substitué, hétéroaryle à 5 à 10 chaînons mono- ou bicyclique éventuellement substitué, hétérocycle à 3 à 8 chaînons mono- ou bicyclique éventuellement substitué, aryloxy éventuellement substitué, hétéroaryloxy éventuellement substitué et hétérocyclyloxy éventuellement substitué ;
L¹ est absent ou un alkylène en C₁-C₆ éventuellement substitué, un alcénylène en C₂-C₆ éventuellement substitué, un alcynylène en C₂-C₆ éventuellement substitué, O, S, S=O, SO₂ ou NR¹⁹ ;
L² est absent ou un alkylène en C₁-C₆ éventuellement substitué, un alcénylène en C₂-C₆ éventuellement substitué, un alcynylène en C₂-C₆ éventuellement substitué, O, S, S=O, SO₂ ou NR¹⁹ ;
R¹⁵ est un cycloalkyle en C₃-C₆ mono- ou bicyclique éventuellement substitué, un aryle en C₆-C₁₂ mono- ou bicyclique éventuellement substitué, un hétéroaryle à 5 à 10 chaînons mono- ou bicyclique éventuellement substitué ou un hétérocycle à 3 à 8 chaînons mono- ou bicyclique éventuellement substitué ;
R¹⁶ est un alkyle en C₁-C₆ éventuellement substitué, un alcényle en C₂-C₆ éventuellement substitué, un alcynyle en C₂-C₆ éventuellement substitué, un cycloalkyle en C₃-C₆ mono- ou bicyclique éventuellement substitué, un aryle en C₆-C₁₂ mono- ou bicyclique éventuellement substitué, un hétéroaryle à 5 à 10 chaînons mono- ou bicyclique éventuellement substitué ou un hétérocycle à 3 à 8 chaînons mono- ou bicyclique éventuellement substitué ; et
R¹⁷ à R¹⁹ sont indépendamment H, un alkyle en C₁-C₆ éventuellement substitué ou un alcényle en C₂-C₆ éventuellement substitué ;
ou un complexe, un sel, un solvate, une forme tautomère ou une forme polymorphe pharmaceutiquement acceptable de celui-ci,
ou une composition pharmaceutique comprenant un composé de formule (I), ou un complexe, un sel, un solvate, une forme tautomère ou une forme polymorphe pharmaceutiquement acceptable de celui-ci, et un véhicule pharmaceutiquement acceptable, destiné à être utilisé dans le traitement, l'amélioration ou la prévention d'une maladie choisie parmi la fibrose hépatique, la stéatose hépatique, la stéatohépatite non alcoolique (NASH), la fibrose pulmonaire, le lupus, la septicémie, la polyarthrite rhumatoïde (PR), le diabète de type I, la vasculopathie associée à STING avec apparition dans la petite enfance (SAVI), le syndrome d'Aicardi-Goutière (AGS), le lupus-engelure familial (FCL), le lupus érythémateux systémique (SLE), la vasculopathie rétinienne, la neuroinflammation, le syndrome de réponse inflammatoire systémique, la pancréatite, les maladies cardiovasculaires, la fibrose rénale, l'accident vasculaire cérébral et la dégénérescence maculaire liée à l'âge (DMLA).

15. Composé ou composition destiné(e) à être utilisé(e) selon la revendication 14, ladite maladie étant la fibrose, et ladite fibrose étant choisie dans le groupe constitué de la fibrose hépatique, la fibrose pulmonaire ou la fibrose rénale ; ou ladite maladie étant la stéatose hépatique, et ladite stéatose hépatique étant la stéatose hépatique non alcoolique (ou simple) ou la stéatohépatite non alcoolique (NASH).
